# EUROPEAN PATENT APPLICATION

(11) **EP 2 390 252 A1**
(43) Date of publication of application: **30.11.2011**
(21) Application number: 10382128.6
(22) Date of filing: 19.05.2010
(51) Int. Cl.: C07D 413/04, C07D 417/04, A61P 37/06, A61K 31/4245, A61K 31/433

(54) **New pyrazole derivatives**

(71) Applicant: Almirall, S.A., 08022 Barcelona (ES)
(72) Inventor: Erra Sola, Montserrat, 08980, SANT FELIU DE LLOBREGAT (ES); Aguilar Izquierdo, Nuria, 08980, Sant Feliu de Llobregat (ES); Carrascal Riera, Marta, 08980, Sant Feliu de Llobregat (ES); Taboada Martinez, Lorena, 08980, Sant Feliu de Llobregat (ES); Navarro Romero, Eloisa, 08980, Sant Feliu de Llobregat (ES); Vidal Juan, Bernat, 08980, Sant Feliu de Llobregat (ES)
(74) Representative: Elzaburu Marquez, Alberto

(57) **Abstract**

The present invention relates to a compound of formula (I), to the process for preparing such compounds and to their use in the treatment of a pathological condition or disease susceptible to amelioration by sphingosine-1-phosphate receptors (S1P1) agonists.

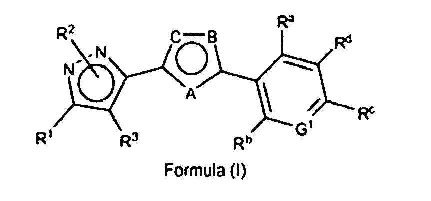

## Description

The present invention relates to new pyrazole derivatives, to processes for their preparation and to pharmaceutical compositions containing them. These compounds are potent agonists of S1 P1 receptors and thus, they are useful in the treatment, prevention or suppression of dise ases and disorders known to be susceptible to improvement by sphingosine-1-phosphate receptors agonists (S1P1), such as autoimmune diseases, chronic immune and inflammatory diseases, transplant rejection, malignant neoplastic diseases, angiogenic-related disorders, pain, neurological diseases, viral and infectious diseases.

Sphingosine -1 phosphate (S1 P) is a pleiotropic lipid mediator that exhibits a broad spectrum of biological activities, including cell proliferation, survival, lymphocyte trafficking, cytoskeletal organization, and morphogenesis. S1 P is generated from endogenous sphingosine through phosphorylation by specific kinases, named sphingosine kinases 1 and 2. The levels of S1 P in biological fluids and tissues are tightly regulated by the balance between its synthesis by sphingosine kinases and its degradation by S1 P lyase. This tight control is important since an excessive production of S1 P has been associated to various pathological conditions, such as angiogenesis and vascular permeability changes in cancer, inflammation, myocardial infarction or transplant rejection.

Gene deletion studies and reverse pharmacology have provided evidence that most of the effects of S1P are mediated via five G-protein coupled receptor subtypes, named S1 P1 to S1P5 (Brinkmann, Pharmacology & therapeutics 115:84-105, 2007). The interest on this family of receptors increased following the discovery that they were the pharmacological target of Gilenia (FTY720). This compound, a synthetic analog of a natural product derived from the fungus *Isaria sinclairii,* exhibited a peculiar immunomodulatory potential in vivo. When administered in vivo, it caused lymphopenia, due to the sequestration of lymphocytes from the blood into the lymph nodes and Peyer's patches. The close structural similarity of Gilenia to sphingosine, together with the discovery of the formation of phosphorylated FTY720 in vivo (FTY720P) prompted to speculate that FTY720-P could be acting as a mimetic of S1 P. This proven to be the case and it was later on demonstrated that FTY-P binds 4 of the five known S1 P receptors, namely S1P1, S1 P3, S1 P4 and S1 P5.

Expression analysis identified S1 P1 as the dominant S1 P receptor expressed on lymphocytes. Moreover, the transfer of S1P1-deficient T cells to normal mice led to the cells being sequestered in lymph nodes, as occurred with animals treated with fingolimod. These two facts strongly pointed out at S1P1 as the main receptor involved in the lymphopenic effect of FTY-P in vivo (Baumruker et al, Exp. Opin. Invest. Drugs 2007; 16(3): 283-289). FTY720 is currently in pre-registered phase for the treatment of relapsing-remitting multiple sclerosis. The drug is presumed to act by causing the retention of pathogenic lymphocytes in lymph nodes, thus preventing them to infiltrate the central nervous system (CNS).

In view of the physiological effects, several S1 P1 agonists have been recently disclosed for the treatment or prevention of autoimmune diseases, such as multiple sclerosis (WO2008000419, WO2008021532), rheumatoid arthritis or Crohn's disease (WO2007091501), chronic immune and inflammatory diseases such as asthma, transplant rejection (WO199400943) cancer (WO2003097028), lymphoid malignancies (WO2007143081), angiogenic-related disorders, pain (WO2004110421, WO2007089715) neurological diseases such as neurodegeneration (WO2005025553) or dementia (WO2005058295) and cardiovascular diseases (W02004010987).

Autoimmune diseases include but are not limited to rheumatoid arthritis, multiple sclerosis, inflammatory bowel diseases such as Crohn's diseases and ulcerative colitis, psoriatic arthritis, thyroiditis such as Hashimoto's thyroiditis, type I diabetes; systemic lupus erythematosis and Sjögrn's syndrome, rejection transplanted organs such as kidney, liver, heart, lung, pancreas, cornea and skin; graft-versus-hist disease brought about by stem cell transplantation.

Immune and inflammatory diseases which may be prevented or treated include but are not limited to asthma, COPD, respiratory distress syndrome, acute or chronic pancreatitis and hepatitis; chronic sarcoidosis, contact dermatitis, atopic dermatitis, allergic rhinitis, allergic conjunctivitis, Behcet syndrome, inflammatory eye conditions such as conjunctivitis and uveitis.

Malignant neoplastic diseases that may be prevented or treated include but are not limited to solid cancer, tumor metastasis and lymphoid malignancies

Angiogenesis-related disorders that may be prevented or treated include but are not limited to hemangiomas, ocular neovascularization, macular degeneration or diabetic retinopathy.

Pain including neuropathic pain, that may be prevented or treated include but are not limited to prophylaxis or treatment of chronic pain, wherein chronic pain is selected from chronic muscular diseases such as back pain, pain during menstruation, pain during osteoarthritis, pain during rheumatoid arthritis, pain during gastrointestinal inflammation, pain during inflammation of the heart muscle, pain during multiple sclerosis, pain during neuritis, pain during AIDS, pain during chemotherapy, tumor pain, neuropathic pain e. g. after amputation, trigeminal neuralgia, migraine or post herpetic neuralgia.

Cardiovascular diseases which may be prevented or treated include but are not limited to chronic heart failure, congestive heart failure, arrhythmia or tachyarrythmia, unstable angina, acute myocardial infarction or complications from cardiac surgery or for improving heart energy efficiency or cardiac output.

Neurological diseases including neurodegeneration, dementia or brain degeneration that may be prevented or treated include but are not limited to neurological disorders including Parkinson's desease, Parkinsonian disorders, Huntington's disease, Alzheimer's disease, amyotrophic lateral sclerosis, spinal ischemia, ischemic stroke, spinal cord injury, cancer-related brain injury, and cancer-related spinal cord injury, Shy-Drager syndrome, progressive supranuclear palsy, Lewy body disease, stroke, cerebral infarction, multi-infarct dementia, and geriatric dementia,

Viral diseases which may be prevented or treated include but are not limited to HIV infection, hepatitis C and cytomegalovirus infection.

Infectious diseases which may be prevented or treated include but are not limited to pathogenic fungal diseases.

It has now been found that certain pyrazoles are novel and potent agonists of S1P1 and can therefore be used in the treatment or prevention of these diseases.

Thus the present invention is directed to new pyrazole derivatives of formula (I) or pharmaceutically acceptable salts or N-oxides or solvates or deuterated derivative thereof: 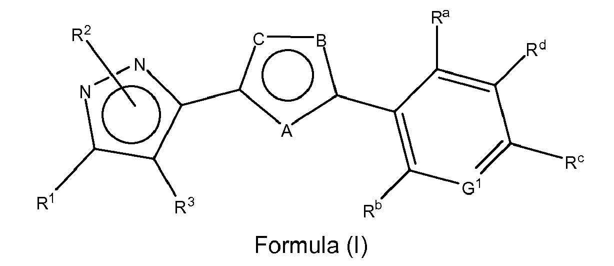 wherein:
- A is selected from the group consisting of -N(H)ᵢ-, -O- and -S-, wherein i is as defined below,
- B and C are independently selected from the group consisting of -N-, and -O-, with the proviso that at least two of A, B and C are nitrogen atoms,
- i is 1 when B and C both represent N, and is otherwise 0,
- G¹ is selected from the group consisting of -CR^{e}= and -N=,
- R¹ represents a hydrogen atom, a linear or branched C₁₋₆ alkyl group, a -(C₁₋₄ alkyl)(C₃₋₇ cycloalkyl) group, a linear or branched C₁₋₄ alkoxy group, a mono- or di- (C₁₋₂ alkyl)amino group, a C₃₋₇ cycloalkyl group, a benzyl group or a C₅₋₁₀ aryl group, wherein the aryl group is optionally substituted with one or more substituents selected from halogen atoms, a C₁₋₂ alkyl group, a C₁₋₂ alkoxy group, and a -CF₃ group,
- R² represents a linear or branched C₁₋₄ alkyl group, a -(C₁₋₄ alkyl)(C₃₋₇ cycloalkyl) group, a C₅₋₁₀ aryl group, or a benzyl group, with the proviso that only one of R¹ R² and R³ is aryl,
- R³ represents a hydrogen atom, a linear or branched C₁₋₄ alkyl group, a -(C₁₋₄ alkyl)(C₃₋₇ cycloalkyl) group, a linear or branched C₁₋₄ alkoxy group, or a C₅₋₁₀ aryl group, wherein the aryl group is optionally substituted with one or more substituents selected from halogen atoms, a C₁₋₂ alkyl group, a C₁₋₂ alkoxy group, and a -CF₃ group, with the proviso that R³ and R¹ cannot be both a hydrogen atom,
- R^{a} and R^{b} independently represent a hydrogen atom or a linear or branched C₁₋₄ alkyl group,
- R^{d} and R^{e} independently represent a hydrogen atom, a linear or branched C₁₋₄ alkyl group, a C₁₋₂ alkoxy group, a C₃₋₄ cycloalkyl group or a -CF₃ group,
- R^{c} represents
   o a hydroxy group,
   o a C₂₋₄ alkyl group substituted with one or more, typically 1 or 2, substituents selected from halogen atom, hydroxy group, carboxy group and amino group;
   o a C₁₋₃ alkoxy group substituted with one or more, typically 1, 2 or 3, substituents selected from amino group, halogen atoms and carbamoyl group, or
- R^{c} represents -(CH₂)₂NR'R" or -O-(CH₂)₂NR'R", wherein
   o R' represents a hydrogen atom or a C₁₋₄ alkyl group, and R" represents a C₁₋₂ alkyl group substituted with one or more, typically 1, 2 or 3 substituents selected from halogen atoms and carboxy group; or
   o R' and R" together with the nitrogen atom to which they are attached form a 4 to 6 membered, saturated heterocyclic ring optionally containing one or two additional nitrogen atoms as heteroatom and substituted with one or more substituents selected from a carboxy group and a C₁₋₂ alkyl group substituted with a carboxy group.

Preferably, when R^{c} is an alkyl group, the alkyl group does not carry more than one carboxy substituent. Preferably, when R^{c} is an alkoxy group, the alkoxy group does not carry more than one carbamoyl substituent.

Preferably, when R^{c} represents -(CH₂)₂NR'R" or -O-(CH₂)₂NR'R" and R" is an alkyl grou, the alkyl group does not carry more than one carboxy group.

Further objectives of the present invention are to provide a method for preparing said compounds; pharmaceutical compositions comprising an effective amount of said compounds; compounds for use in the treatment of a human or animal body, in particular, for the treatment of pathological conditions or diseases susceptible to improvement by sphingosine-1-phosphate receptors agonists (S1P1), wherein the pathological condition or disease is selected from autoimmune diseases, chronic immune and inflammatory diseases, transplant rejection, malignant neoplastic diseases, angiogenic-related disorders, pain, neurological diseases,viral and infectious diseases. The present invention further provides the use of the compounds in the manufacture of a medicament for the treatment of such pathological condition or diseases and methods of treatment of such pathological conditions or diseases susceptible to amelioration by sphingosine-1-phosphate receptors agonists (S1P1), wherein the pathological condition or disease is selected from autoimmune diseases, chronic immune and inflammatory diseases, transplant rejection, malignant neoplastic diseases, angiogenic-related disorders, pain, neurological diseases,viral and infectious diseases comprising the administration of the compounds of the invention to a subject in need of treatment.

As used herein the term alkyl embraces linear or branched hydrocarbon radicals having 1 to 6 carbon atoms. Examples include methyl, ethyl, *n-*propyl, *i-*propyl, *n-*butyl, *i*-butyl, *t*-butyl, n-pentyl and n-hexyl radicals.

As used herein, the term C₁₋₄ alkoxy (or alkyloxy) embraces linear or branched oxy-containing radicals each having alkyl portions of 1 to 4 carbon atoms.

Preferred alkoxy radicals include methoxy, ethoxy, n-propoxy, i-propoxy, n-butoxy, sec-butoxy and t-butoxy radicals.

As used herein, the term mono(C₁₋₂)alkylamino embraces radicals containing an alkyl radicals of 1 to 2 carbon atoms attached to a divalent -NH- radical.

Preferred mono(C₁₋₂)alkylamino radicals include methylamino, and ethylamino radicals.

As used herein, the term di(C₁₋₂)alkylamino embraces radicals containing a trivalent nitrogen atoms with two alkyl radicals of 1 to 2 carbon atoms in each alkyl radical.

Preferred di(C₁₋₂)alkylamino radicals include dimethylamino, diethylamino and methyl(ethyl)amino radicals.

As used herein, the term cycloalkyl embraces saturated carbocyclic radicals and, unless otherwise specified, a cycloalkyl radical typically has from 3 to 7 carbon atoms.

Examples include cyclopropyl and cyclobutyl, cyclopentyl, cyclohexyl and cycloheptyl radicals.

As used herein, the term C₅₋₁₀ aryl radical is preferably a C₆-C₁₀ monocyclic or polycyclic aryl radical such as phenyl, naphthyl, anthranyl and phenanthryl. Phenyl is preferred.

As used here, the term 4 to 6-membered heterocyclic ring in which R' and R" may form together with the nitrogen to which they are attached is typically a saturated C₄-C₆ carbocyclic ring system in which one of the carbon atoms is replaced by a nitrogen atom and optionally in which one or two further carbon atoms are replaced by a nitrogen. Thus, said 4 to 6-membered saturated heterocyclic group contains, as heteroatoms, the N atom to which R' and R" are attached and 0, 1 or 2 further N atoms.

Examples of 4 to 6-membered heterocyclic radicals include azetidyl, piperidyl, pyrrolidyl and piperazinyl. Piperidyl is preferred.

When R¹, R² or R³ represent a (C₁₋₄ alkyl)(C₃₋₇ cycloalkyl) group, it is to be understood that the C₁₋₄ alkyl portion is bonded to the pyrazole moiety of the molecule as depicted in formula (I).

As used herein, some of the atoms, radicals, moieties, chains or cycles present in the general structures of the invention are "optionally substituted". This means that these atoms, radicals, moieties, chains or cycles can be either unsubstituted or substituted in any position by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles. When two or more substituents are present, each substituent may be the same or different.

As used herein, the term halogen atom embraces chlorine, fluorine, bromine or iodine atoms typically a fluorine, chlorine or bromine atom, most preferably chlorine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X⁻⁾ is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

As used herein, an N-oxide is formed from the tertiary basic amines or imines present in the molecule, using a convenient oxidising agent.

As used herein, the term solvate means a compound which further includes a stoichiometric or non-stoichiometric amount of solvent such as water, acetone, ethanol, methanol, dichloromethane, 2-propanol, or the like, bound by non-covalent intermolecular forces. When the solvent is water, the term hydrate is used instead of solvate.

As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

Hydrogen deuterium exchange (deuterium incorporation) is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known too en ordinary skilled in the art, including mass spectrometry (MS) and nuclear magnetic resonance (NMR).

Typically, both A and B are -N- groups and C is -O- group.

Typically, both A and C are -N- groups and B is -O- group

Typically, A represents -O- or -S- and both B and C represent -N- groups

Typically, G¹ represents -CR^{e}= and R^{d} and/or R^{e} represents a hydrogen atom or a C₁₋₂ alkyl group, preferably a methyl group.

Typically, R¹ represents a branched C₃₋₄ alkyl group, a linear or branched C₁₋₄ alkoxy group, a di(C₁₋₂ alkyl)amino group, a C₄₋₆ cycloalkyl group, a benzyl group or a C₅₋₆ aryl group, wherein the aryl group is optionally substituted with one substituent selected from a halogen atom, a C₁₋₂ alkoxy group and a -CF₃ group.

Preferably, R¹ represents a branched C₃₋₄ alkyl group, a linear or branched C₃₋₄ alkoxy group, a di-(C₁₋₂ alkyl)amino group, a cyclohexyl group or a phenyl group, wherein the phenyl group is optionally substituted with one substituent selected from a chlorine atom, a methoxy group and a -CF₃ group. More preferably R¹ represents a branched C₃₋₄ alkyl group, a linear or branched C₃₋₄ alkoxy group or a di- (C₁₋₂ alkyl)amino group.

Typically, R² represents a C₁₋₂ alkyl group or a -(C₁₋₂ alkyl)(C₃₋₄ cycloalkyl) group. Preferably R² represents a methyl group, an ethyl group or a cyclopropyl-methyl group, being more preferably R² represents a methyl or an ethyl group.

Typically, R³ represents a hydrogen atom, a C₁₋₂ alkyl group, a -(C₁₋₂ alkyl)(C₃₋₄ cycloalkyl) group, a C₁₋₂ alkoxy group. Preferably R³ represents a hydrogen atom or a methyl group, being more preferably R³ represents a hydrogen atom.

Typically, R^{a} and R^{b} independently represent a hydrogen atom or a methyl group, preferably both R^{a} and R^{b} represent a hydrogen atom.

Typically, R^{d} and R^{e} independently represent a hydrogen atom, a C₁₋₂ alkyl group, or a -CF₃ group, preferably R^{d} represents a hydrogen atom, a methyl group, an ethyl group or -CF₃ group, being more preferably a methyl group or -CF₃ group.

Typically, R^{c} represents a C₂₋₄ alkyl group substituted with one or more substituents selected from a hydroxy group, a carboxy group and an amino group or R^{c} represents -(CH₂)₂NR'R" group, wherein
o R' represents a hydrogen atom or a methyl group, and R" represents a C₁₋₂ alkyl group substituted with one or more substituents selected from halogen atoms and a carboxy group; or
o R' and R" together with the nitrogen atom to which they are attached form a 4 to 6 membered, saturated heterocyclic ring which is substituted with one substituent selected from a carboxy group and a methyl group which is substituted with a carboxy group.

Preferably, R^{c} represents -(CH₂)₂NR'R" group, wherein
o R' represents a hydrogen atom or a methyl group, and R" represents a C₁₋₂ alkyl group substituted with a carboxy group; or
o R' and R" together with the nitrogen atom to which they are attached form a 4 to 6 membered, saturated heterocyclic ring which is substituted with a carboxy group.

In a preferred embodiment of the invention, R¹ represents a branched C₃₋₄ alkyl group, a linear or branched C₃₋₄ alkoxy group or a di-(C₁₋₂ alkyl)amino group, R² represents a methyl or an ethyl group, R³ represents a hydrogen atom, both R^{a} and R^{b} represent a hydrogen atom, G¹ represents -CR^{e}=, one of R^{d} and R^{e} represents a methyl group or -CF₃ group, and the other represents a hydrogen atom or a methyl group, and R^{c} represents -(CH₂)₂NR'R" group, wherein
o R' represents a hydrogen atom or a methyl group, and R" represents a C₁₋₂ alkyl group substituted with a carboxy group; or
o R' and R" together with the nitrogen atom to which they are attached form a 4 to 6 membered, saturated heterocyclic ring which is substituted with a carboxy group.

In a preferred embodiment both R^{d} and R^{e} are methyl group

In another preferred embodiment one of R^{d} and R^{e} represents -CF₃ group and the other is a hydrogen atom.

In another embodiment of the invention, G¹ is selected from the group consisting of - CR^{e}= and -N=, R¹ represents a hydrogen atom, an isopropyl group, an isobutyl group, a tert-butyl group, a neopentyl group, a propoxy group, an isopropoxy group, a diethylamino group, a cyclohexyl group, a benzyl group, a phenyl group which is optionally substituted with one substituent selected from a chlorine atom, a fluorine atom, a methyl group, a methoxy group and a -CF₃ group, R² represents a methyl group, an ethyl group, a cycloproylmethyl group or a phenyl group, R³ represents a hydrogen atom, a methyl group or a phenyl group, with the proviso that R³ and R¹ cannot be both a hydrogen atom, R^{a} and R^{b} independently represent a hydrogen atom or a methyl group, R^{d} and R^{e} independently represent a hydrogen atom, a methyl group, an ethyl group or a -CF₃ group, R^{c} represents a hydroxy group, a C₂₋₄ alkyl group substituted with one or two substituents selected from a halogen atom, a hydroxy group, a carboxy group and an amino group; a C₁₋₃ alkoxy group substituted with one to three substituents selected from an amino group, a fluorine atom and a carbamoyl group, or R^{c} represents -(CH₂)₂NR'R" group or -O-(CH₂)₂NR'R" group, wherein
o R' represents a hydrogen atom or a methyl group, and R" represents a C₁₋₂ alkyl group substituted with one to three substituents selected from a fluorine atom and a carboxy group; or
o R' and R" together with the nitrogen atom to which they are attached form a 4 to 6 membered, saturated heterocyclic ring which is substituted with one substituent selected from a carboxy group and a carboxymethyl group

Particular individual compounds of the invention include:
3-{2 ,6-dimethyl-4-[5-(1-methyl-5-phenyl-1H-pyrazol-3-yl)-1,2,4-oxadiazol-3-yl]phenyl}propane-1,2-diol,
N-(2-{2,6-dimethyl-4-[5-(1-methyl-5-phenyl-1H-pyrazol-3-yl)-1,2,4-oxadiazol-3-yl]phenyl}ethyl)-2,2,2-trifluoroethanamine,
N-(2-{2,6-dimethyl-4-[5-(1-methyl-5-phenyl-1H-pyrazol-3-yl)-1,2,4-oxadiazol-3-yl]phenyl}ethyl)glycine,
1-(2-{2,6-dimethyl-4-[5-(1-methyl-5-phenyl-1H-pyrazol-3-yl)-1,2,4-oxadiazol-3-yl]phenyl}ethyl)piperidine-4-carboxylic acid,
3-ethyl-6-methyl-5-[5-(1-methyl-5-phenyl-1H-pyrazol-3-yl)-1,2,4-oxadiazol-3-yl]pyridin-2-ol,
(2-{2-ethyl-6-methyl-4-[5-(1-methyl-5-phenyl-1H-pyrazol-3-yl)-1,2,4-oxadiazol-3-yl]ph nyl}ethyl)amine,
(2-{2-ethyl-6-methyl-4-[5-(1-methyl-5-phenyl-1H-pyrazol-3-yl)-1,2,4-oxadiazol-3-yl]phenyl}ethyl)amine,
[3-({3-ethyl-6-methyl-5-[5-(1-methyl-5-phenyl-1H-pyrazol-3-yl)-1,2,4-oxadiazol-3-yl]pyridin-2-yl}oxy)propyl]amine,
(2-{2,6-dimethyl-4-[5-(1-methyl-5-phenyl-1H-pyrazol-3-yl)-1,2,4-oxadiazol-3-yl]phenoxy}ethyl)amine,
2-{2,6-dimethyl-4-[5-(1-methyl-5-phenyl-1H-pyrazol-3-yl)-1,2,4-oxadiazol-3-yl]phenoxy}-2,2-difluoroacetamide,
(2-{2,6-dimethyl-4-[5-(1-methyl-5-phenyl-1H-pyrazol-3-yl)-1,2,4-oxadiazol-3-yl]phenoxy}-2,2-difluoroethyl)amine,
{2-[4-[5-(1-methyl-5-phenyl-1H-pyrazol-3-yl)-1 ,2,4-oxadiazol-3-yl]-2-(trifluoromethyl)phenoxy]ethyl}amine,
1-(2-{2,6-dimethyl-4-[5-(1-methyl-5-phenyl-1H-pyrazol-3-yl)-1,2,4-oxadiazol-3-yl]phenoxy}ethyl)piperidine-4-carboxylic acid,
3-ethyl-5-[5-(1-ethyl-5-phenyl-1H-pyrazol-3-yl)-1,2 ,4-oxadiazol-3-yl]-6-methylpyridin-2-ol,
5-{5-[5-(2-chlorophenyl)-1-ethyl-1H-pyrazol-3-yl]-1,2,4-oxadiazol-3-yl}-3-ethyl-6-methylpyridin-2-ol,
{2-[4-[5-(1-methyl-5-phenyl-1H-pyrazol-3-yl)-1,2,4-oxadiazol-3-yl]-2-(trifluoromethyl)phenyl]ethyl}amine,
5-{5-[3-(2-chlorophenyl)-1-ethyl-1H-pyrazol-5-yl]-1,2,4-oxadiazol-3-yl}-3-ethyl-6-methylpyridin-2-ol,
1-(2-{4-[5-(1-ethyl-5-phenyl-1H-pyrazol-3-yl)-1,2,4-oxadiazol-3-yl]-2,6-dimethylphenyl}ethyl)piperidine-4-carboxylic acid,
N-(2-{4-[5-(1-ethyl-5-phenyl-1H-pyrazol-3-yl)-1,2,4-oxadiazol-3-yl]-2,6-dimethylphenyl}ethyl)glycine,
1-(2-{4-[5-(1-ethyl-5-phenyl-1H-pyrazol-3-yl)-1,2,4-oxadiazol-3-yl]-2,6-dimethylphenyl}ethyl)azetidine-3-carboxylic acid,
N-(2-{4-[5-(1-ethyl-5-phenyl-1H-pyrazol-3-yl)-1,2,4-oxadiazol-3-yl]-2,6-dimethylphenyl}ethyl)-N-methylglycine,
[1-(2-{4-[5-(1-ethyl-5-phenyl-1H-pyrazol-3-yl)-1,2,4-oxadiazol-3-yl]-2,6-dimethylphenyl}ethyl)piperidin-4-yl]acetic acid,
1-(2-{4-[5-(1-ethyl-5-phenyl-1H-pyrazol-3-yl)-1,2,4-oxadiazol-3-yl]-2,6-dimethylphenyl}ethyl)pyrrolidine-3-carboxylic acid,
1-[2-(4-{5-[5-(2-chlorophenyl)-1-ethyl-1H-pyrazol-3-yl]-1,2,4-oxadiazol-3-yl}-2,6-dimethylphenyl)ethyl]piperidine-4-carboxylic acid,
1-(2-{4-[5-(1-ethyl-5-isopropyl-1H-pyrazol-3-yl)-1,2,4-oxadiazol-3-yl]-2,6-dimethylphenyl}ethyl)piperidine-4-carboxylic acid,
1-[2-(4-{5-[5-(3-chlorophenyl)-1-ethyl-1H-pyrazol-3-yl]-1,2,4-oxadiazol-3-yl}-2,6-dimethylphenyl)ethyl]piperidine-4-carboxylic acid,
1-(2-{3-methyl-4-[5-(1-methyl-5-phenyl-1H-pyrazol-3-yl)-1,2,4-oxadiazol-3-yl]phenyl}ethyl)piperidine-4-carboxylic acid,
1-[2-(4-{5-[1-ethyl-5-(3-fluorophenyl)-1H-pyrazol-3-yl]-1,2,4-oxadiazol-3-yl}-2,6-dimethylphenyl)ethyl]piperidine-4-carboxylic acid,
1-(2-{2-methyl-4-[5-(1-methyl-5-phenyl-1H-pyrazol-3-yl)-1,2,4-oxadiazol-3-yl]phenyl}ethyl)piperidine-4-carboxylic acid,
1-{2-[4-[5-(1-methyl-5-phenyl-1H-pyrazol-3-yl)-1,2,4-oxadiazol-3-yl]-2-(trifluoromethyl)phenyl]ethyl}piperidine-4-carboxylic acid,
1-(2-{4-[5-(5-tert-butyl-1-methyl-1H-pyrazol-3-yl)-1,2,4-oxadiazol-3-yl]-2,6-dimethylphenyl}ethyl)piperidine-4-carboxylic acid,
1-(2-{4-[5-(5-cyclohexyl-1-ethyl-1H-pyrazol-3-yl)-1,2,4-oxadiazol-3-yl]-2,6-dimethylphenyl}ethyl)piperidine-4-carboxylic acid,
1-(2-{4-[5-(5-cyclohexyl-1-ethyl-1H-pyrazol-3-yl)-1,2,4-oxadiazol-3-yl]-2,6-dimethylphenyl}ethyl)azetidine-3-carboxylic acid,
1-[2-(4-{5-[1-ethyl-5-(4-methoxyphenyl)-1H-pyrazol-3-yl]-1,2,4-oxadiazol-3-yl}-2,6-dimethylphenyl)ethyl]piperidine-4-carboxylic acid,
1-(2-{4-[5-(1-ethyl-5-isobutyl-1H-pyrazol-3-yl)-1,2,4-oxadiazol-3-yl]-2,6-dimethylphenyl}ethyl)piperidine-4-carboxylic acid,
1-(2-{4-[5-(1-ethyl-5-isobutyl-1H-pyrazol-3-yl)-1,2,4-oxadiazol-3-yl]-2,6-dimethylphenyl}ethyl)azetidine-3-carboxylic acid,
1-[2-(4-{5-[1-ethyl-5-(4-fluorophenyl)-1H-pyrazol-3-yl]-1,2,4-oxadiazol-3-yl}-2,6-dimethylphenyl)ethyl]piperidine-4-carboxylic acid,
1-[2-(4-{5-[1-ethyl-5-(2-methoxyphenyl)-1H-pyrazol-3-yl]-1,2,4-oxadiazol-3-yl}-2,6-dimethylphenyl)ethyl]piperidine-4-carboxylic acid,
1-(2-{4-[5-(1-ethyl-5-phenyl-1H-pyrazol-3-yl)-1,2,4-oxadiazol-3-yl]-2,5-dimethylphenyl}ethyl)piperidine-4-carboxylic acid,
1-[2-(4-{5-[1-ethyl-5-(2-fluorophenyl)-1H-pyrazol-3-yl]-1,2,4-oxadiazol-3-yl}-2,6-dimethylphenyl)ethyl]piperidine-4-carboxylic acid,
1-(2-{4-[5-(1-ethyl-4-methyl-5-phenyl-1H-pyrazol-3-yl)-1,2,4-oxadiazol-3-yl]-2,6-dimethylphenyl}ethyl)piperidine-4-carboxylic acid,
1-{2-[4-(5-{1-ethyl-5-[2-(trifluoromethyl)phenyl]-1H-pyrazol-3-yl}-1,2,4-oxadiazol-3-yl)-2,6-dimethylphenyl]ethyl}piperidine-4-carboxylic acid,
1-[2-(4-{5-[1-ethyl-5-(2-methylphenyl)-1H-pyrazol-3-yl]-1,2,4-oxadiazol-3-yl}-2,6-dimethylphenyl)ethyl]piperidine-4-carboxylic acid,
3-{4-[5-(1-ethyl-5-phenyl-1H-pyrazol-3-yl)-1,2,4-oxadiazol-3-yl]-2,6-dimethylphenyl}propanoic acid,
N-(2-{2,6-dimethyl-4-[5-(1-methyl-5-phenyl-1H-pyrazol-3-yl)-1,3,4-oxadiazol-2-yl]phenyl}ethyl)glycine,
1-(2-{4-[5-(5-benzyl-1-ethyl-1H-pyrazol-3-yl)-1,2,4-oxadiazol-3-yl]-2,6-dimethylphenyl}ethyl)piperidine-4-carboxylic acid,
1-[2-(4-{5-[5-(2,2-dimethylpropyl)-1-ethyl-1H-pyrazol-3-yl]-1,2,4-oxadiazol-3-yl}-2,6-dimethylphenyl)ethyl]piperidine-4-carboxylic acid,
1-{2-[4-[5-(1-ethyl-5-cyclohexyl-1H-pyrazol-3-yl)-1,2,4-oxadiazol-3-yl]-2-(trifluoromethyl)phenyl]ethyl}piperidine-4-carboxylic acid,
1-(2-{4-[5-(1-ethyl-5-isobutyl-4-methyl-1H-pyrazol-3-yl)-1,2,4-oxadiazol-3-yl]-2,6-dimethylphenyl}ethyl)piperidine-4-carboxylic acid,
1-(2-{4-[5-(1-ethyl-5-isobutyl-1H-pyrazol-3-yl)-4H-1,2,4-triazol-3-yl]-2,6-dimethylphenyl}ethyl)piperidine-4-carboxylic acid,
1-[2-(4-(5-[1-(cyclopropylmethyl)-5-phenyl-1H-pyrazol-3-yl]-1,2,4-oxadiazol-3-yl}-2,6-dimethylphenyl)ethyl]piperidine-4-carboxylic acid,
1-(2-{4-[5-(1-ethyl-5-isopropoxy-1H-pyrazol-3-yl)-1,2,4-oxadiazol-3-yl]-2,6-dimethylphenyl}ethyl)piperidine-4-carboxylic acid,
1-(2-{4-[5-(1-ethyl-5-isobutyl-1H-pyrazol-3-yl)-1,3,4-thiad iazol-2-yl]-2,6-dimethylphenyl}ethyl)piperidine-4-carboxylic acid,
1-(4-(3-(5-cyclohexyl-1-ethyl-1H-pyrazol-3-yl)-1,2,4-oxadiazol-5-yl)-2,6-dimethylphenethyl)piperidine-4-carboxylic acid,
1-(4-(5-(1-ethyl-4-phenyl-1H-pyrazol-3-yl)-1,2,4-oxadiazol-3-yl)-2,6-dimethylphenethyl)piperidine-4-carboxylic acid,
1-(4-(5-(1-ethyl-4-phenyl-1H-pyrazol-3-yl)-1,2,4-oxadiazol-3-yl)-2-(trifluoromethyl)phenethyl)piperidine-4-carboxylic acid,
1-(4-(5-(1-ethyl-5-isobutyl-1H-pyrazol-3-yl)-1,2,4-oxadiazol-3-yl)-2-(trifluoromethyl)phenethyl)piperidine-4-carboxylic acid,
1-(4-(5-(1-ethyl-5-isobutyl-1H-pyrazol-3-yl)-1,2,4-oxadiazol-3-yl)-2-(trifluoromethyl)phenethyl)azetidine-3-carboxylic acid,
2-((4-(5-(1-ethyl-5-isobutyl-1H-pyrazol-3-yl)-1,2,4-oxadiazol-3-yl)-2-(trifluoromethyl)phenethyl)(methyl)amino)acetic acid,
3-(4-(5-(5-(diethylamino)-1-ethyl-1H-pyrazol-3-yl)-1,2,4-oxadiazol-3-yl)-2,6-dimethylphenyl)propanoic acid,
1-(4-(5-(1-ethyl-5-propoxy-1H-pyrazol-3-yl)-1,2,4-oxadiazol-3-yl)-2,6-dimethylphenethyl)piperidine-4-carboxylic acid,
1-(4-(5-(1-ethyl-5-isopropoxy-1H-pyrazol-3-yl)-1,2,4-oxadiazol-3-yl)-2-(trifluoromethyl)phenethyl)piperidine-4-carboxylic acid,
or a pharmaceutically acceptable salt or N-oxides or solvates or deuterated derivative thereof.

Of outstanding interest are:
N-(2-{2,6-dimethyl-4-[5-(1-methyl-5-phenyl-1H-pyrazol-3-yl)-1,2,4-oxadiazol-3-yl]phenyl}ethyl)-2,2,2-trifluoroethanamine,
1-(2-{4-[5-(1-ethyl-5-phenyl-1H-pyrazol-3-yl)-1,2,4-oxadiazol-3-yl]-2,6-dimethylphenyl}ethyl)piperidine-4-carboxylic acid,
1-[2-(4-{5-[5-(2-chlorophenyl)-1-ethyl-1H-pyrazol-3-yl]-1,2,4-oxadiazol-3-yl}-2,6-dimethylphenyl)ethyl]piperidine-4-carboxylic acid,
1-(2-{4-[5-(5-cyclohexyl-1-ethyl-1H-pyrazol-3-yl)-1,2,4-oxadiazol-3-yl]-2,6-dimethylphenyl}ethyl)piperidine-4-carboxylic acid,
1-(2-{4-[5-(1-ethyl-5-isobutyl-1H-pyrazol-3-yl)-1,2,4-oxadiazol-3-yl]-2,6-dimethylphenyl}ethyl)piperidine-4-carboxylic acid,
N-(2-{2,6-dimethyl-4-[5-(1-methyl-5-phenyl-1H-pyrazol-3-yl)-1,3,4-oxadiazol-2-yl]phenyl}ethyl)glycine,
1-{2-[4-[5-(1-ethyl-5-cyclohexyl-1H-pyrazol-3-yl)-1,2,4-oxadiazol-3-yl]-2-(trifluoromethyl)phenyl]ethyl}piperidine-4-carboxylic acid,
1-(2-{4-[5-(1-ethyl-5-isopropoxy-1H-pyrazol-3-yl)-1,2,4-oxadiazol-3-yl]-2,6-dimethylphenyl}ethyl)piperidine-4-carboxylic acid,
1-(2-{4-[5-(1-ethyl-5-isobutyl-1H-pyrazol-3-yl)-1,3,4-thiad iazol-2-yl]-2,6-dimethylphenyl}ethyl)piperidine-4-carboxylic acid,
1-(4-(3-(5-cyclohexyl-1-ethyl-1H-pyrazol-3-yl)-1,2,4-oxadiazol-5-yl)-2,6-dimethylphenethyl)piperidine-4-carboxylic acid,
1-(4-(5-(1-ethyl-5-isobutyl-1H-pyrazol-3-yl)-1,2,4-oxadiazol-3-yl)-2-(trifluoromethyl)phenethyl)piperidine-4-carboxylic acid and
2-((4-(5-(1-ethyl-5-isobutyl-1H-pyrazol-3-yl)-1,2,4-oxadiazol-3-yl)-2-(trifluoromethyl)phenethyl)(methyl)amino)acetic acid,

Compounds of general formula (I) may be obtained following the synthetic scheme depicted in figure 1.

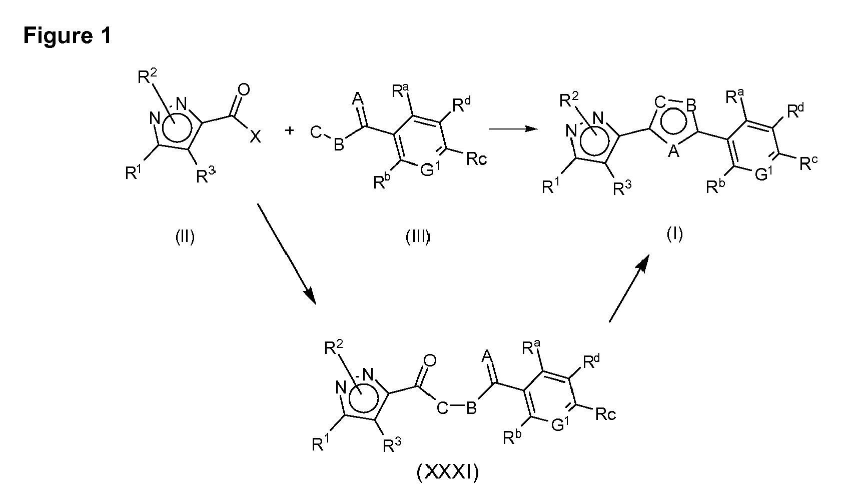

Compounds of general formula (I) may be prepared by reacting intermediates of general formula (II) wherein X represents a hydroxy group or a chlorine atom, with intermediates formula (III) in a one pot reaction. This reaction is carried out in a solvent such as dimethylformamide (DMF), N-methylpyrrolidone (NMP) or tetrahydrofurane (THF), optionally in the presence of one or more coupling agents such as 2-(1H-benzotriazole-1-yl)-1,2,3,3-tetramethyluronium tetrafluoroborate (TBTU), N-(3-dimethylaminopropyl)-N'-ethyl-carbodiimide (EDC), dicyclohexyl carbodiimide (DCC), 1-hydroxybenzotriazole (HOBt), O-(benzotriazol-1-yl)-N,N,N',N'-tetramethyluroniium hexafluorophosphate (HBTU), carbonyl diimidazole (CDI), or the like and optionally in the presence of a base such as triethylamine, Hünig's base, sodium hydride, potassium carbonate, or the like. The reaction is carried out in a standard reactor at a temperature renaging between 40 and 150°C.

An alternative method for the preparation of compounds of formula (I) may be done following a two steps synthesis. The first step is carried out by coupling intermediates of formula (II) and formula (III) with one or more coupling agent as described before thus giving intermediates of formula (XXXI) and then, in a second step, a subsequent cyclization of intermediates of ofmrula (XXXI) in a solvent such as xylene, toluene, benzene, pyridine, DMF, dichloromethane, acetic acid, trifluoroacetic acid, at room temperature or elevated temperatures, optionally in the presence of auxiliaries such as acids (e.g. trifluoroacetic acid, acetic acid, hydrochloric acid, etc.), bases (e.g., sodium hydride, sodium acetate, sodium carbonate, potassium carbonate, triethylamine, etc.), tetralkylammonium salts or water removing agents such as oxalyl chloride, a carboxylic acid anhydride, phosphoryl trichloride (POCl₃), tetrabutylammonium fluoride (TBAF), molecular sieves, etc.

In the particular case in which A is -S- group, a thiation reagent such as Laweson Reagent (2,4-bis(4-methoxyphenyl)-1,3,2,4-dithiadiphosphetane-2,4-disulfide) or P₄S₁₀ may be used in the cyclization step. A solvent such as toluene, xylene or benzene is used in a temperature range from room temperature to the solvent boiling point.

Alternatively compounds of general formula (I) may be prepared as shown in figure 2.

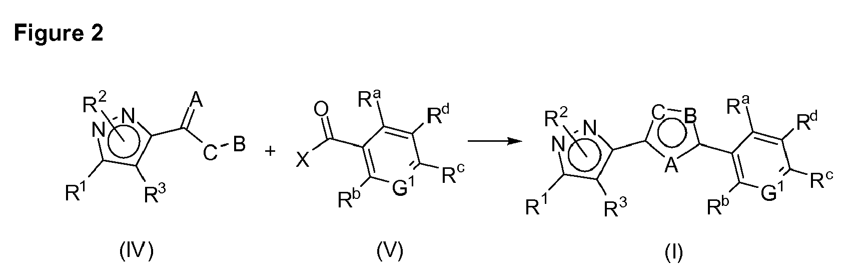

The reaction is carried out by reacting intermediates of general formula (IV) with intermediates of general formula (V) following the same synthetic procedures described in figure 1.

Intermediates of general formula (III) and (IV) may be prepared following the synthetic schemes depicted in figure 3.
**Figure 3:**

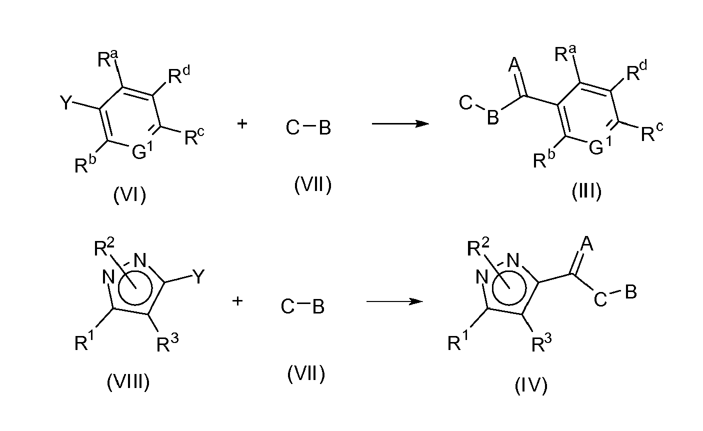

Intermediates of formula (III) and (IV) may be obtained from the corresponding intermediates of formula (VI) and (VIII), respectively wherein Y represents -CN, (in this case A represents -N-), -COOH, -COCI or -COOR', wherein R' is as defined above (in this case A represents -O- group). The synthesis is carried out by reacting intermediates of formula (VI) or (VIII), with hydroxylamine or hydrazine or any salt thereof as intermediates of formula (VII), respectively, in a solvent such as THF, methanol, ethanol, pyridine, optionally in the presence of a base such as sodium bicarbonate, sodium carbonate, potassium carbonate, triethylamine, sodium ethoxide, and at a temperature ranging from room temperature to the boiling point of the solvent. This reaction may be optionally carried out in the presence of a coupling agent such as 2-(1H-benzotriazole-1-yl)-1,2,3,3-tetramethyluroniumtetrafluoroborate, N-(3-dimethyl aminopropyl)-N'-ethyl-carbodiimide, dicyclohexyl carbodiimide, 1-hydroxybenzotriazole.

In the particular case of compounds of general formula (I) wherein A and B are nitrogen and C is an oxygen, compounds of general formula (Ia) may be prepared following the synthetic scheme depicted in figure 4.

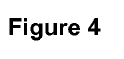

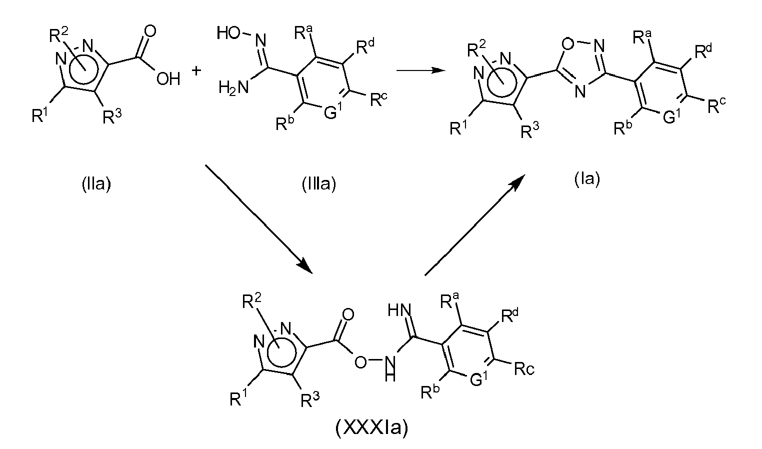

Compounds of general formula (Ia) may be prepared by the condensation of the carboxylic acid derivatives of formula (IIa) with the corresponding carboximidamide derivative of formula (IIIa) in a solvent such as DMF, NMP or THF, in the presence of one or more coupling agents such as 2-(1H-benzotriazole-1-yl)-1,2,3,3-tetramethyluronium tetrafluoroborate, N-(3-dimethylaminopropyl)-N'-ethyl-carbodiimide, dicyclohexyl carbodiimide, 1-hydroxybenzotriazole, O-(benzotriazol-1-yl)-N,N,N',N'-tetramethyluroniium hexafluorophosphate, carbonyl diimidazole, and optionally in the presence of a base such as triethylamine, Hünig's base, sodium hydride, potassium carbonate, at a temperature between 40 and 150°C and in a standard reactor.

An alternative synthetic method may be carried out by first coupling intermediates of formula (IIa) with intermediates of formula (IIIa) as described before thus giving Intermediates of formula (XXXIa), and subsequent cyclization of intermediates (XXXIa) in a solvent such as xylene, toluene, benzene, pyridine, dimethylformamide, dichloromethane, acetic acid, trifluoroacetic acid, at room temperature or elevated temperatures, optionally in the presence of auxiliaries such as acid (e.g. trifluoroacetic acid, acetic acid, hydrochloric acid, etc.), bases (e.g., sodium hydride, sodium acetate, sodium carbonate, potassium carbonate, triethylamine, etc.), tetralkylammonium salts or water removing agents (e.g. oxalyl chloride, a carboxylic acid anhydride, phosphoryl trichloride, tetrabutilamonium fluoride, molecular sieves etc.)

Compounds of general formula (IIa) may be prepared following the synthetic scheme depicted in figure 5.

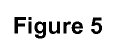

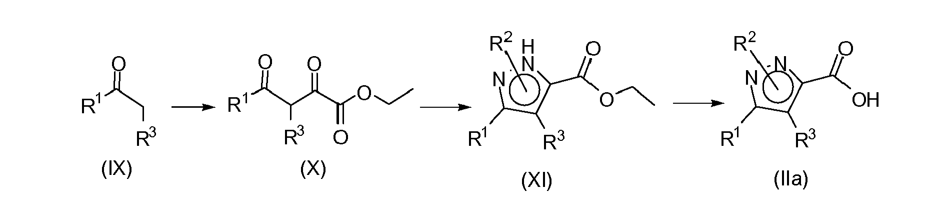

Hydrolysis of the ethyl ester derivatives of formula (XI) in basic conditions with a base such as aqueous sodium hydroxide or lithium hydroxide in a solvent such as methanol, ethanol or THF or a mixture of them at a temperature between 20 and 80°C or in acidic conditions with an acid such as HCl and a solvent such as water or ethanol or a mixture of them at a temperature between 20 and 80°C gives the acid intermediates of formula (IIa).

Intermediates of general formula (XI) may be prepared by the condensation of the intermediates of formula (X) with the corresponding hydrazine in basic media such as triethylamine and in a protic solvent such as ethanol at a temperature between 20°C and the boiling point of the solvent.

Intermediates of general formula (X) may be prepared by the reaction of the corresponding ketone derivatives of formula (IX) with diethyloxalate in a basic media such as sodium ethoxide, in a protic solvent such as ethanol and at a temperature between 20°C and the boiling point of the solvent. Ketones of general formula (IX) are either commercially available or may be prepared using synthetic methods known in the art.

In the particular case where R¹ is a linear or branched C₁₋₄ alkoxy group and R³ is a hydrogen atom, intermediates of general formula (IIb) may be obtained as shown in figure 6.

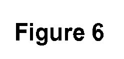

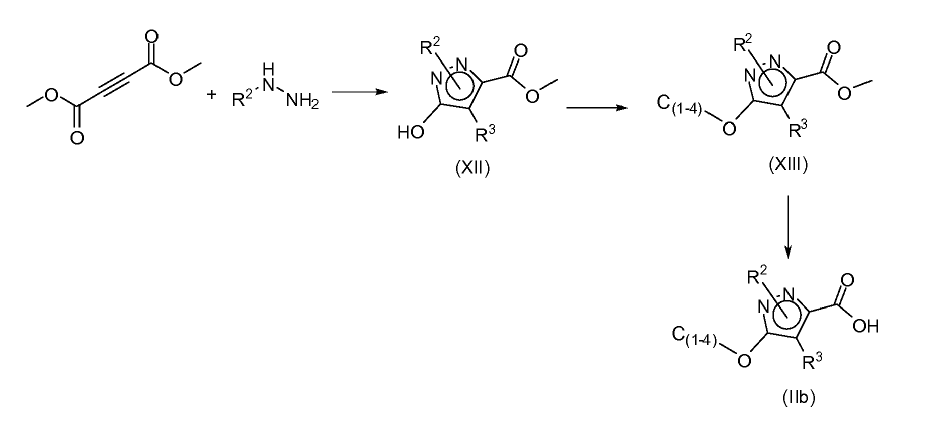

Intermediates of general formula (XII) may be obtained by cyclization of dialkyl but-2-ynedioate and the corresponding substituted hydrazine or any salt thereof in the presence of a base such as potassium carbonate, sodium carbonate or triethylamine, in a solvent such as dioxane, THF, ethanol or methanol at a temperature between 40°C and the boiling point of the solvent. By alkylation of these precursors with the corresponding linear or branched alkyl halide in a solvent such as DMF or THF in the presence of potassium carbonate or sodium hydride at a temperature between 40°C and the boiling point of the solvent, intermediates of formula (XIII) may be obtained, followed by the final deprotection using the synthetic methods already known in the art.

Intermediates of general formula (IIIa) may be prepared following the synthetic scheme depicted in figure 7.
**Figure 7**

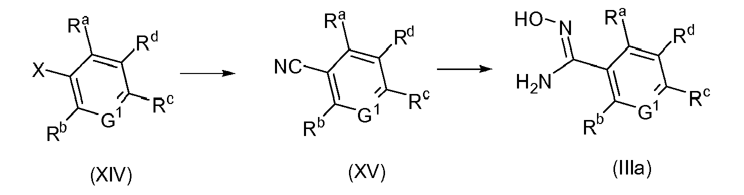

Intermediates of formula (IIIa) may be obtained by the reaction of hydroxylamine hydrochloride or any of its salts with the corresponding nitrile (XV) in basic media such as sodium bicarbonate or triethylamine in a solvent such as THF, methanol or ethanol and at a temperature from 40 to 100°C. If the cyanoaryl derivative of formula (XV) is not commercially available it may be obtained from the corresponding derivative of formula (XIV) wherein X represents a bromide or a triflate, by reacting with a source of a cyanide such as copper (I) cyanide, in a high boiling point solvent such as N-methylpirrrolidine, dimethylformamide or dimethylsulfoxide at a temperature between 150-200°C. Alternatively dicyanozinc may be used with a palladium catalyst such as tetrakis(triphenylphosphine)-palladium(0) in a high boiling point solvent, in a standard reactor or a microwave reactor.

In the particular case where R_{c} is -(CH₂)₂COOR', intermediates of formula (IIIb) may be obtained following the synthetic path shown in Figure 8.

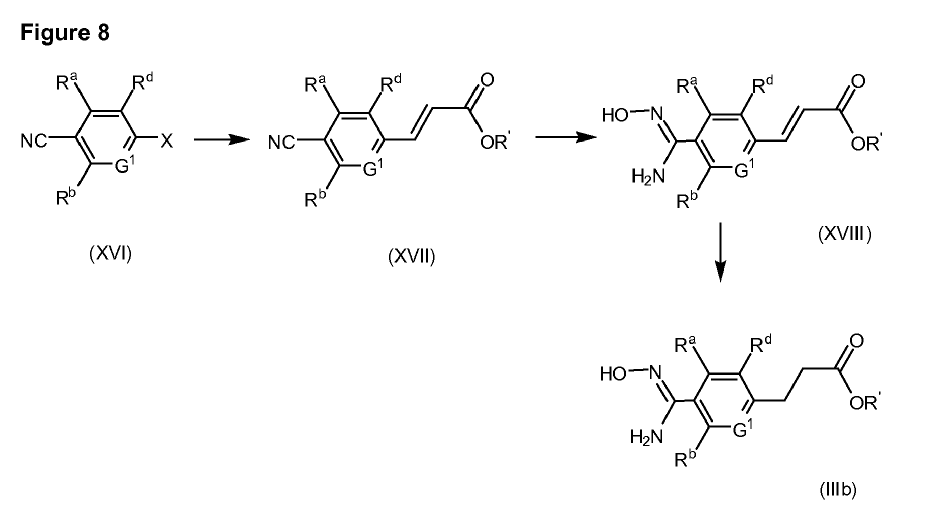

A Heck reaction of the corresponding precursor (XVI), wherein X represents a triflate group, a bromine atom, a iodine atom or a chlorine atom, which are known or may be prepared according to known procedures, with an alkyl acrylate yields the intermediate of formula (XVII). Reaction of these intermediates with hydroxylamine as described above, for the preparation of (IIIa) from (XV), followed by a catalytic hydrogenation, gives intermediates of general formula (IIIb).

General compounds of formula (Id) may be obtained following the synthetic path shown in Figure 9.

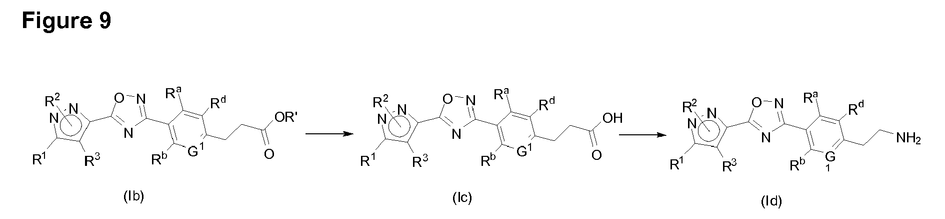

From compounds of formula (Ib), the corresponding acid derivatives may be prepared by basic hydrolysis in a protic solvent such as methanol, ethanol or water with a base such as litium hydroxide or sodium hydroxide or by acidic hydrolysis in trifluoroacetic acid, hydrochloric acid or dioxane/HCI, thus yielding to compounds of formula (Ic).

Moreover, primary amine derivatives of general formula (Id) may be prepared by Curtius rearrangement of the acid derivatives of general formula (Ic) using an azide such as sodium azide, diphenylphosphoryl azide (DPPA), etc, in acidic media such as sulphuric acid or basic media such as triethylamine, in solvent such as toluene, chloroform, THF, etc. or in a protic solvent such as *tert*-butanol or benzyl alcohol to yield the *tert*-butylcarbonyl (BOC) or benzyloxycarbonyl (CBZ or Z) protected amine and subsequent deprotection as known in the art.

In the particular case where R_{c} is -(CH₂)₂-NR'R", general compounds of formula (If) may be obtained following the synthetic path shown in Figure 10

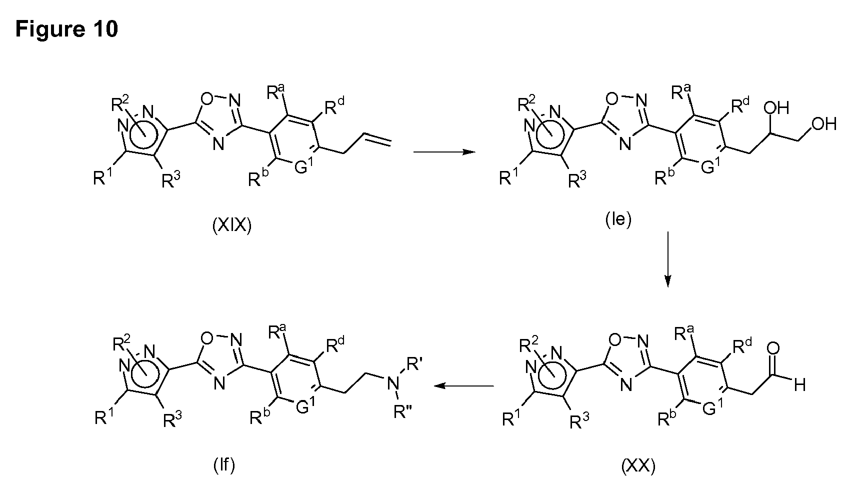

The compounds of general formula (If) may be prepared by the reductive amination of the aldehyde derivatives of general formula (XX) with the corresponding amine or amino acid in acid media such as acetic acid, in a protic solvent such as methanol or ethanol and with a reductive agent such as sodium borohydride, sodium triacetoxyborohydride or sodium cyanoborohydride at a temperature from 0°C to the boiling point of the solvent.

Intermediates of formula (XX) may be obtained by oxidation of diols of general formula (Ie) with an oxidative reagent such as sodium periodate, sodium perchlorate, potassium periodate, etc. in a solvent such as methanol, ethanol, THF, dioxane, ether, acetone, optionally with the presence of water.

Diol derivatives of general formula (Ie) may be prepared by oxidation of the allyl derivatives of general formula (XIX) using a catalytic amount of an oxidazing agent such as osmium tetroxide and a cooxidant such as N-methylmorpholine-N-oxide in a solvent such as methanol, ethanol, THF, dioxane, ether, acetone, optionally with the presence of water.

Allyl derivatives of general formula (XIX) may be prepared by the condensation of the corresponding carboximidamide of formula (IIIc) with the corresponding acid of formula (IIa) as described in Figure 11 and following the same procedure described before (Figure 4) for preparing compounds of formula (Ia) from intermediates of formula (IIa) and Intermediates of formula (IIIa).

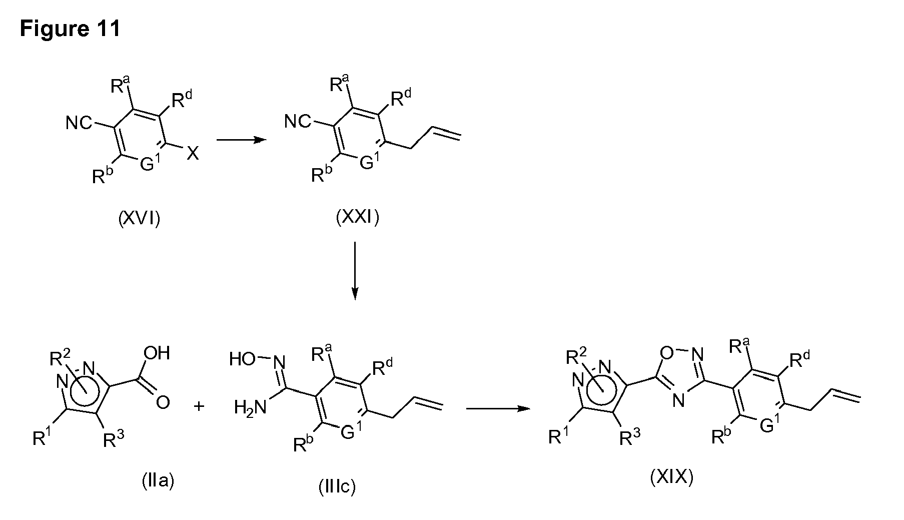

Intermediates of formula (IIIc) may be obtained by standard Stille reaction of the corresponding precursor (XVI) wherein X represents a triflate group, a bromine atom, an iodine atom or a chlorine atom using an allylstannane and a catalyst such as tetrakis(triphenylphosphine)-palladium(0), palladium acetate, bis(triphenylphosphine)palladium(II) chloride or tris(dibenzylideneacetone)-dipalladium(0), in a solvent such dimethylformamide, acetonitrile, and subsequent reaction with hydroxylamine as described before.

Alternatively, intermediates of general formula (XXII) may be obtained as shown in figure 12 and following the same procedure described before (Figure 4) for preparing compounds of formula (Ia) from intermediates of formula (IIa) and Intermediates of formula (IIIa).

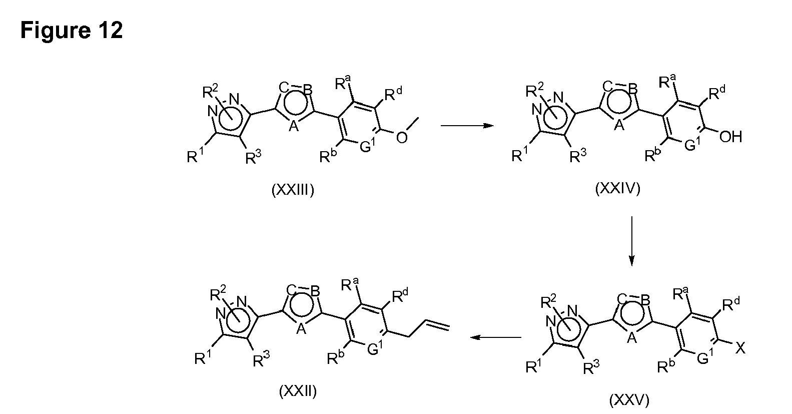

Intermediates of formula (XXII) may be obtained by standard Stille reaction of the corresponding precursor (XXV) wherein X represents a triflate group, a bromine atom, an iodine atom or a chlorine atom, using an allylstannane and a catalyst such as tetrakis(triphenylphosphine)-palladium(0), palladium acetate, bis(triphenylphosphine)palladium(II) chloride or tris(dibenzylideneacetone)-dipalladium(0), in a solvent such dimethylformamide, acetonitrile, and subsequent reaction with hydroxylamine as described before.

Intermediates of general formula (XXV) may be obtained from phenols of general formula (XXIV) by several alternative procedures. In the case in which X is a triflate the reaction is performed by the use of a triflating agent such as triflic anhydride or N-phenyltrifluoromethanesulfonimide. In the particular case in which X is chlorine or bromine the reaction can be performed by using POCl₃, POCl₃/PCl₅ or POBr₃/ PBr₃.

The compounds of general formula (XXIV) may be prepared by demethylation of the corresponding compound of general formula (XXIII) using BBr₃ or AlBr₃ or BF₃ or iodotrimethylsilane as demethylating agent in a solvent such as dichloromethane or 1,2-dichloroethane, chloroform at a temperature between 0 and the 60°C. Alternatively compounds of general formula (XXIV) may be prepared by demethylation using HBr in acetic acid as a solvent.

Finally, compounds of general formula (XXIII) may be prepared according to the general procedures described in figures 1 and 2 for compounds of general formula (I). Alternatively, the compounds of general formula (If) may be prepared by condensation of the carboxylic acid derivatives of formula (IIa) with the corresponding carboximidamide derivative of formula (IIId) as shown in Figure 13 and following the same procedure described before (Figure 4) for preparing compounds of formula (Ia) from intermediates of formula (IIa) and Intermediates of formula (IIIa).

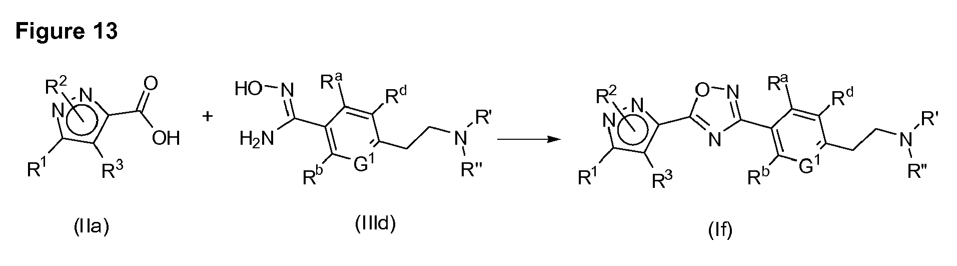

Intermediates of formula (IIId) may be obtained as described in figure 14.

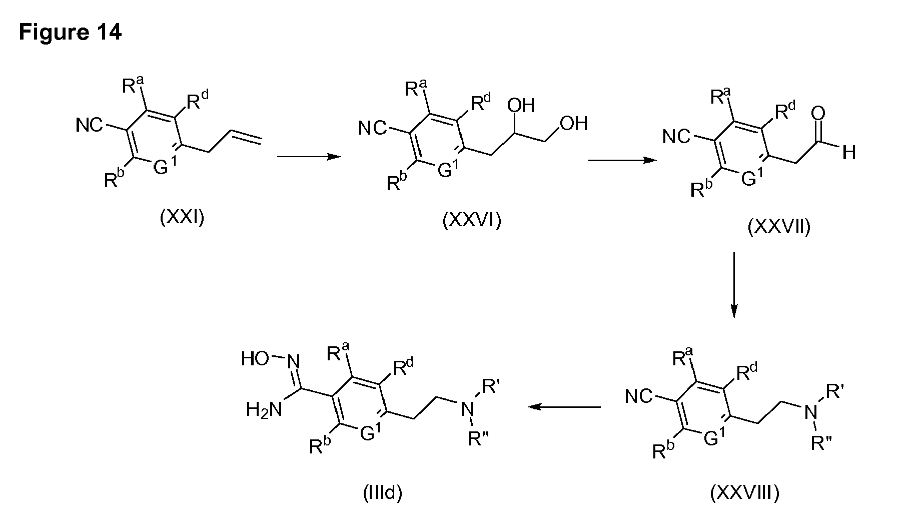

Intermediates of formula (IIId) may be prepared by reductive amination of the aldehyde derivatives of general formula (XXVII) with the corresponding amine or amino acid to give intermediates of formula (XXVIII), followed by reaction with hydroxylamine as described before.

Intermediates of formula (XXVII) may be obtained by oxidation of diols of general formula (XXVI) with an oxidative reagent such as sodium periodate, sodium perchlorate, potassium periodate, etc. in a solvent such as methanol, ethanol, THF, dioxane, ether, acetone, optionally with the presence of water.

Diol derivatives of general formula (XXVI) may be prepared by oxidation of the allyl derivatives of general formula (XXI) using a catalytic amount of an oxidazing agent such as osmium tetroxide and a cooxidant such as N-methylmorpholine-N-oxide in a solvent such as methanol, ethanol, THF, dioxane, ether, acetone, optionally with the presence of water.

In the particular case were R^{c} is -O-(CH₂)₂-NR'R" compounds of general formula (Ig) may be obtained as shown in figure 15.

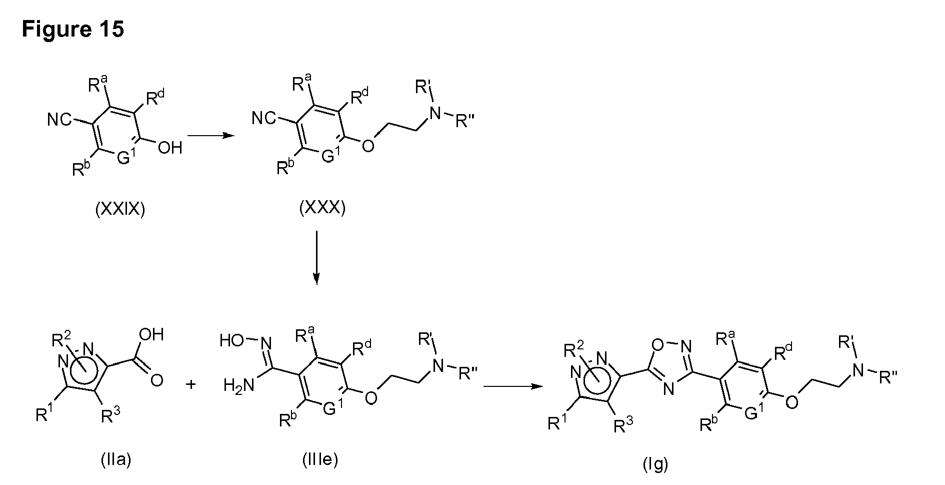

Final compounds of formula (Ig) may be prepared by the condensation of carboxylic acid derivatives of formula (IIa) with the corresponding carboximidamide derivatives of formula (IIIe) in a solvent such as DMF, NMP, THF, in the presence of one or more coupling agents such as 2-(1H-benzotriazole-1-yl)-1,2,3,3-tetramethyluronium tetrafluoroborate, N-(3-dimethylaminopropyl)-N'-ethyl-carbodiimide, dicyclohexyl carbodiimide, 1-hydroxybenzotriazole, O-(benzotriazol-1-yl)-N,N,N',N'-tetramethyl uronium hexafluorophosphate, carbonyl diimidazole, and optionally in the presence of a base such as triethylamine, Hünig's base, sodium hydride, potassium carbonate, at a temperature between 40 and 150°C and in a standard reactor.

Compounds of general formula (IIIe) may be obtained by the reaction of hydroxylamine hydrochloride or any of its salts with the corresponding nitrile (XXX) in basic media such as sodium bicarbonate or triethylamine in a solvent such as THF, methanol or ethanol and at a temperature from 40 to 100°C.

The compounds of formula (XXX) may be obtained by the reaction of compounds of general formula (XXIX) with the corresponding alkylating agent in basic media such as sodium hydride in a solvent such as THF or DMF at a temperature from 0 to 150°C. Alternatively, the phenolic functionality of (XXIX) may be coupled to suitable alcohol derivatives using a Mitsunobu coupling procedure (Mitsunobu, O., Synthesis 1 (1981)). Preferred coupling conditions include the use of a trialkylphosphine or triarylphosphine, such as tri-n-butylphosphine or triphenylphosphine (PPh₃), in a suitable solvent, such as THF or dichloromethane, and an azodicarbonyl reagent, such as diethyl azodicarboxylate or 1,1'-(azodicarbonyl)dipiperidine.

Compounds of general formula (XXIX) are commercial available or may be prepared according to known procedures.

Starting compounds are commercially available or may be obtained following the conventional synthetic methods already known in the art.

Depending on the nature of the functionalities present in the residue R^{c}, these functionalities may require temporary protection. Appropriate protecting groups are known in the art and include e.g a *tert*-butyl or ethyl or methyl to protect an acid, a *tert-*butyloxycarbonyl (BOC) to protect an amine, etc. These protecting groups may be employed according to standard methodology (e.g. T.W. Greene, P. G. M. Wuts, Protective Groups in Organic Synthesis, 3rd Edition, Wiley New York, 1991).

The syntheses of the compounds of the invention are illustrated by the following Examples (1 to 62) including Preparation Examples (1 to 189) which do not limit the scope of the invention in any way.

¹H Nuclear Magnetic Resonance Spectra were recorded on a Varian Mercury 200 spectrometer. Low Resolution Mass Spectra (m/z) were recorded on a Micromass ZMD mass spectrometer using ESI ionization. The chromatographic separations were obtained using a Waters 2690 system equipped with a Symmetry C18 (2.1 x 50 mm, 3.5 µM) column for method A and B and a Symmetry C18 (2.1 x 100 mm, 3.5 µM) for method C. The mobile phase was formic acid (0.4 mL), ammonia (0.1 mL), methanol (500 mL) and acetonitrile (500 mL) (B) and formic acid (0.46 mL), ammonia (0.115 mL) and water (1000 mL) (A), the gradients are specified in the following table for each methode used. The reequilibration time between two injections was 1 min. The flow rate was 0.8 mL/min for method A and 0.4 mL/min for method B, C and D. The injection volume was 5 microliter for method A, B and D and 3 microliter for method C. Diode array chromatograms were collected at 210 nM.

| | 0% B | 0 to 95% B | 95% B |
|---|---|---|---|
| A | 0.2 min | 3 min | 0.8 min |
| B | 0.5 min | 6.5 min | 1 min |
| C | 0 min | 20 min | 4 min |
| D | 0 min | 10.5 min | 1.5 min |

### General Purification Method:

The solid was dissolved in DMSO/MeOH, injected into a Biotage C18 silica column (40M, 25M or 25S according to the crude amount) and eluted on the SP1® automated purification system from Biotage. The gradient used was H₂O/Acetonitrile/MeOH (1:1) (0.1% v/v HCOONH₄ both phases) from 0% to 100% acetonitrile/MeOH (1:1) in 80 column volumes. The appropriate fractions were collected and the organic solvent evaporated under reduced pressure or liofilized.

### GENERAL SYNTHETIC METHODS:

### General Method 1:

A mixture of the corresponding benzonitrile (39.2 mmol) in methanol (50 mL), hydroxylamine hydrochloride (5.45 g, 78.4 mmol) and sodium bicarbonate (13.17 g, 156.8 mmol) was stirred at 75°C for 6 h. The mixture was filtered off and the filtrate evaporated to dryness to yield the title compound (75-100% yield) as a white solid.

### General Method 2:

A mixture of the corresponding acid derivative (1.13 mmol), EDC (1.13 mmol) and HOBt (1.13 mmol) in DMF (5 mL) was stirred at room temperature for 10 min. Then the corresponding benzimidamide (1.13 mmol) was added and the mixture stirred at 140°C for 4 h. The reaction mixture was poured over basic ice/water and the solid formed filtered and washed with water, dried in a vacuum oven to give the desired compound (yield=10-90%) as a white solid.

### General Method 3:

To a mixture of the corresponding methyl or ethyl ester (0.67 mmol) in methanol or ethanol (3 mL) respectively a 2M solution of aqueous NaOH (12 mmol) was added and the reaction mixture stirred at 90°C overnight. The organic solvent was evaporated, water was added and the mixture extracted with diethyl ether. The pH of the aqueous layer was adjusted to 5-6 and the solid formed filtered and dried in the vacuum oven. The desired compound (60-95% yield) was obtained as a white solid.

### General Method 4:

A mixture of the corresponding *tert*-butyl ester (0.56 mmol) in 4M HCl in dioxane (3.5 mL) was stirred at room temperature overnight. The solvent was evaporated and the solid obtained washed with diisopropyl ether twice. The solid was dried in the vacuum oven to yield (70-95% yield) of the desired compound.

### General Method 5:

A mixture of the corresponding acid (0.46 mmol), EDC (133 mg, 0.69 mmol), HOBt (94 mg, 0.69 mmol) and 32% solution of aqueous ammonia (85 µL, 0.69 mmol) in DMF (6.5 mL) was stirred overnight at room temperature. Then ethyl acetate and water were added and the organic layer separated, washed with 4% NaHCO₃, water and brine and dried to give the title compound (5-77% yield) as a white solid.

### General Method 6:

To a solution of the corresponding aldhehyde (200 mg, 0,46 mmol) in methanol (10 mL) the corresponding amine (44 µL, 0,55 mmol) and acetic acid (236 µL, 4,14 mmol) were added and the mixture was stirred at room temperature for 30 min. Then NaBH₃CN (15 mg, 0.23 mmol) was added and the reaction mixture stirred at r.t. overnight. Solvent was concentrated and the residue dissolved in ethyl acetate, washed with water and brine, dried over magnesium sulphate, filtered and concentrated. The crude obtained was purified according to the General Purification Method to give the desired compound (25-85% yield).

### General Method 7:

A mixture of the corresponding acid derivative (0.77 mmol), DPPA (189 µL, 0.88 mmol) and triethylamine (235 µL, 0.51 mmol) in *tert*-butanol (4 mL) was stirred at 100°C overnight. Ethyl acetate was added and the organic layer washed with 4% NaHCO₃ and brine, dried and concentrated. The residue was redissolved in 4M HCl in dioxane (10 mL) and the mixture stirred overnight at room temperature. The reaction mixture was concentrated and the residue purified according to General Purification Method. The solid obtained was redissolved in 4M HCl in dioxane and stirred for 2 h at r.t. Then the solvent was concentrated and the product crystallized in diethyl ether. The title compound was obtained (5-40% yield) as hydrochloride salt.

### General Method 8:

To a solution of the corresponding amide (75 mg, 0.18 mmol) in tetrahydrofurane (4 mL) was added BH₃.SMe₂ (107 µL, 0.21 mmol) dropwise. The reaction mixture was stirred overnight at 65°C. Solvent was concentrated and ethyl acetate was added. The organic layer was washed with 4% NaHCO₃ and brine, dried and concentrated. The residue was purified according to General Purification Method. The solid obtained was redissolved in 5N HCl in dioxane and stirred for 2 h at room temperature Then the solvent was concentrated and the product crystallized in diethyl ether. The title compound was obtained (20-65% yield) as hydrochloride salt.

### PREPARATION EXAMPLES

### Preparation 1

### 4-cyano-2,6-dimethylphenyl trifluoromethanesulfonate

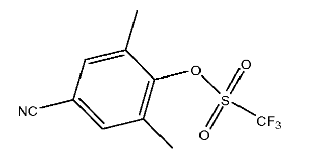

To a suspension of 4-hydroxy-3,5-dimethylbenzonitrile (5.10 g, 34.65 mmol) in dichloromethane (100 mL) triethylamine (7.25 mL, 52.02 mmol) was added. To the solution obtained 1,1,1-trifluoro-N-phenyl-N-(trifluoromethylsulfonyl)methansulfonamide (14.9 g, 41.7 mmol) was added and the mixture stirred overnight at r.t. More dichloromethane was added and then washed with 0.5N NaOH, water and brine. The organic layer was dried over magnesium sulphate and concentrated to yield the desired compound as a solid (100% yield).
LRMS: no signal
Retention time: 6.90 min (Method B)

### Preparation 2

### 4-allyl-N'-hydroxy-3,5-dimethylbenzimidamide

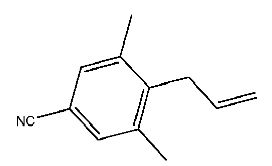

To a solution of Preparation 1 (5 g, 14.74 mmol) in DMF (175 mL) under nitrogen atmosphere, allyltributylstannane (5.50 mL, 17.74 mmol) and Pd(PPh₃)₄ (1.71 g, 1.48 mmol) were added and the mixture stirred overnight at 90°C. The reaction mixture was poured onto ice/water and ethyl acetate was added. The mixture was filtered through Decalite and the organic layer separated, washed with water and brine, dried over magnesium sulphate and concentrated. The crude obtained was used without further purification (80% yield).
LRMS: no signal
Retention time: 6.69 min (Method B)

### Preparation 3

### 4-allyl-N'-hydroxy-3,5-dimethylbenzimidamide

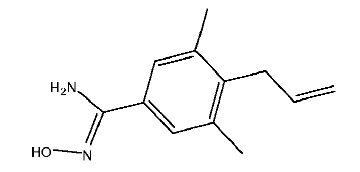

Obtained (69% yield) from Preparation 2 following General Method 1.
LRMS: m/z 205 (M+H)⁺
Retention time: 4.07 min (Method B)

### Preparation 4

### 3-(4-allyl-3,5-dimethylphenyl)-5-(1-methyl-5-phenyl-1H-pyrazol-3-yl)-1,2,4-oxadiazole

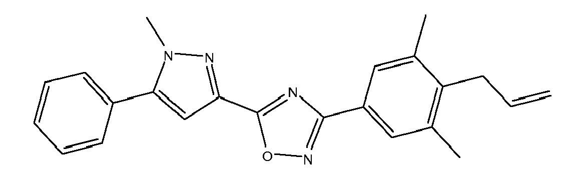

Obtained (56% yield) from 1-methyl-5-phenyl-1H-pyrazole-3-carboxylic acid and Preparation 3 following General Method 2.
LRMS: m/z 371 (M+1)⁺
Retention time: 7.85 min (Method B)

### Preparation 5

### 2-(2,6-dimethyl-4-(5-(1-methyl-5-phenyl-1H-pyrazol-3-yl)-1,2,4-oxadiazol-3-yl)phenyl)acetaldehyde

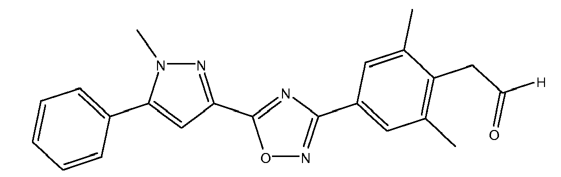

To a suspension of Example 1 (505 mg, 1.25 mmol) in methanol (14 mL) and water (1.6 mL) NalO₄ (401 mg, 1.87 mmol) was added and the mixture stirred overnight at r.t. Methanol was concentrated and the residue dissolved in ethyl acetate and water. Organic layer was separated, washed with water and brine, dried over magnesium sulphate and concentrated to give the title compound (100% yield).
LRMS: m/z 373 (M+1)⁺
Retention time: 9.25 min (Method B)

### Preparation 6

### 3-ethyl-6-methylpyridin-2(1H)-one

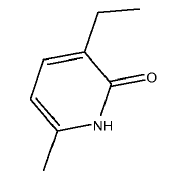

To a solution of 3-ethyl-6-methylpyridin-2-amine (5 g, 36.7 mmol) in sulfuric acid (78 mL, 1.4 mol) at 0°C a solution of sodium nitrite (2.18 g, 31.6 mmol) in water (20 mL) was added dropwise. The reaction mixture was stirred overnight at r.t. The pH was adjusted to 9 by addition of solid sodium hydroxide and the product extracted twice with ethyl acetate. The organic layer was dried and concentrated to give a solid which was recrystallized in cyclohexane to afford the desired compound (64% yield).
LRMS: m/z 138 (M+1)⁺
Retention time: 4.93 min (Method B)
¹H NMR (300 MHz, CHLOROFORM-d) d ppm 1.2 (m, 3 H) 2.30 (s, 3 H) 2.54 (q, *J*=7.23 Hz, 2 H) 5.98 (d, *J*=6.87 Hz, 1 H) 7.18 (d, *J*=6.87 Hz, 1 H)

### Preparation 7

### 5-bromo-3-ethyl-6-methylpyridin-2(1H)-one

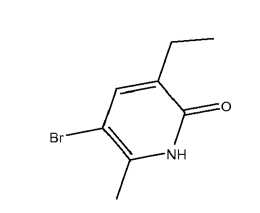

To a solution of Preparation 6 (3.22 g, 23.47 mmol) in methanol (85 mL) at 0°C N-bromosuccinimide (4.39 g, 24.67 mmol) was added and the reaction mixture was stirred at r.t. overnight. Water was added and the solid obtained filtered. The filtrate was concentrated partially, cooled and the solid formed also filtered and mixed with the previous one (78% yield).
LRMS: m/z 218 (M+1)⁺
Retention time: 6.07 min (Method B)
¹H NMR (300 MHz, CHLOROFORM-d) d ppm 1.19 (m, 3 H) 2.40 (s, 3 H) 2.53 (m, 2 H) 7.32 (s, 1 H).

### Preparation 8

### 3-bromo-5-ethyl-6-methoxy-2-methylpyridine

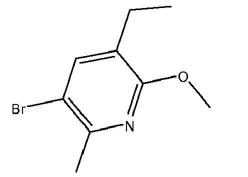

To a mixture of Preparation 7 (2.89 g, 13.37 mmol) and Ag₂CO₃ (4.98 g, 18.05 mmol) in DCM (100 mL) under nitrogen atmosphere methyl iodide (4.83 mL, 77.5 mmol) was added and the mixture stirred at r.t. overnight. The reaction mixture was then filtered over Celite and concentrated under reduced pressure to yield the final compound (75% yield).
LRMS: m/z 232 (M+1)⁺
Retention time: 7.50 min (Method B)
¹H NMR (300 MHz, CHLOROFORM-*d*) d ppm 1.17 (m, 3 H) 2.50 (m, 5 H) 3.91 (s, 3 H) 7.44 (s, 1 H).

### Preparation 9

### 5-ethyl-6-methoxy-2-methylnicotinonitrile

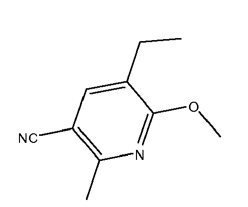

In a microwave oven vessel Preparation 8 (2.3 g, 10 mmol) was dissolved in DMF (30 mL) and dicyanozinc (1.18 g, 10.05 mmol) and Pd(PPh₃)₄ (1.73 g, 1.5 mmol) were added. The mixture was heated under nitrogen atmosphere in a Biotage Initiator device at 180°C and normal absorvance for 30 min. The mixture was poured over ethyl acetate/water and the organic layer washed with water, brine, dried over magnesium sulphate and concentrated. The residue obtained was purified by column chromatography with a mixture of ethyl acetate in hexane from 0 to 5% (88% yield).
LRMS: m/z 177 (M+1)⁺
Retention time: 7.12 min (Method B)
¹H NMR (300 MHz, CHLOROFORM-*d*) d ppm 1.18 (t, *J*=7.55 Hz, 3 H) 2.56 (q, *J*=7.60 Hz, 2 H) 2.62 (s, 3 H) 3.99 (s, 3 H) 7.50 (s, 1 H).

### Preparation 10

### 5-ethyl-N'-hydroxy-6-methoxy-2-methylnicotinimidamide

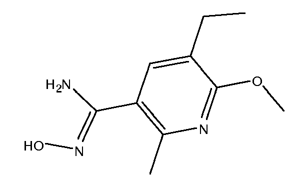

Obtained (32% yield) from Preparation 9 following the General Method 1.
LRMS: m/z 210 (M+1)⁺
Retention time: 4.20 min (Method B)

### Preparation 11

### 3-(5-ethyl-6-methoxy-2-methylpyridin-3-yl)-5-(1-methyl-5-phenyl-1H-pyrazol-3-yl)-1,2,4-oxadiazole

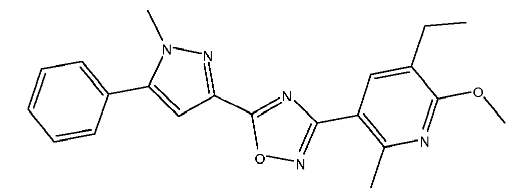

Obtained (58% yield) from 1-methyl-5-phenyl-1H-pyrazole-3-carboxylic acid and Preparation 10 following General Method 2.
LRMS: m/z 376 (M+1)⁺
Retention time: 7.87 min (Method B)

### Preparation 12

### 4-bromo-2-ethyl-6-methylphenol

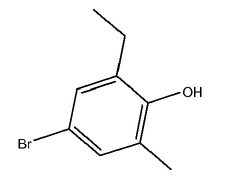

To a solution of 2-ethyl-6-methylphenol (5 g, 36.7 mmol) in chloroform (52 mL) bromine (1.88 mL, 37 mmol) was added dissolved in chloroform (2 mL) and reaction was stirred at r.t. for 3 h. Then NaHSO₃ 40% p/v (50 mL) was added and mixture stirred for 30 min, organic layer washed with water, dried over sodium sulphate and concentrated. The oil thus obtained was recristallized with cold hexane to yield the title compound (70%) as a white solid.
LRMS: m/z 216 (M+1)⁺
Retention time: 6.47 min (Method B)
¹H NMR (300 MHz, CHLOROFORM-d) δ ppm 1.23 (t, *J*=7.55 Hz, 3 H) 2.22 (s, 3 H) 2.59 (q, *J*=7.42 Hz, 2 H) 7.11 (s, 2 H)

### Preparation 13

### 3-Ethyl-4-hydroxy-5-methylbenzonitrile

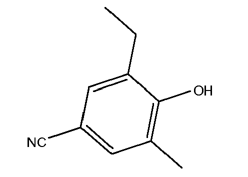

Obtained (78% yield) from Preparation 12 following the procedure described in Preparation 9.
LRMS: m/z 162 (M+1)⁺
Retention time: 6.57 min (Method D)
¹H NMR (300 MHz, CHLOROFORM-d) δ ppm 1.24 (t, *J*=7.42 Hz, 3 H) 2.27 (s, 3 H) 2.64 (q, *J*=7.51 Hz, 2 H) 5.55 (s, 1 H) 7.30 (s, 2 H)

### Preparation 14

### 4-cyano-2-ethyl-6-methylphenyl trifluoromethanesulfonate

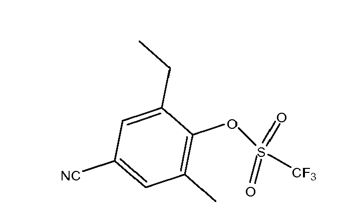

Obtained (90% yield) from Preparation 13 following the procedure described in Preparation 1.
LRMS: no signal
Retention time: 6.95 min (Method C)
¹H NMR (300 MHz, CHLOROFORM-d) δ ppm 1.27 (t, *J*=7.55 Hz, 3 H) 2.44 (s, 3 H) 2.81 (q, *J*=7.69 Hz, 2 H) 7.34 - 7.67 (m, 2 H)

### Preparation 15

### Ethyl 3-(4-cyano-2-ethyl-6-methyl phenyl)acrylate

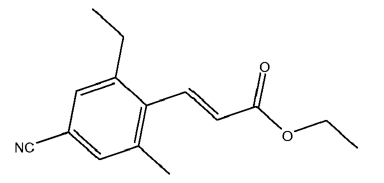

To a mixture of Preparation 14 (14.4 g, 49.1 mmol), ethyl acrylate (26.8 mL, 245 mmol), 1,1'-Bis(diphenylphosphino)ferrocene (1.22 g, 2.96 mmol) and triethylamine (16.4 mL, 117.9 mmol) in DMF (106 mL), palladium acetate (0.66 g, 2.94 mmol) was added under nitrogen atmosphere. The reaction mixture was stirred overnight at 80°C. After cooling to room temperature, the mixture was poured onto water and extracted with diethyl ether twice. The combined organic layer was washed with water and brine, dried over magnesium sulphate and concentrated. A brown oil was obtained (44% yield) as the desired compound.
LRMS: m/z 244 (M+H)⁺
Retention time: 8.52 min (Method D)

### Preparation 16

### Ethyl 3-(2-ethyl-4-(N'-hydroxycarbamimidoyl)-6-methylphenyl)acrylate

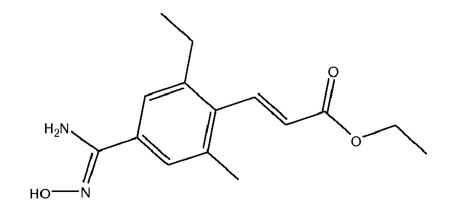

Obtained (60% yield) from Preparation 15 following General Method 1.
LRMS: m/z 277 (M+H)⁺
Retention time: 4.95 min (Method D)

### Preparation 17

### Ethyl 3-(2-ethyl-4-(N'-hydroxycarbamimidoyl)-6-methylphenyl)propanoate

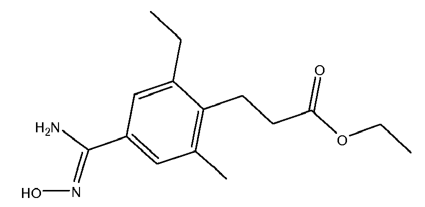

Preparation 16 (1.80 g, 6.51 mmol) was dissolved in ethanol (40 mL) and sodium acetate (0.80 g, 9.75 mmol) was added. Finally palladium chloride (0.23 g, 1.30 mmol) was added and the mixture hydrogenated overnight. The catalyst was filtered off and the filtrate concentrated. The residue was redissolved in dichloromethane and washed with water. The organic layer was dried over magnesium sulphate and concentrated to yield the title compound (50% yield).
LRMS: m/z 279 (M+H)⁺
Retention time: 4.63 min (Method D)
¹H NMR (300 MHz, CHLOROFORM-d) δ ppm 1.04 - 1.43 (m, 6 H) 2.36 (s, 3 H) 2.44 (dd, *J*=8.65, 7.83 Hz, 2 H) 2.68 (q, *J*=7.51 Hz, 2 H) 2.88 - 3.11 (m, 2 H) 4.02 - 4.28 (m, *J*=7.14, 7.14, 6.18, 0.96 Hz, 2 H) 4.87 (br. s., 2 H) 7.29 (d, *J*=9.89 Hz, 2 H).

### Preparation 18

### Ethyl 3-(2-ethyl-6-methyl-4-(5-(1-methyl-5-phenyl-1H-pyrazol-3-yl)-1,2,4-oxadiazol-3-yl)phenyl)propanoate

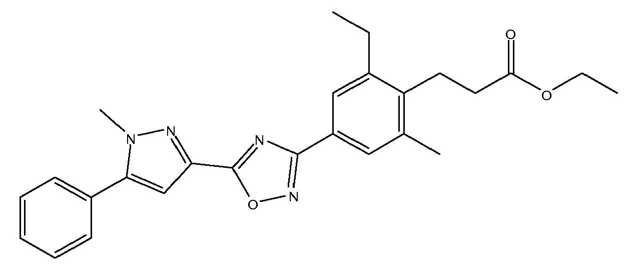

Obtained (57% yield) from 1-methyl-5-phenyl-1H-pyrazole-3-carboxylic acid and Preparation 17 following General Method 2.
LRMS: m/z 445 (M+1)⁺
Retention time: 7.72 min (Method B)

### Preparation 19

### tert-butyl 3-(3-ethyl-6-methyl-5-(5-(1-methyl-5-phenyl-1H-pyrazol-3-yl)-1,2,4-oxadiazol-3-yl)pyridin-2-yloxy)propylcarbamate

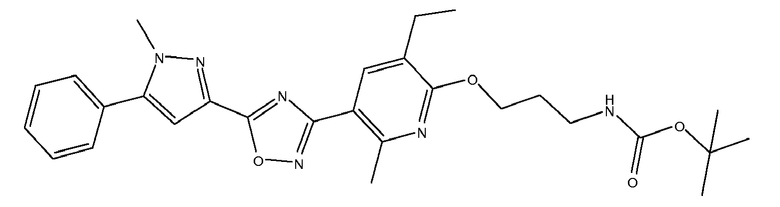

In a microwave vial Example 5 (130 mg, 0.36 mmol), 2-(*tert*-butoxymethylamino) ethanol (107 mg, 0.61 mmol), triphenylphosphine (136 mg, 0.52 mmol) and DIAD (102 µL, 0.50 mmol) were dissolved in anhydrous THF (2 mL). Mixture was stirred at 80°C for 1 h and then solvent was removed, crude redissolved in dichloromethane, washed with water and brine, dried and concentrated. The residue was purified according to General Purification method to yield the title compound (95% yield).
LRMS: m/z 519 (M+1)⁺
Retention time: 7.88 min (Method B)

### Preparation 20

### N', 4-dihydroxy-3,5-dimethylbenzimidamide

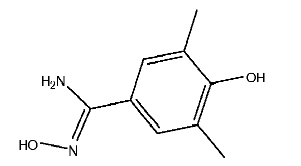

Obtained (75% yield) from 4-hydroxy-3,5-dimethylbenzonitrile following General Method 1.
LRMS: m/z 181 (M+1)⁺
Retention time: 1.80 min (Method B)
¹H NMR (300 MHz, DMSO-d₆) δ ppm 2.21 (s, 6 H) 5.66 (br. s., 2 H) 7.28 (s, 2 H) 8.47 (s, 1 H) 9.36 (s, 1 H).

### Preparation 21

### 2,6-dimethyl-4-(5-(1-methyl-5-phenyl-1H-pyrazol-3-yl)-1,2,4-oxadiazol-3-yl)phenol

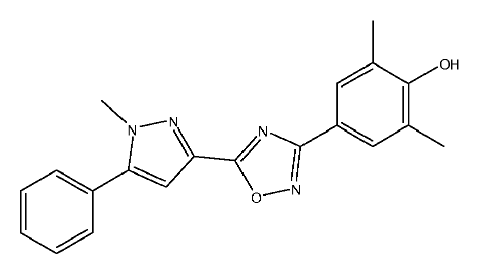

Obtained (34% yield) from 1-methyl-5-phenyl-1H-pyrazole-3-carboxylic acid and Preparation 20 following General Method 2.
LRMS: m/z 490 (M+1)⁺
Retention time: 7.57 min (Method B)

### Preparation 22

### tert-butyl 2-(2,6-dimethyl-4-(5-(1-methyl-5-phenyl-1H-pyrazol-3-yl)-1,2,4-oxadiazol-3-yl)phenoxy)ethylcarbamate

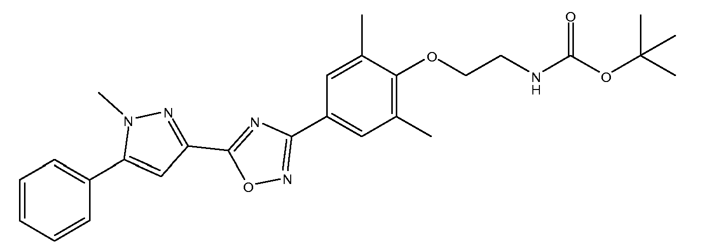

Obtained (82% yield) from Preparation 21 and *tert*-butyl 2-hydroxyethylcarbamate following the procedure described in Preparation 19.
LRMS: m/z 490 (M+1)⁺
Retention time: 7.57 min (Method B)

### Preparation 23

### Ethyl 2-(2,6-dimethyl-4-(5-(1-methyl-5-phenyl-1H-pyrazol-3-yl)-1,2,4-oxadiazol-3-yl)phenoxy)-2,2-difluoroacetate

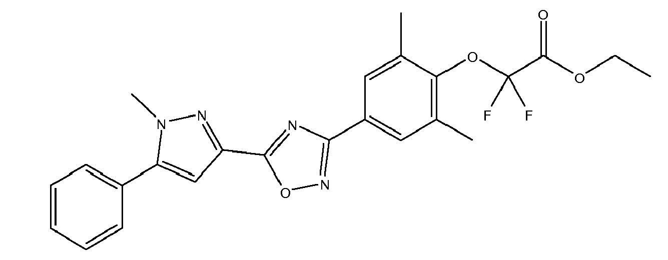

To a suspension of NaH (60%) (229 mg, 1.91 mmol) in DMF (10 mL) under nitrogen atmosphere a solution of Preparation 21 (660 mg, 1.91 mmol) in DMF (8 mL) was added. The mixture was stirred at room temperature for 30 min and then ethyl 2-bromo-2,2-difluoroacetate (586 µL, 3.81 mmol) in DMF (8 mL) was added and the reaction mixture stirred overnight at r.t. Solvent was removed and the residue was dissolved in ethyl acetate, washed with water and brine, dried over magnesium sulphate and concentrated. The crude was purified according to the General Purification Method to give the desired compound (10% yield).
LRMS: m/z 469 (M+1)⁺
Retention time: 7.77 min (Method B)

### Preparation 24

### N',4-dihydroxy-3-(trifluoromethyl)benzimidamide

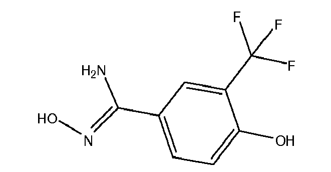

Obtained (100%) from 4-hydroxy-3-(trifluoromethyl)benzonitrile following General Method 1.
LRMS: m/z 221 (M+1)⁺
Retention time: 1.67 min (Method B)

### Preparation 25

### 4-(5-(1-methyl-5-phenyl-1H-pyrazol-3-yl)-1,2,4-oxadiazol-3-yl)-2-(trifluoromethyl)phenol

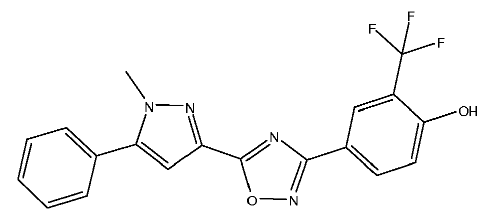

Obtained (30% yield) from 1-methyl-5-phenyl-1H-pyrazole-3-carboxylic acid and Preparation 24 following General Method 2.
LRMS: m/z 387 (M+1)⁺
Retention time: 7.05 min (Method B)

### Preparation 26

### tert-Butyl 2-(4-(5-(1-methyl-5-phenyl-1H-pyrazol-3-yl)-1,2,4-oxadiazol-3-yl)-2-(tri fluoromethyl)phenoxy)ethylcarbamate

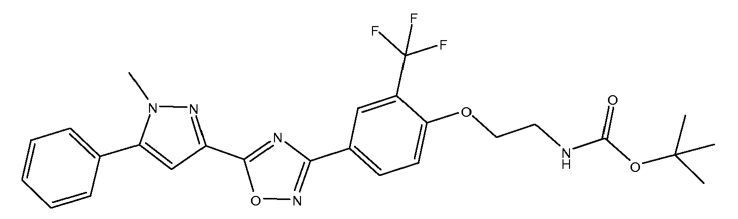

Obtained (94% yield) from Preparation 25 and *tert*-butyl 2-hydroxyethylcarbamate following the procedure described in Preparation 19.
LRMS: m/z 530 (M+1)⁺
Retention time: 7.62 min (Method B)
¹H NMR (300 MHz, METHANOL-d₄) δ ppm 1.51 (s, 9 H) 3.57 (t, *J*=6.45 Hz, 2 H) 4.09 (s, 3 H) 4.31 (t, 2 H) 7.20 (d, *J*=1.92 Hz, 1 H) 7.36 - 7.53 (m, 1 H) 7.52 - 7.76 (m, 4 H) 8.41 (br. s., 2 H).

### Preparation 27

### 3-(4-(2-bromoethoxy)-3,5-dimethylphenyl)-5-(1-methyl-5-phenyl-1H-pyrazol-3-yl)-1,2,4-oxadiazole

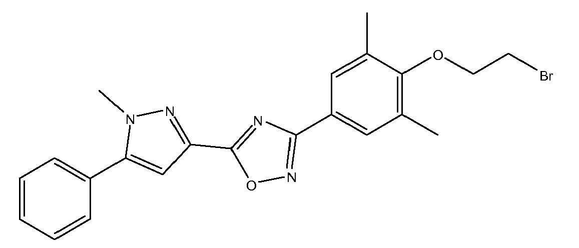

Obtained (72% yield) from Preparation 21 and 2-bromoethanol following the procedure described in Preparation 19.
LRMS: m/z 454 (M+1)⁺
Retention time: 7.68 min (Method B)

### Preparation 28

### Ethyl 2,4-dioxo-4-phenylbutanoate

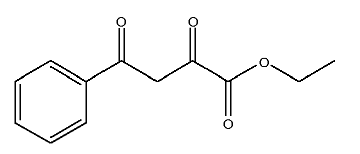

In a three-necked flask acetophenone (5 g, 41.61 mmol) and diethyl oxalate (6.76 mL, 50 mmol) were dissolved in anhydrous DMF (65 mL) at 0°C and NaH 60% (2 g, 83.25 mmol) was slowly added, mixture was stirred at 0°C for 15 min and 30 min more at r.t before heating at 45°C for 1 h. Reaction was poured onto water-acetic acid and product extracted with ethyl acetate. Organic layers were combined, washed with water and brine, dried over magnesium sulphate, filtered and concentrated to yield the title compound as an oil (62% yield).
LRMS: m/z 221 (M+1)⁺
Retention time: 6.30 min (Method B)

### Preparation 29

### 1-Ethyl-5-phenyl-1H-pyrazole-3-carboxylic acid

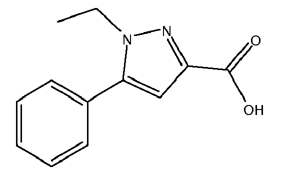

Ethyl hydrazine oxalic salt (2.51 g, 16.72 mmol) was suspended ethanol (30 mL) and triethylamine (2.33 mL, 16.72 mmol) was added. This mixture was dropwise added to a solution of Preparation 28 (3.07 g, 13.94 mmol) in ethanol (110 mL) and it was heated to reflux temperature for 3 h. Solvent was removed, crude redissolved in ethyl acetate and washed with water and brine, dried over magnesium sulphate, filtered and concentrated. Crude was purified by normal phase chromatography using hexane/ethyl acetate from 0 to 25%. Pure fractions were concentrated, solid dissolved in ethanol (35 mL) and 8N NaOH (3 mL) was added and mixture stirred overnight at r.t. Ethanol was removed, suspension was acidified with concentrated HCl and solid filtered, washed with water and dried in the vacuum oven to yield the title compound as a white solid (65% yield).
LRMS: m/z 217 (M+1)⁺
Retention time: 5.30 min (Method B)

### Preparation 30

### 5-(1-Ethyl-5-phenyl-1H-pyrazol-3-yl)-3-(5-ethyl-6-methoxy-2-methylpyridin-3-yl)-1,2,4-oxadiazole

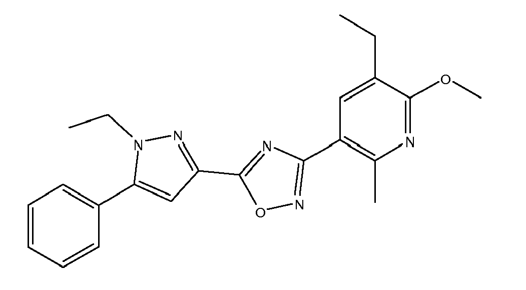

Obtained (45% yield) from Preparation 29 and Preparation 10 following General Method 2.
LRMS: m/z 390 (M+1)⁺
Retention time: 7.95 min (Method B)

### Preparation 31

### 5-(2-chlorophenyl)-1-ethyl-1H-pyrazole-3-carboxylic acid

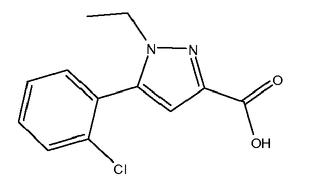

Obtained (82% yield) from Preparation 28 and ethyl hydrazine oxalic salt following the procedure described in Preparation 29.
LRMS: m/z 249 (M-1)⁺
Retention time: 5.57 min (Method B)

### Preparation 32

### 5-(5-(2-chlorophenyl)-1-ethyl-1H-pyrazol-3-yl)-3-(5-ethyl-6-methoxy-2-methylpyridin-3-yl)-1,2,4-oxadiazole

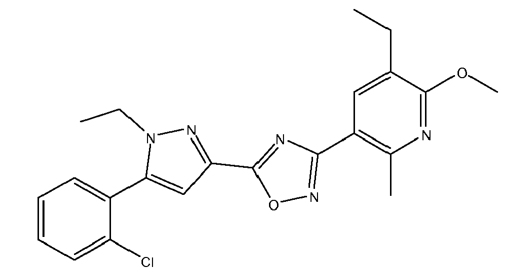

Obtained (31 % yield) from Preparation 31 and Preparation 10 following General Method 2.
LRMS: m/z 423 (M+1)⁺
Retention time: 8.02 min (Method B)

### Preparation 33

### 4-cyano-2-(trifluoromethyl)phenyl trifluoromethanesulfonate

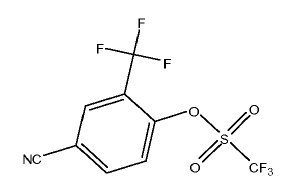

Obtained (72% yield) from 4-hydroxy-3-(trifluoromethyl)benzonitrile following the procedure described in Preparation 1.
LRMS: m/z 320 (M+1)⁺
Retention time: 6.62 min (Method B)

### Preparation 34

### tert-Butyl 3-(4-cyano-2-(trifluoromethyl)phenyl)acrylate

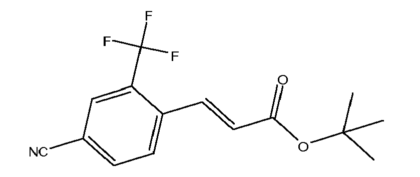

Obtained (60% yield) from Preparation 33 and *tert*-butyl acrylate following the procedure described in Preparation 15 heating at 110°C.
LRMS: no signal
Retention time: 7.08 min (Method B)

### Preparation 35

### tert-Butyl 3-(4-(N'-hydroxycarbamimidoyl)-2-(trifluoromethyl)phenyl)acrylate

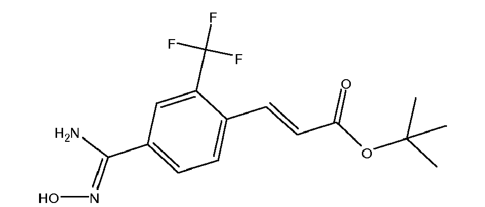

Obtained (100% yield) from Preparation 34 following General Method 1.
LRMS: m/z 331 (M+1)⁺
Retention time: 6.07 min (Method B)

### Preparation 36

### tert-Butyl 3-(4-(N'-hydroxycarbamimidoyl)-2-(trifluoromethyl)phenyl)propanoate

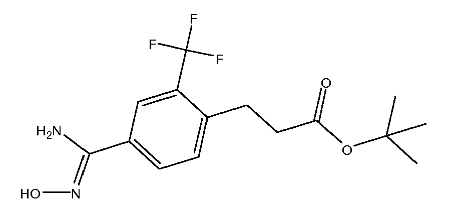

Obtained (58% yield) from Preparation 35 following the procedure described in Preparation 17.
LRMS: m/z 333 (M+1)⁺
Retention time: 5.53 min (Method B)

### Preparation 37

### tert-Butyl 3-(4-(5-(1-methyl-5-phenyl-1H-pyrazol-3-yl)-1,2,4-oxadiazol-3-yl)-2-(trifluoromethyl)phenyl)propanoate

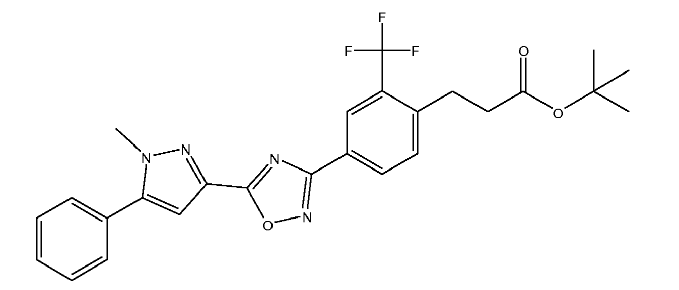

Obtained (42% yield) from 1-methyl-5-phenyl-1H-pyrazole-3-carboxylic acid and Preparation 36 following General Method 2.
LRMS: m/z 499 (M+1)⁺
Retention time: 7.93 min (Method B)

### Preparation 38

### 3-(4-(5-(1-methyl-5-phenyl-1H-pyrazol-3-yl)-1,2,4-oxadiazol-3-yl)-2-(trifluoromethyl)phenyl)propanoic acid

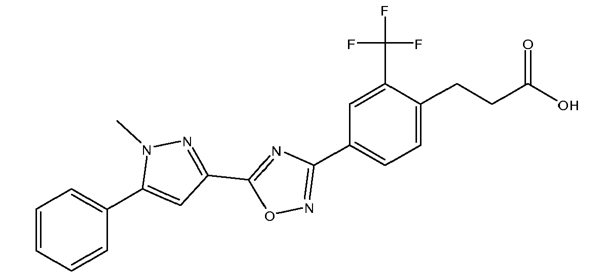

Obtained (78% yield) from Preparation 37 following General Method 4.
LRMS: m/z 443 (M+1)⁺
Retention time: 7.23 min (Method B)

### Preparation 39

### 3-(2-chlorophenyl)-1-ethyl-1H-pyrazole-5-carboxylic acid

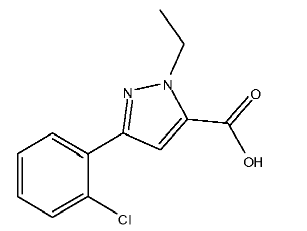

Obtained as a by-product of Preparation 31.
LRMS: m/z 249 (M-1)⁺
Retention time: 6.17 min (Method B)

### Preparation 40

### 5-(3-(2-chlorophenyl)-1-ethyl-1H-pyrazol-5-yl)-3-(5-ethyl-6-methoxy-2-methylpyridin-3-yl)-1,2,4-oxadiazole

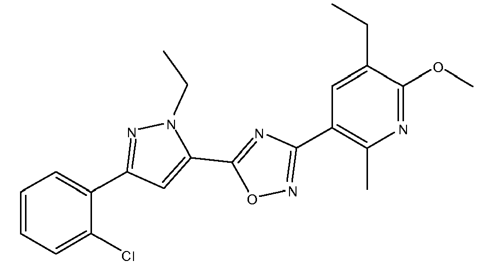

Obtained (40%) from Preparation 39 and Preparation 10 following General Method 2.
LRMS: m/z 424 (M+1)⁺
Retention time: 11.73 min (Method D)

### Preparation 41

### 3-(4-allyl-3,5-dimethylphenyl)-5-(1-ethyl-5-phenyl-1H-pyrazol-3-yl)-1,2,4-oxadiazole

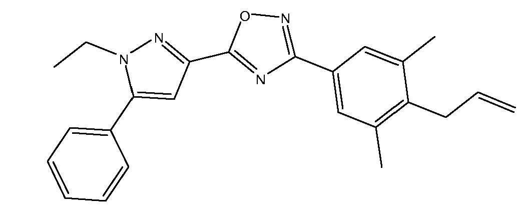

Obtained (80%) from Preparation 29 and Preparation 3 following General Method 2.
LRMS: m/z 385 (M+1)⁺
Retention time: 7.92 min (Method B)

### Preparation 42

### 2-(4-(5-(1-ethyl-5-phenyl-1H-pyrazol-3-yl)-1,2,4-oxadiazol-3-yl)-2,6-dimethylphenyl)acetaldehyde

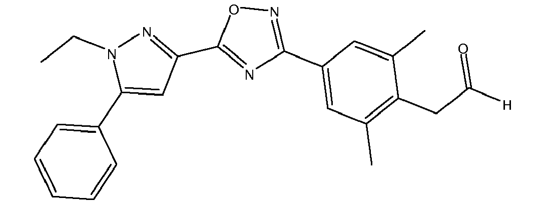

Obtained (70%) from Preparation 41 following the procedure described in Example 1 followed by procedure described in Preparation 5.
LRMS: m/z 387 (M+1)⁺
Retention time: 7.12 min (Method B)

### Preparation 43

### 3-(4-allyl-3,5-dimethylphenyl)-5-(5-(2-chlorophenyl)-1-ethyl-1H-pyrazol-3-yl)-1,2,4-oxadiazole

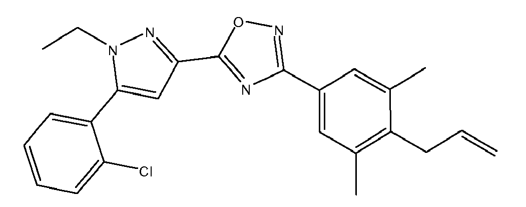

Obtained (70%) from Preparation 31 and Preparation 3 following General Method 2.
LRMS: m/z 419 (M+1)⁺
Retention time: 7.98 min (Method B)

### Preparation 44

### 2-(4-(5-(5-(2-chlorophenyl)-1-ethyl-1H-pyrazol-3-yl)-1,2,4-oxadiazol-3-yl)-2,6-dimethylphenyl)acetaldehyde

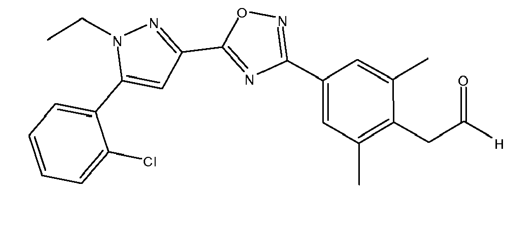

Obtained (66%) from Preparation 43 following the procedure described in Preparation 42.
LRMS: m/z 421 (M+1)⁺
Retention time: 7.32 min (Method B)

### Preparation 45

### 3-(4-allyl-3,5-dimethylphenyl)-5-(5-isopropyl-1H-pyrazol-3-yl)-1,2,4-oxadiazole

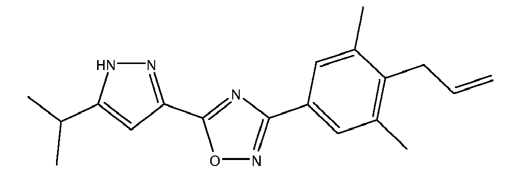

Obtained (50%) from 5-isopropyl-1H-pyrazole-3-carboxylic acid and Preparation 3 following General Method 2.
LRMS: m/z 323 (M+1)⁺
Retention time: 7.65 min (Method B)

### Preparation 46

### 3-(4-allyl-3,5-dimethylphenyl)-5-(1-ethyl-5-isopropyl-1H-pyrazol-3-yl)-1,2,4-oxadiazole

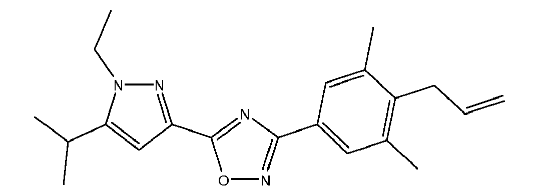

Obtained (82%) from Preparation 45 and iodoethane following the procedure described in Preparation 23 using THF as solvent.
LRMS: m/z 351 (M+1)⁺
Retention time: 7.88 min (Method B)
¹H NMR (400 MHz, CHLOROFORM-d) δ ppm 1.34 (d, *J*=6.64 Hz, 6 H) 1.53 (t, *J*=7.42 Hz, 3 H) 2.36 (s, 6 H) 3.03 (dt, *J*=13.68, 6.84 Hz, 1 H) 3.44 (dt, *J*=5.47, 1.76 Hz, 2 H) 4.26 (q, *J*=7.16 Hz, 2 H) 4.86 (dq, *J*=17.15, 1.84 Hz, 1 H) 5.02 (dq, *J*=10.16, 1.69 Hz, 1 H) 5.91 (dddd, *J*=17.15, 10.40,5.47,5.28 Hz, 1 H) 6.78 (s, 1 H)7.87(s,2H).

### Preparation 47

### 2-(4-(5-(1-ethy)-5-isopropy)-1H-pyrazol-3-yl)-1,2,4-oxadiazol-3-yl)-2,6-dimethylphenyl)acetaldehyde

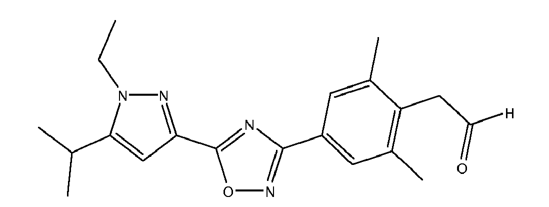

Obtained (74%) from Preparation 46 following the procedure described in Preparation 42.
LRMS: m/z 353 (M+1)⁺
Retention time: 6.53 min (Method B)

### Preparation 48

### Ethyl 4-(3-chlorophenyl)-2,4-dioxobutanoate

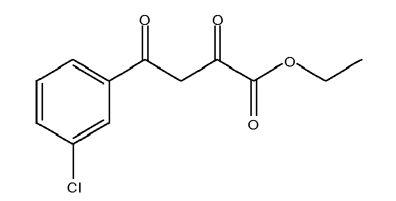

Obtained (99%) from 1-(3-chlorophenyl)ethanone and diethyl oxalate following the procedure described in Preparation 28.
LRMS: m/z 255 (M+1)⁺
Retention time: 6.87 min (Method B)

### Preparation 49

### 5-(3-Chlorophenyl)-1-ethyl-1H-pyrazole-3-carboxylic acid

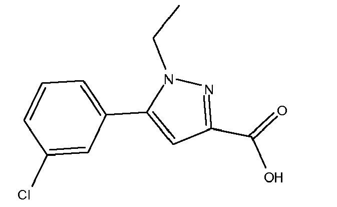

Obtained (38%) from Preparation 48 and ethyl hydrazine oxalic salt following the procedure described in Preparation 29.
LRMS: m/z 251 (M+1)⁺
Retention time: 5.85 min (Method B)
¹H NMR (400 MHz, DMSO*-d*₆) δ ppm 1.32 (t, *J*=7.23 Hz, 3 H) 4.19 (q, *J*=7.42 Hz, 2 H) 7.31 - 7.75 (m, 4 H).

### Preparation 50

### 3-(4-Allyl-3,5-dimethylphenyl)-5-(5-(3-chlorophenyl)-1-ethyl-1H-pyrazol-3-yl)-1,2,4-oxadiazole

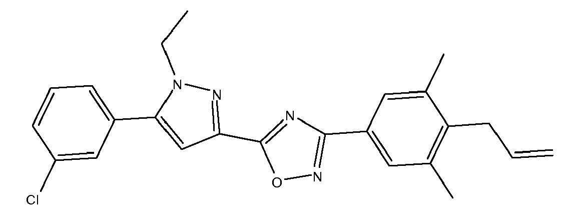

Obtained (59%) from Preparation 49 and Preparation 3 following General Method 2.
LRMS: m/z 419 (M+1)⁺
Retention time: 8.18 min (Method B)

### Preparation 51

### 2-(4-(5-(5-(3-Chlorophenyl)-1-ethyl-1H-pyrazol-3-yl)-1,2,4-oxadiazol-3-yl)-2,6-dimethylphenyl)acetaldehyde

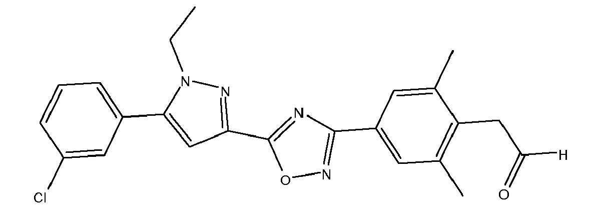

Obtained (86%) from Preparation 50 following the procedure described in Preparation 42.
LRMS: m/z 421 (M+1)⁺
Retention time: 7.52 min (Method B)

### Preparation 52

### 4-Allyl-2-methylbenzonitrile

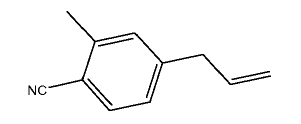

Obtained (63%) from 4-allyl-1-bromo-2-methylbenzene following the procedure described in Preparation 2.
LRMS: No signal
Retention time: 6.51 min (Method B)
¹H NMR (400 MHz, CHLOROFORM*-d*) δ ppm 2.51 (s, 3 H) 3.39 (d, *J*=6.64 Hz, 2 H) 4.93 - 5.30 (m, 2 H) 5.75 - 6.08 (m, 1 H) 7.09 (dd, *J*=7.62, 0.98 Hz, 1 H) 7.14 (s, 1 H) 7.51 (d, *J*=7.82 Hz, 1 H).

### Preparation 53

### 4-allyl-N'-hydroxy-2-methylbenzimidamide

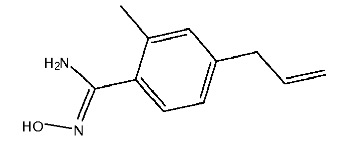

Obtained (38%) from Preparation 52 following General Method 1.
LRMS: m/z 191 (M+1)⁺
Retention time: 3.27 min (Method B)

### Preparation 54

### 3-(4-Allyl-2-methylphenyl)-5-(1-methyl-5-phenyl-1H-pyrazol-3-yl)-1,2,4-oxadiazole

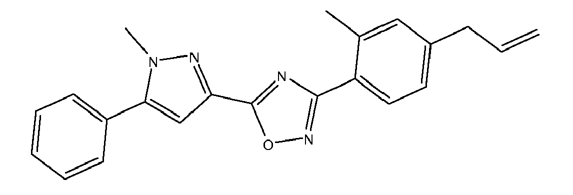

Obtained (58%) from 1-methyl-5-phenyl-1H-pyrazole-3-carboxylic acid and Preparation 53 following General Method 2.
LRMS: m/z 357 (M+1)⁺
Retention time: 7.68 min (Method B)

### Preparation 55

### 2-(3-Methyl-4-(5-(1-methyl-5-phenyl-1H-pyrazol-3-yl)-1,2,4-oxadiazol-3-yl)phenyl)acetaldehyde

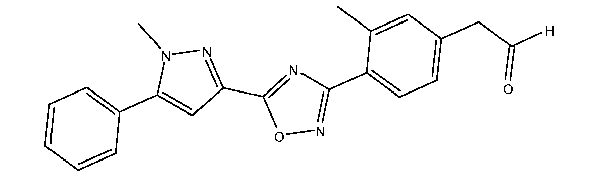

Obtained (90%) from Preparation 54 following the procedure described in Preparation 42.
LRMS: m/z 359 (M+1)⁺
Retention time: 6.80 min (Method B)

### Preparation 56

### Ethyl 4-(3-fluorophenyl)-2,4-dioxobutanoate

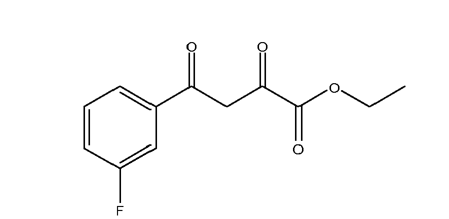

Obtained (68%) from 1-(3-fluorophenyl)ethanone and diethyl oxalate following the procedure described in Preparation 28.
LRMS: m/z 239 (M+1)⁺
Retention time: 6.38 min (Method B)
¹H NMR (400 MHz, CHLOROFORM*-d*) δ ppm 1.42 (t, *J*=7.03 Hz, 3 H) 4.41 (q, *J*=7.16 Hz, 2 H) 7.27-7.37 (m, *J*=8.21, 8.21, 2.54, 0.98 Hz, 1 H) 7.49 (td, *J*=8.01, 5.47 Hz, 1 H)7.69 (dd, *J*=8.99, 2.34 Hz, 1 H)7.78 (dt, *J*=7.82, 1.17 Hz, 1 H).

### Preparation 57

### 1-Ethyl-5-(3-fluorophenyl)-1H-pyrazole-3-carboxylic acid

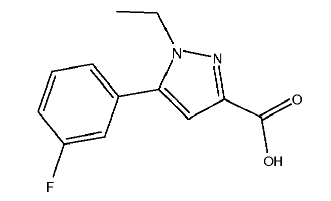

Obtained (55%) from Preparation 56 and ethyl hydrazine oxalic salt following the procedure described in Preparation 29.
LRMS: m/z 235 (M+1)⁺
Retention time: 5.37 min (Method B)

### Preparation 58

### 3-(4-allyl-3,5-dimethylphenyl)-5-(1-ethyl-5-(3-fluorophenyl)-1H-pyrazol-3-yl)-1,2,4-oxadiazole

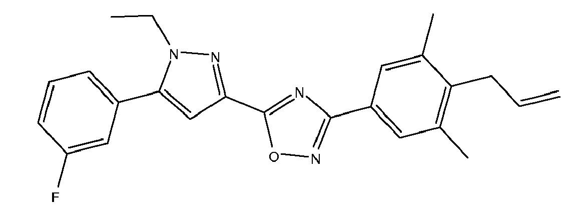

Obtained (54%) from Preparation 57 and Preparation 3 following General Method 2.
LRMS: m/z 403 (M+1)⁺
Retention time: 7.92 min (Method B)

### Preparation 59

### 2-(4-(5-(1-ethyl-5-(3-fluorophenyl)-1H-pyrazol-3-yl)-1,2,4-oxadiazol-3-yl)-2,6-dimethylphenyl)acetaldehyde

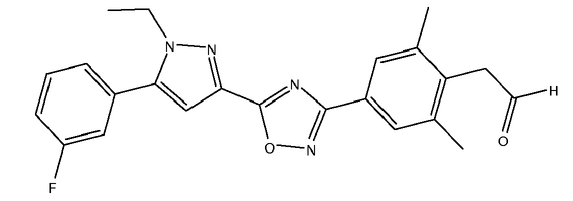

Obtained (70%) from Preparation 58 following the procedure described in Preparation 42.
LRMS: m/z 423 (M+18)⁺
Retention time: 7.25 min (Method B)

### Preparation 60

### 4-Allyl-3-methylbenzonitrile

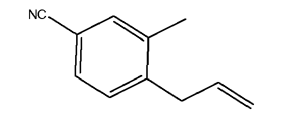

Obtained (100%) from 4-bromo-3-methylbenzonitrile following the procedure described in Preparation 2.
LRMS: No signal
Retention time: 6.45 min (Method B)
¹H NMR (400 MHz, CHLOROFORM*-d*) δ ppm 2.32 (s, 3 H) 3.41 (d, *J*=6.25 Hz, 2 H)4.99 (dq, *J*=17.05, 1.74 Hz, 1 H) 5.13 (dq, *J*=10.01, 1.48 Hz, 1 H) 5.69-6.12 (m, *J*=16.75, 10.21,6.25,6.25 Hz, 1 H) 7.23 (d, *J*=8.21 Hz, 1 H) 7.35-7.52 (m, 2 H).

### Preparation 61

### 4-allyl-N'-hydroxy-3-methylbenzimidamide

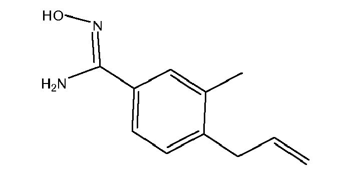

Obtained (100%) from Preparation 60 following General Method 1.
LRMS: m/z 191 (M+1)⁺
Retention time: 3.45 min (Method B)

### Preparation 62

### 3-(4-Allyl-3-methylphenyl)-5-(1-methyl-5-phenyl-1H-pyrazol-3-yl)-1,2,4-oxadiazole

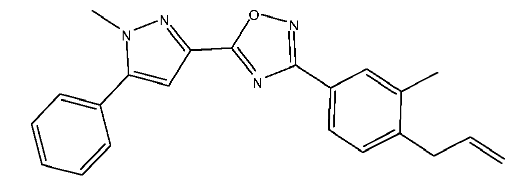

Obtained (43%) from 1-methyl-5-phenyl-1H-pyrazole-3-carboxylic acid and Preparation 61 following General Method 2.
LRMS: m/z 357 (M+1)⁺
Retention time: 7.83 min (Method B)

### Preparation 63

### 2-(2-Methyl-4-(5-(1-methyl-5-phenyl-1H-pyrazol-3-yl)-1,2,4-oxadiazol-3-yl)phenyl)acetaldehyde

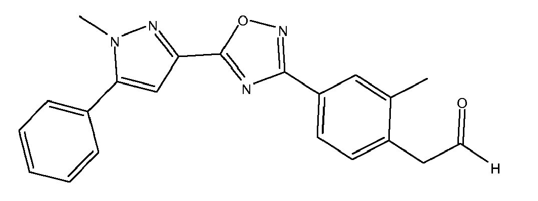

Obtained (82%) from Preparation 62 following the procedure described in Preparation 42.
LRMS: m/z 373 (M+1)⁺
Retention time: 6.80 min (Method B)

### Preparation 64

### 4-allyl-3-(trifluoromethyl)benzonitrile

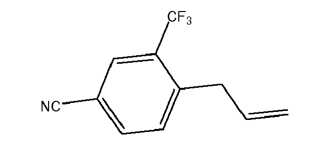

Obtained (85%) from Preparation 33 following the procedure described in Preparation 2.
LRMS: No signal
Retention time: 6.72 min (Method B)

### Preparation 65

### 4-allyl-N'-hydroxy-3-(trifluoromethyl)benzimidamide

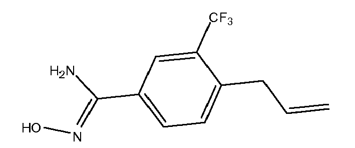

Obtained (97%) from Preparation 64 following General Method 1.
LRMS: m/z 245 (M+1)⁺
Retention time: 5.05 min (Method B)

### Preparation 66

### 3-(4-Allyl-3-(trifluoromethyl)phenyl)-5-(1-methyl-5-phenyl-1H-pyrazol-3-yl)-1,2,4-oxadiazole

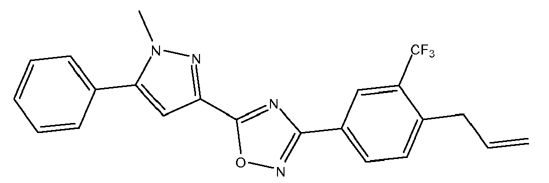

Obtained (43%) from 1-methyl-5-phenyl-1H-pyrazole-3-carboxylic acid and Preparation 65 following General Method 2.
LRMS: m/z 411 (M+1)⁺
Retention time: 6.97 min (Method B)

### Preparation 67

### 2-(4-(5-(1-Methyl-5-phenyl-1H-pyrazol-3-yl)-1,2,4-oxadiazol-3-yl)-2-(trifluoromethyl)phenyl)acetaldehyde

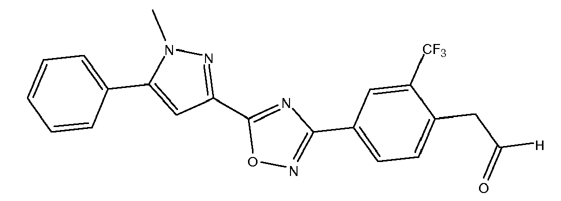

Obtained (76%) from Preparation 66 following the procedure described in Preparation 42.
LRMS: m/z 413 (M+1)⁺
Retention time: 7.45 min (Method B)

### Preparation 68

### 5-tert-Butyl-1-methyl-1H-pyrazole-3-carboxylic acid

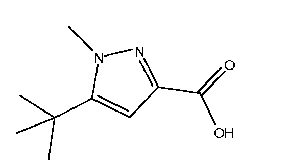

Obtained (11%) from ethyl 5,5-dimethyl-2,4-dioxohexanoate and methyl hydrazine following the procedure described in Preparation 29.
LRMS: m/z 211 (M+1)⁺
Retention time: 3.00 min (Method A)

### Preparation 69

### 3-(4-Allyl-3,5-dimethylphenyl)-5-(5-tert-butyl-1-phenyl-1H-pyrazol-3-yl)-1,2,4-oxadiazole

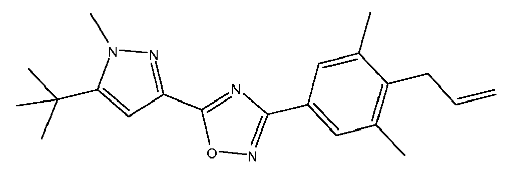

Obtained (54%) from Preparation 68 and Preparation 3 following General Method 2.
LRMS: m/z 351 (M+1)⁺
Retention time: 7.82 min (Method B)

### Preparation 70

### 2-(4-(5-(5-tert-Butyl-1-methyl-1H-pyrazol-3-yl)-1,2,4-oxadiazol-3-yl)-2,6-dimethylphenyl)acetaldehyde

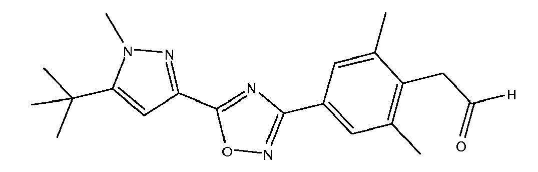

Obtained (80%) from Preparation 69 following the procedure described in Preparation 42.
LRMS: m/z 353 (M+1)⁺
Retention time: 6.97 min (Method B)

### Preparation 71

### Ethyl 4-cyclohexyl-2,4-dioxobutanoate

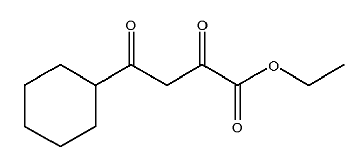

Obtained (66%) from 1-cyclohexylethanone and diethyl oxalate following the procedure described in Preparation 28.
LRMS: m/z 227 (M+1)⁺
Retention time: 6.78 min (Method B)

### Preparation 72

### 5-Cyclohexyl-1-ethyl-1H-pyrazole-3-carboxylic acid

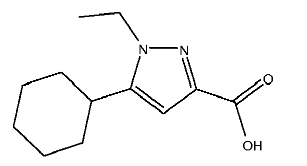

Obtained (51%) from Preparation 71 and ethyl hydrazine oxalic salt following the procedure described in Preparation 29.
LRMS: m/z 223 (M+1)⁺
Retention time: 5.77 min (Method B)

### Preparation 73

### 3-(4-Allyl-3,5-dimethylphenyl)-5-(5-cyclohexyl-1-ethyl-1H-pyrazol-3-yl)-1,2,4-oxadiazole

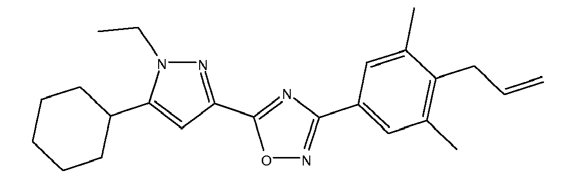

Obtained (74%) from Preparation 72 and Preparation 3 following General Method 2.
LRMS: m/z 391 (M+1)⁺
Retention time: 8.13 min (Method B)

### Preparation 74

### 2-(4-(5-(5-Cyclohexyl-1-ethyl-1H-pyrazol-3-yl)-1,2,4-oxadiazol-3-yl)-2,6-dimethylphenyl)acetaldehyde

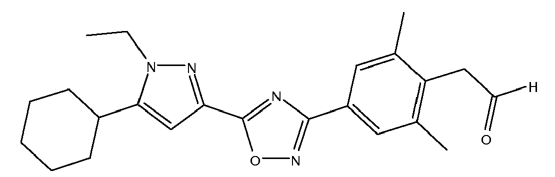

Obtained (70%) from Preparation 73 following the procedure described in Preparation 42.
LRMS: m/z 393 (M+1)⁺
Retention time: 7.52 min (Method B)

### Preparation 75

### 1-Ethyl-5-(4-methoxyphenyl)-1H-pyrazole-3-carboxylic acid

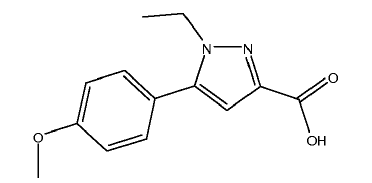

Obtained (69%) from ethyl 3-(4-methoxyphenyl)-2,3-dioxopropanoate and ethyl hydrazine oxalic salt following the procedure described in Preparation 29.
LRMS: m/z 247 (M+1)⁺
Retention time: 5.30 min (Method B)

### Preparation 76

### 3-(4-Allyl-3,5-dimethylphenyl)-5-(1-ethyl-5-(4-methoxyphenyl)-1H-pyrazol-3-yl)-1,2,4-oxadiazole

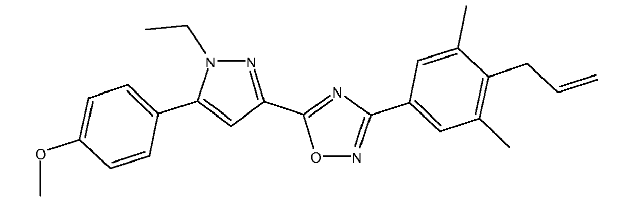

Obtained (71%) from Preparation 75 and Preparation 3 following General Method 2.
LRMS: m/z 415 (M+1)⁺
Retention time: 7.92 min (Method B)

### Preparation 77

### 2-(4-(5-(1-Ethyl-5-(4-methoxyphenyl)-1H-pyrazol-3-yl)-1,2,4-oxadiazol-3-yl)-2,6-dimethylphenyl)acetaldehyde

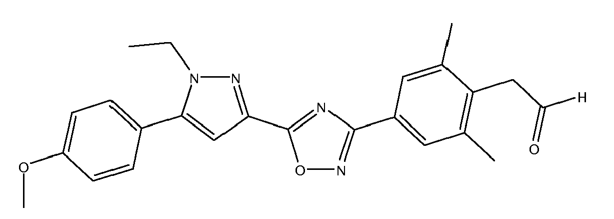

Obtained (48%) from Preparation 76 following the procedure described in Preparation 42.
LRMS: m/z 417 (M+1)⁺
Retention time: 7.23 min (Method B)

### Preparation 78

### Ethyl 6-methyl-2,4-dioxoheptanoate

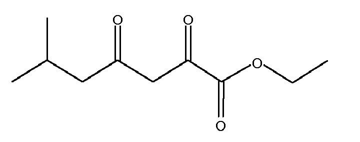

Obtained (87%) from 4-methylpentan-2-one and diethyl oxalate following the procedure described in Preparation 28.
¹H NMR (400 MHz, CHLOROFORM*-d*) δ ppm 0.97 (d, *J*=6.64 Hz, 6 H) 1.38 (t, *J*=7.23 Hz, 3 H) 2.05 - 2.24 (m, *J*=13.87, 6.84, 6.84, 6.64, 6.45 Hz, 1 H) 2.35 (d, *J*=7.03 Hz, 2 H) 4.35 (q, *J*=7.29 Hz, 2 H) 6.34 (s, 1 H).

### Preparation 79

### 1-Ethyl-5-isobutyl-1H-pyrazole-3-carboxylic acid

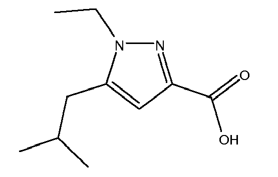

Obtained (68%) from Preparation 78 and ethyl hydrazine oxalic salt following the procedure described in Preparation 29.
LRMS: m/z 197 (M+1)⁺
Retention time: 7.22 min (Method B)

### Preparation 80

### 3-(4-Allyl-3,5-dimethylphenyl)-5-(1-ethyl-5-isobutyl-1H-pyrazol-3-yl)-1,2,4-oxadiazole

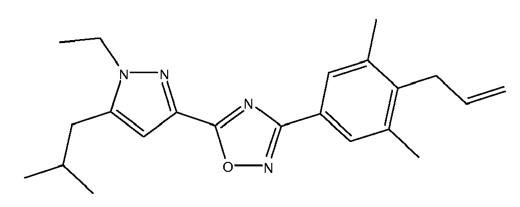

Obtained (40%) from Preparation 79 and Preparation 3 following General Method 2.
LRMS: m/z 365 (M+1)⁺
Retention time: 7.95 min (Method B)

### Preparation 81

### 2-(4-(5-(1-Ethyl-5-isobutyl-1H-pyrazol-3-yl)-1,2,4-oxadiazol-3-yl)-2,6-dimethylphenyl)acetaldehyde

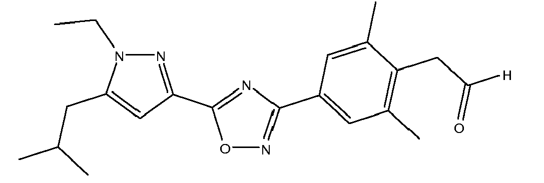

Obtained (100%) from Preparation 80 following the procedure described in Preparation 42.
LRMS: m/z 367 (M+1)⁺
Retention time: 7.23 min (Method B)

### Preparation 82

### Ethyl 4-(4-fluorophenyl)-2,4-dioxobutanoate

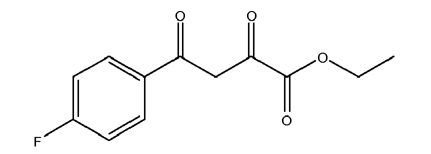

Obtained (100%) from 1-(4-fluorophenyl)ethanone and diethyl oxalate following the procedure described in Preparation 28.
LRMS: m/z 239 (M+1)⁺
Retention time: 6.45 min (Method B)

### Preparation 83

### 1-Ethyl-5-(4-fluorophenyl)-1H-pyrazole-3-carboxylic acid

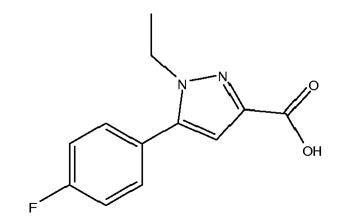

Obtained (55%) from Preparation 82 and ethyl hydrazine oxalic salt following the procedure described in Preparation 29.
LRMS: m/z 235 (M+1)⁺
Retention time: 5.45 min (Method B)
¹H NMR (300 MHz, CHLOROFORM*-d*) δ ppm 1.44 (t, *J*=7.28 Hz, 3 H) 4.23 (q, *J*=7.14 Hz, 2 H) 6.86 (s, 1 H) 7.14 - 7.25 (m, 2 H) 7.33 - 7.45 (m, 2 H).

### Preparation 84

### 3-(4-Allyl-3,5-dimethylphenyl)-5-(1-ethyl-5-(4-fluorophenyl)-1H-pyrazol-3-yl)-1,2,4-oxadiazole

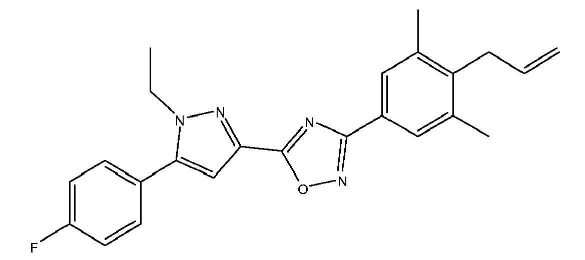

Obtained (35%) from Preparation 83 and Preparation 3 following General Method 2.
LRMS: m/z 403 (M+1)⁺
Retention time: 7.95 min (Method B)
¹H NMR (300 MHz, CHLOROFORM*-d*) δ ppm 1.50 (t, *J*=6.87 Hz, 3 H) 2.39 (s, 6 H) 3.46 (br. s., 2 H) 4.31 (q, 2 H) 4.88 (d, *J*=17.03 Hz, 1 H) 5.04 (d, *J*=10.16 Hz, 1 H) 5.78 - 6.06 (m, 1 H) 7.01 (s, 1 H) 7.16 - 7.27 (m, 2 H) 7.37 - 7.52 (m, 2 H) 7.90 (s, 2 H).

### Preparation 85

### 2-(4-(5-(1-Ethyl-5-(4-fluorophenyl)-1H-pyrazol-3-yl)-1,2,4-oxadiazol-3-yl)-2,6-dimethylphenyl)acetaldehyde

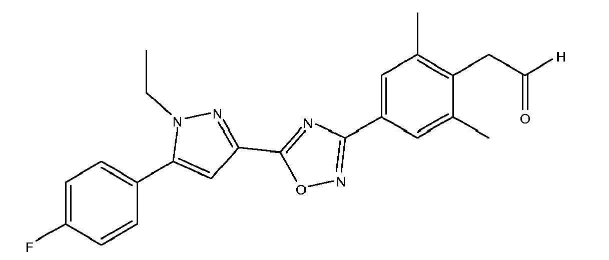

Obtained (81%) from Preparation 84 following the procedure described in Preparation 42.
LRMS: m/z 405 (M+1)⁺
Retention time: 7.28 min (Method B)
¹H NMR (300 MHz, DMSO*-d*₆) δ ppm 1.39 (t, *J*=7.14 Hz, 3 H) 2.31 (s, 6 H) 3.98 (s, 2 H) 4.28 (q, 2 H) 7.21 (s, 1 H) 7.34 - 7.56 (m, 2 H) 7.60 - 7.75 (m, 2 H) 7.78 (s, 2 H) 9.68 (br. s., 1 H).

### Preparation 86

### Ethyl 4-(2-methoxyphenyl)-2,4-dioxobutanoate

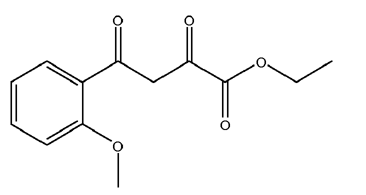

Obtained (45%) from 1-(2-methoxyphenyl)ethanone and diethyl oxalate following the procedure described in Preparation 28.
LRMS: m/z 251 (M+1)⁺
Retention time: 6.25 min (Method B)

### Preparation 87

### 1-Ethyl-5-(2-methoxyphenyl)-1H-pyrazole-3-carboxylic acid

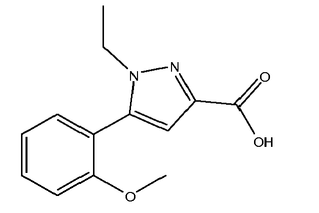

Obtained (46%) from Preparation 86 and ethyl hydrazine oxalic salt following the procedure described in Preparation 29.
LRMS: m/z 247 (M+1)⁺
Retention time: 5.33 min (Method B)

### Preparation 88

### 3-(4-Allyl-3,5-dimethylphenyl)-5-(1-ethyl-5-(2-methoxyphenyl)-1H-pyrazol-3-yl)-1,2,4-oxadiazole

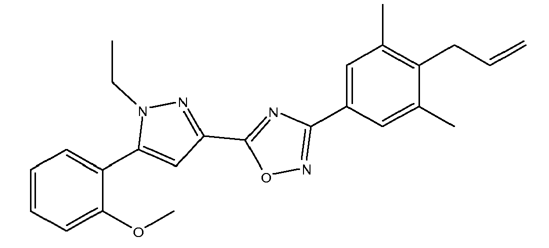

Obtained (32%) from Preparation 87 and Preparation 3 following General Method 2.
LRMS: m/z 415 (M+1)⁺
Retention time: 7.87 min (Method B)

### Preparation 89

### 2-(4-(5-(1-Ethyl-5-(2-methoxyphenyl)-1H-pyrazol-3-yl)-1,2,4-oxadiazol-3-yl)-2,6-dimethylphenyl)acetaldehyde

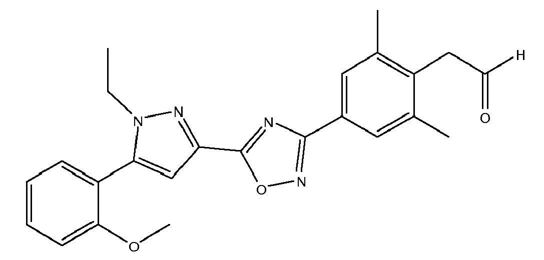

Obtained (74%) from Preparation 88 following the procedure described in Preparation 42.
LRMS: m/z 417 (M+1)⁺
Retention time: 7.12 min (Method B)

### Preparation 90

### 4-Bromo-2,5-dimethylbenzonitrile

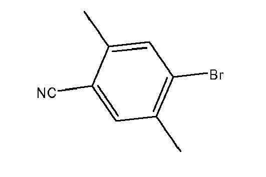

To a solution of CuCN (4.1 g, 45.46 mmol) in DMF (150 mL) 1,4-dibromo-2,5-dimethylbenzene (10 g, 37.88 mmol) was added and mixture was stirred at 150°C for 16 h. Reaction was cooled down and a solution of FeCl₃·6H₂O in concentrated HCl (13 mL) and water (50 mL) was slowly added and mixture was heated at 70°C for 30 min. Then water was added and desired product extracted with ethyl acetate, organic layers were combined and washed with brine, dried over sodium sulphate, filtered and concentrated. Crude thus obtained was purified by normal phase chromatography to yield the title compound (31 % yield).
LRMS: no signal
Retention time: 3.38 min (Method A)
¹H NMR (400 MHz, DMSO*-d*₆) δ ppm 2.38 (s, 3 H), 2.48 (s, 3 H), 7.43 (s, 1 H), 7.51 (s, 1 H).

### Preparation 91

### 4-Allyl-2,5-dimethylbenzonitrile

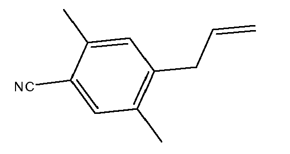

Obtained (68%) from Preparation 90 following the procedure described in Preparation 2.
LRMS: no signal
Retention time: 3.45 min (Method A)

### Preparation 92

### 4-Allyl-N'-hydroxy-2,5-dimethylbenzimidamide

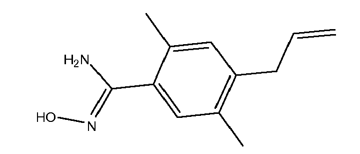

Obtained (30%) from Preparation 91 following General Method 1, using triethylamine at room temperature.
LRMS: m/z 205 (M+1)⁺
Retention time: 3.67 min (Method B)

### Preparation 93

### 3-(4-Allyl-2,5-dimethylphenyl)-5-(1-ethyl-5-phenyl-1H-pyrazol-3-yl)-1,2,4-oxadiazole

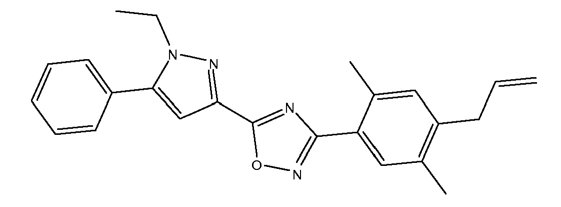

Obtained (41%) from Preparation 29 and Preparation 92 following General Method 2.
LRMS: m/z 385 (M+1)⁺
Retention time: 7.93 min (Method B)

### Preparation 94

### 2-(4-(5-(1-Ethyl-5-phenyl-1H-pyrazol-3-yl)-1,2,4-oxadiazol-3-yl)-2,5-dimethylphenyl)acetaldehyde

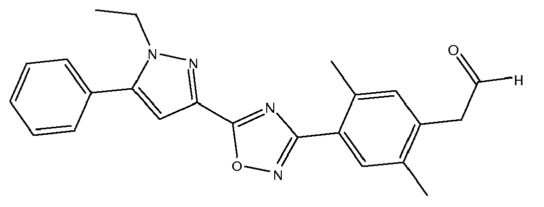

Obtained (64%) from Preparation 93 following the procedure described in Preparation 42.
LRMS: m/z 387 (M+1)⁺
Retention time: 7.08 min (Method B)

### Preparation 95

### Ethyl 4-(2-fluorophenyl)-2,4-dioxobutanoate

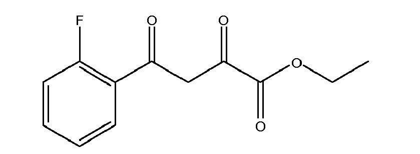

Obtained (87%) from 1-(2-fluorophenyl)ethanone and diethyl oxalate following the procedure described in Preparation 28, preparing the NaH in situ with Na in ethanol.
LRMS: m/z 239 (M+1)⁺
Retention time: 6.28 min (Method B)

### Preparation 96

### 1-Ethyl-5-(2-fluorophenyl)-1H-pyrazole-3-carboxylic acid

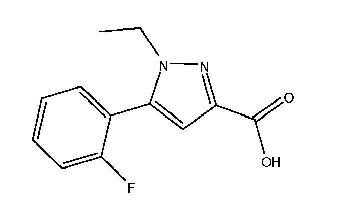

Obtained (49%) from Preparation 95 and ethyl hydrazine oxalic salt following the procedure described in Preparation 29.
LRMS: m/z 235 (M+1)⁺
Retention time: 5.23 min (Method B)

### Preparation 97

### 3-(4-Allyl-3,5-dimethylphenyl)-5-(1-ethyl-5-(2-fluorophenyl)-1H-pyrazol-3-yl)-1,2,4-oxadiazole

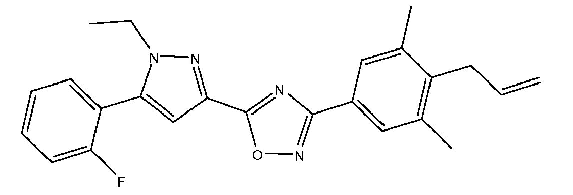

Obtained (50%) from Preparation 96 and Preparation 3 following General Method 2.
LRMS: m/z 403 (M+1)⁺
Retention time: 7.88 min (Method B)

### Preparation 98

### 2-(4-(5-(1-ethyl-5-(2-fluorophenyl)-1H-pyrazol-3-yl)-1,2,4-oxadiazol-3-yl)-2,6-dimethylphenyl)acetaldehyde

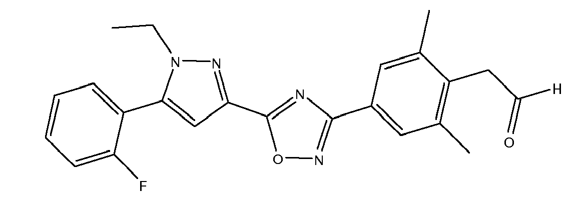

Obtained (92%) from Preparation 97 following the procedure described in Preparation 42.
LRMS: m/z 405 (M+1)⁺
Retention time: 7.18 min (Method B)

### Preparation 99

### Ethyl 3-methyl-2,4-dioxo-4-phenylbutanoate

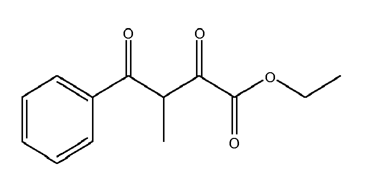

Obtained (87%) from propiophenone and diethyl oxalate following the procedure described in Preparation 28, preparing the NaH in situ with Na in ethanol.
LRMS: m/z 239 (M+1)⁺
Retention time: 6.28 min (Method B)

### Preparation 100

### 1-ethyl-4-methyl-5-phenyl-1H-pyrazole-3-carboxylic acid

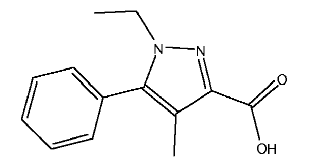

Obtained (29%) from Preparation 99 and ethyl hydrazine oxalic salt following the procedure described in Preparation 29.
LRMS: m/z 231 (M+1)⁺
Retention time: 5.62 min (Method B)

### Preparation 101

### 3-(4-allyl-3,5-dimethylphenyl)-5-(1-ethyl-4-methyl-5-phenyl-1H-pyrazol-3-yl)-1,2,4-oxadiazole

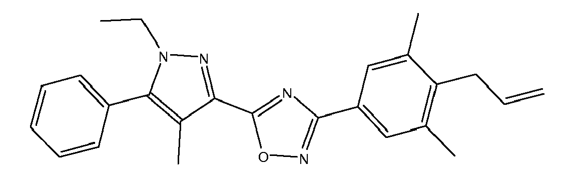

Obtained (46%) from Preparation 100 and Preparation 3 following General Method 2.
LRMS: m/z 399 (M+1)⁺
Retention time: 8.07 min (Method B)

### Preparation 102

### 2-(4-(5-(1-ethyl-4-methyl-5-phenyl-1H-pyrazol-3-yl)-1,2,4-oxadiazol-3-yl)-2,6-dimethylphenyl)acetaldehyde

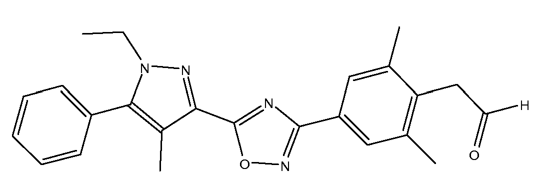

Obtained (91%) from Preparation 101 following the procedure described in Preparation 42.
LRMS: m/z 401 (M+1)⁺
Retention time: 7.43 min (Method B)

### Preparation 103

### Ethyl 2,4-dioxo-4-(2-(trifluoromethyl)phenyl)butanoate

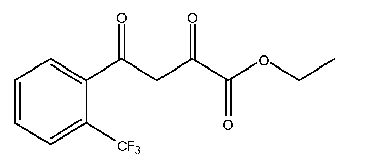

Obtained (90%) from 1-(2-(trifluoromethyl)phenyl)ethanone and diethyl oxalate following the procedure described in Preparation 28, preparing the NaH in situ with Na in ethanol.
LRMS: m/z 289 (M+1)⁺
Retention time: 6.43 min (Method B)

### Preparation 104

### 1-Ethyl-5-(2-(trifluoromethyl)phenyl)-1H-pyrazole-3-carboxylic acid

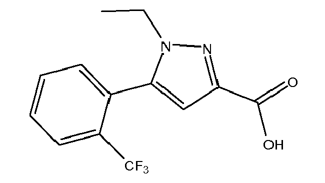

Obtained (66%) from Preparation 103 and ethyl hydrazine oxalic salt following the procedure described in Preparation 29.
LRMS: m/z 285 (M+1)⁺
Retention time: 5.63 min (Method B)

### Preparation 105

### 3-(4-Allyl-3,5-dimethylphenyl)-5-(1-ethyl-5-(2-(trifluoromethyl)phenyl)-1H-pyrazol-3-yl)-1,2,4-oxadiazole

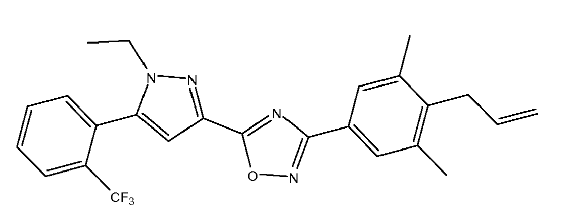

Obtained (44%) from Preparation 104 and Preparation 3 following General Method 2.
LRMS: m/z 453 (M+1)⁺
Retention time: 7.93 min (Method B)

### Preparation 106

### 2-(4-(5-(1-Ethyl-5-(2-(trifluoromethyl)phenyl)-1H-pyrazol-3-yl)-1,2,4-oxadiazol-3-yl)-2,6-dimethylphenyl)acetaldehyde

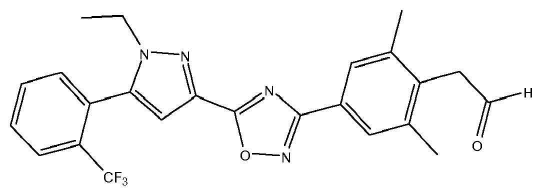

Obtained (100%) from Preparation 105 following the procedure described in Preparation 42.
LRMS: m/z 455 (M+1)⁺
Retention time: 7.30 min (Method B)

### Preparation 107

### 3,5-Dimethyl-4-(2-oxoethyl)benzonitrile

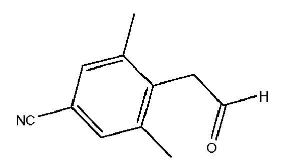

Obtained (100%) from Preparation 2 following the procedure described in Preparation 42.
LRMS: m/z 174 (M+1)⁺
Retention time: 5.23 min (Method B)

### Preparation 108

### Ethyl 1-(4-cyano-2,6-dimethylphenethyl)piperidine-4-carboxylate

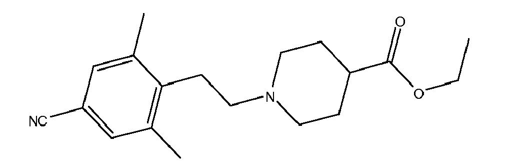

Obtained (61%) from Preparation 107 and ethyl piperidine-4-carboxylate following General Method 6.
LRMS: m/z 315 (M+1)⁺
Retention time: 3.88 min (Method B)

### Preparation 109

### Ethyl 1-(4-(N'-hydroxycarbamimidoyl)-2,6-dimethylphenethyl)piperidine-4-carboxylate

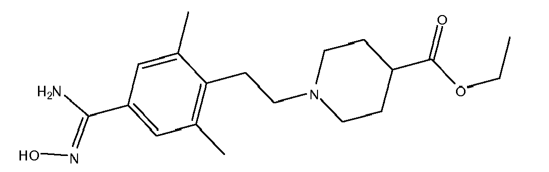

Obtained (80%) from Preparation 108 following General Method 1, using triethylamine and heating to reflux overnight.
LRMS: m/z 348 (M+1)⁺
Retention time: 2.82 min (Method B)
¹H NMR (400 MHz, DMSO*-d*₆) δ ppm 1.19 (t, *J*=7.03 Hz, 3 H) 1.68 (br. s., 2 H) 1.88 (br. s., 2 H) 2.30 (s, 6 H) 2.81 (br. s., 2 H) 3.04 (br. s., 2 H) 4.08 (q, *J*=6.90 Hz, 2 H) 5.69 (s, 2 H) 7.30 (s, 2 H) 9.48 (br. s., 1 H).

### Preparation 110

### Ethyl 2,4-dioxo-4-o-tolylbutanoate

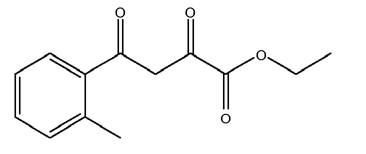

Obtained (44%) from 1-o-tolylethanone and diethyl oxalate following the procedure described in Preparation 28, preparing the NaH *in situ* with Na in ethanol.
LRMS: m/z 235 (M+1)⁺
Retention time: 6.58 min (Method B)
¹H NMR (400 MHz, CHLOROFORM*-d*) δ ppm 1.39 (t, *J*=7.23 Hz, 3 H) 2.55 (s, 3 H) 4.38 (q, *J*=7.03 Hz, 2 H) 6.83 (s, 1 H) 7.28 (t, 2 H) 7.42 (t, *J*=7.42 Hz, 1 H) 7.63 (d, *J*=7.42 Hz, 1 H).

### Preparation 111

### 1-Ethyl-5-o-tolyl-1H-pyrazole-3-carboxylic acid

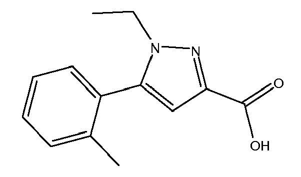

Obtained (31%) from Preparation 110 and ethyl hydrazine oxalic salt following the procedure described in Preparation 29.
LRMS: m/z 231 (M+1)⁺
Retention time: 5.55 min (Method B)

### Preparation 112

### Ethyl 1-(4-(5-(1-ethyl-5-o-tolyl-1H-pyrazol-3-yl)-1,2,4-oxadiazol-3-yl)-2,6-dimethyl phenethyl)piperidine-4-carboxylate

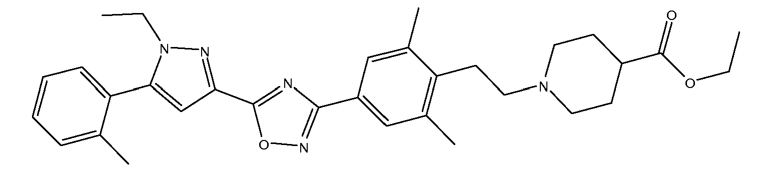

Obtained (58%) from Preparation 111 and Preparation 109 following General Method 2.
LRMS: m/z 455 (M+1)⁺
Retention time: 7.30 min (Method B)

### Preparation 113

### tert-Butyl 3-(4-cyano-2,6-dimethylphenyl)acrylate

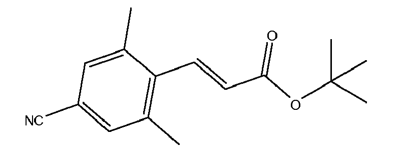

Obtained (53%) from Preparation 1 and *tert*-Butyl acrylate following the procedure described in Preparation 15.
LRMS: no signal
Retention time: 7.10 min (Method B)

### Preparation 114

### tert-Butyl 3-(4-(N'-hydroxycarbamimidoyl)-2,6-dimethylphenyl)acrylate

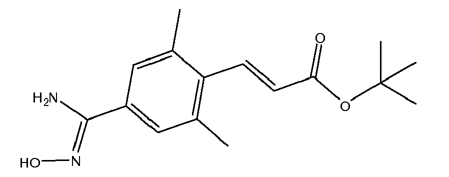

Obtained (69%) from Preparation 113 following General Method 1.
LRMS: m/z 291 (M+1)⁺
Retention time: 4.89 min (Method B)

### Preparation 115

### tert-Butyl 3-(4-(N'-hydroxycarbamimidoyl)-2,6-dimethylphenyl)propanoate

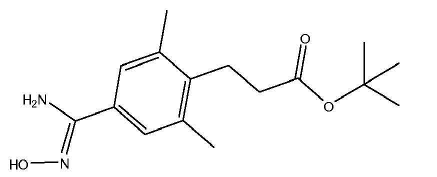

Obtained (77%) from Preparation 114 following the experimental procedure described in Preparation 17.
LRMS: m/z 293 (M+1)⁺
Retention time: 4.66 min (Method B)

### Preparation 116

### tert-Butyl 3-(4-(5-(1-ethyl-5-phenyl-1H-pyrazol-3-yl)-1,2,4-oxadiazol-3-yl)-2,6-di methylphenyl)propanoate

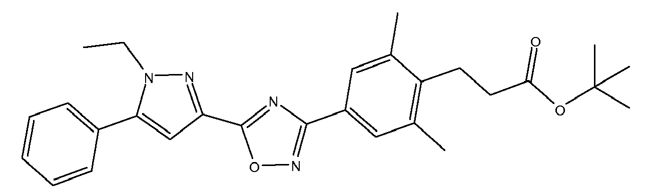

Obtained (73%) from Preparation 29 and Preparation 115 following General Method 2.
LRMS: m/z 473 (M+1)⁺
Retention time: 7.93 min (Method B)

### Preparation 117

### Methyl 4-methoxy-3,5-dimethylbenzoate

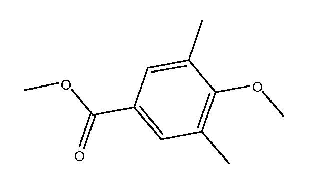

To a suspension of 4-methoxy-3,5-dimethylbenzoic acid (4 g, 22.2 mmol) in MeOH (60 mL) sulphuric acid was added (1 mL) and mixture stirred at reflux for 1 day. Water was added and it was extracted with AcOEt, organic layers were put together and dried over sodium sulphate and concentrated. The oil thus obtained was purified by normal phase chromatography with 5% MeOH/DCM to yield the title compound as a colorless oil (99% yield).
LRMS: m/z 195 (M+1)⁺
Retention time: 6.17 min (Method B)
¹H NMR (250 MHz, DMSO*-d*₆) δ ppm 2.30 (s, 6 H) 3.74 (s, 3 H) 3.87 (s, 3 H) 7.71 (s,2H).

### Preparation 118

### 4-methoxy-3,5-dimethylbenzohydrazide

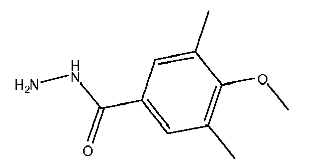

A solution of Preparation 117 (4.29 g, 22.1 mmol) in hydrazine hydrate (16.1 mL, 331.3 mmol) was stirred to reflux for 1 h and then solvent was removed to yield the title compound as a white solid (100% yield).
LRMS: m/z 195 (M+1)⁺
Retention time: 4.02 min (Method B)
¹H NMR (250 MHz, DMSO*-d*₆) δ ppm 2.24 (s, 6 H) 3.66 (s, 3 H) 7.50 (s, 2 H).

### Preparation 119

### N'-(4-methoxy-3,5-dimethylbenzoyl)-1-methyl-5-phenyl-1H-pyrazole-3-carbohydrazide

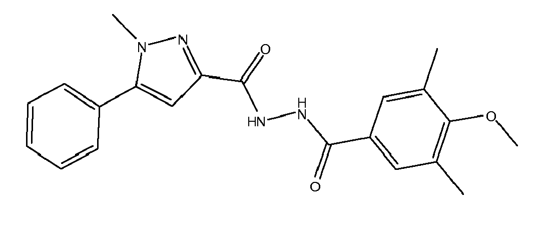

Obtained (48%) from 1-methyl-5-phenyl-1H-pyrazole-3-carboxylic acid and Preparation 118 following General Method 5.
LRMS: m/z 379 (M+1)⁺
Retention time: 5.70 min (Method B)

### Preparation 120

### 2-(4-methoxy-3,5-dimethylphenyl)-5-(1-methyl-5-phenyl-1H-pyrazol-3-yl)-1,3,4-oxadiazole

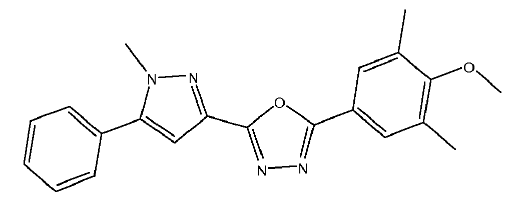

A solution of Preparation 119 (783 mg, 2.07 mmol) in POCl₃ (6 mL) was heated at reflux for 20 min, solvent was removed and the residue was poured onto saturated aquous NaHCO₃ solution. Product was extracted with DCM and organic layer dried over sodium sulphate and concentrated to yield the title compound (95% yield).
LRMS: m/z 361 (M+1)⁺
Retention time: 7.03 min (Method B)

### Preparation 121

### 2,6-dimethyl-4-(5-(1-methyl-5-phenyl-1H-pyrazol-3-yl)-1,3,4-oxadiazol-2-yl)phenol

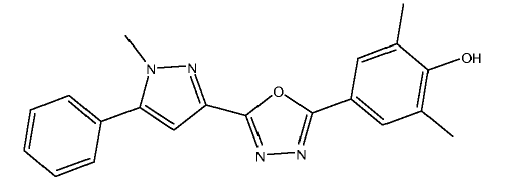

To Preparation 120 (480 mg, 1.26 mmol) in dichloromethane (9 mL) BBr₃ 1 M in DCM (7 mL, 7 mmol) was added and mixture stirred at r.t. for 2 h. Then crude reaction was poured onto MeOH saturated with NaHCO₃, filtered and solution concentrated. Solid was redissolved in DCM and washed with water, dried and concentrated to yield the title compound (68% yield).
LRMS: m/z 347 (M+1)⁺
Retention time: 6.38 min (Method B)

### Preparation 122

### 2,6-Dimethyl-4-(5-(1-methyl-5-phenyl-1H-pyrazol-3-yl)-1,3,4-oxadiazol-2-yl)phenyl trifluoromethanesulfonate

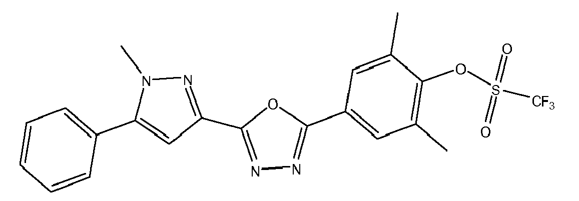

Obtained (92%) from Preparation 121 following the procedure described in Preparation 1 using NaH as base.
LRMS: m/z 479 (M+1)⁺
Retention time: 7.55 min (Method B)

### Preparation 123

### 2-(4-Allyl-3,5-dimethylphenyl)-5-(1-methyl-5-phenyl-1H-pyrazol-3-yl)-1,3,4-oxadiazole

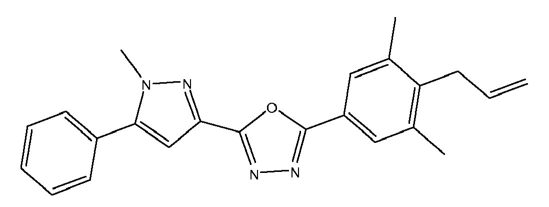

Obtained (81%) from Preparation 122 following the procedure described in Preparation 2.
LRMS: m/z 371 (M+1)⁺
Retention time: 7.47 min (Method B)

### Preparation 124

### 2-(2,6-Dimethy)-4-(5-(1-methyl-5-phenyl-1H-pyrazol-3-yl)-1,3,4-oxadiazol-2-yl)phenyl)acetaldehyde

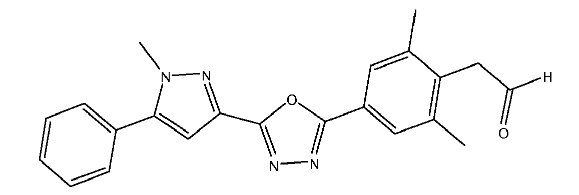

Obtained (83%) from Preparation 123 following the procedure described in Preparation 42.
LRMS: m/z 373 (M+1)⁺
Retention time: 6.45 min (Method B)

### Preparation 125

### Ethyl 2,4-dioxo-5-phenylpentanoate

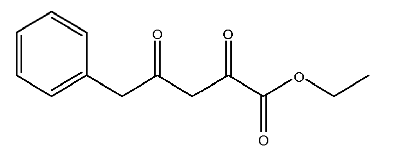

Obtained (46%) from 1-phenylpropan-2-one and diethyl oxalate following the procedure described in Preparation 28, preparing the NaH in situ with Na in ethanol.
LRMS: m/z 235 (M+1)⁺
Retention time: 6.18 min (Method B)

### Preparation 126

### 5-Benzyl-1-ethyl-1H-pyrazole-3-carboxylic acid

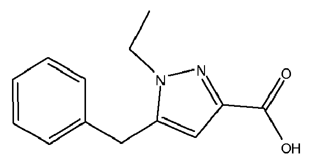

Obtained (54%) from Preparation 125 and ethyl hydrazine oxalic salt following the procedure described in Preparation 29.
LRMS: m/z 231 (M+1)⁺
Retention time: 5.35 min (Method B)
¹H NMR (300 MHz, CHLOROFORM*-d*) δ ppm 1.34 (t, *J*=7.28 Hz, 3 H) 4.01 (s, 2 H)4.11 (q, *J*=7.14 Hz, 2 H) 6.61 (s, 1 H) 7.16 (m, 3 H) 7.24 - 7.43 (m, 2 H).

### Preparation 127

### 3-(4-Allyl-3,5-dimethylphenyl)-5-(5-methyl-1-phenyl-1H-pyrazol-3-yl)-1,2,4-oxadiazole

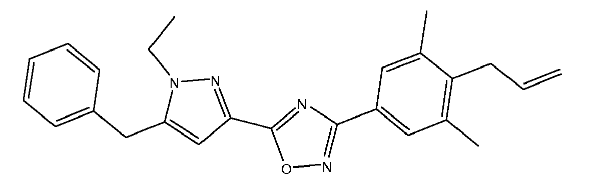

Obtained (27%) from Preparation 126 and Preparation 3 following General Method 2.
LRMS: m/z 399 (M+1)⁺
Retention time: 7.95 min (Method B)
¹H NMR (300 MHz, CHLOROFORM*-d*) δ ppm 1.40 (t, *J*=7.14 Hz, 3 H) 2.36 (s, 6 H) 3.36 - 3.49 (m, 2 H) 4.08 (s, 2 H) 4.20 (q, *J*=7.32 Hz, 2 H) 4.86 (dt, *J*=17.10, 1.75 Hz, 1 H) 5.02 (dt, *J*=10.16, 1.65 Hz, 1 H) 5.75 - 6.05 (m, *J*=10.99, 10.99, 10.71, 5.49Hz, 1 H) 7.10 - 7.42 (m, 5 H) 7.86 (s, 2 H).

### Preparation 128

### 2-(4-(5-(5-Benzy)-1-ethy)-1H-pyrazol-3-yl)-1,2,4-oxadiazol-3-yl)-2,6-dimethylphenyl)acetaldehyde

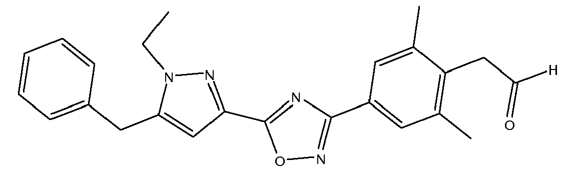

Obtained (67%) from Preparation 127 following the procedure described in Preparation 42.
LRMS: m/z 401 (M+1)⁺
Retention time: 7.07 min (Method B)

### Preparation 129

### Ethyl 6,6-dimethyl-2,4-dioxoheptanoate

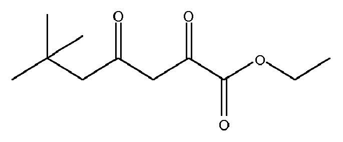

Obtained (47%) from 4,4-dimethylpentan-2-one and diethyl oxalate following the procedure described in Preparation 28, preparing the NaH in situ with Na in ethanol.
LRMS: m/z 215 (M+1)⁺
Retention time: 6.52 min (Method B)

### Preparation 130

### 1-Ethyl-5-neopentyl-1H-pyrazole-3-carboxylic acid

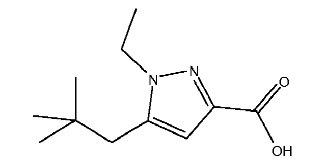

Obtained (64%) from Preparation 129 and ethyl hydrazine oxalic salt following the procedure described in Preparation 29.
LRMS: m/z 211 (M+1)⁺
Retention time: 5.60 min (Method B)

### Preparation 131

### 3-(4-Allyl-3,5-dimethylphenyl)-5-(1-ethyl-5-neopentyl-1H-pyrazol-3-yl)-1,2,4-oxadiazole

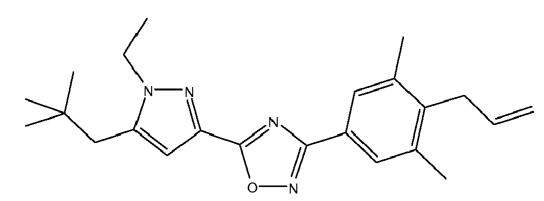

Obtained (54%) from Preparation 130 and Preparation 3 following General Method 2.
LRMS: m/z 379 (M+1)⁺
Retention time: 8.10 min (Method B)

### Preparation 132

### 2-(4-(5-(1-Ethyl-5-neopentyl-1H-pyrazol-3-yl)-1,2,4-oxadiazol-3-yl)-2,6-dimethylphenyl)acetaldehyde

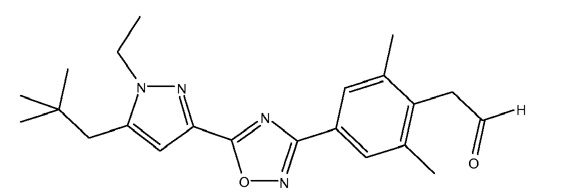

Obtained (80%) from Preparation 131 following the procedure described in Preparation 42.
LRMS: m/z 381 (M+1)⁺
Retention time: 7.40 min (Method B)

### Preparation 133

### 4-(2-Oxoethyl)-3-(trifluoromethyl)benzonitrile

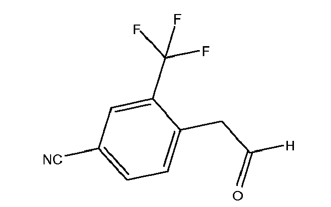

Obtained (83%) from Preparation 64 following the procedure described in Preparation 42.
LRMS: m/z 214 (M+1)⁺
Retention time: 4.73 min (Method B)
¹H NMR (400 MHz, CHLOROFORM*-d*) δ ppm 4.03 (s, 2 H) 7.47 (d, *J*=7.82 Hz, 1 H) 7.85 (d, *J*=8.21 Hz, 1 H) 8.00 (s, 1 H) 9.79 (s, 1 H).

### Preparation 134

### Ethyl 1-(4-cyano-2-(trifluoromethyl)phenethyl)piperidine-4-carboxylate

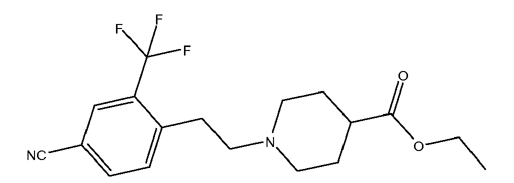

Obtained (78%) from Preparation 133 and ethyl piperidine-4-carboxylate following General Method 6.
LRMS: m/z 355 (M+1)⁺
Retention time: 4.02 min (Method B)
¹H NMR (400 MHz, CHLOROFORM*-d*) δ ppm 1.27 (td, *J*=7.13, 5.28 Hz, 3 H) 1.77 (dd, *J*=13.29, 3.52 Hz, 1 H) 1.93 (d, *J*=10.16 Hz, 2 H) 1.99-2.42 (m, 7 H) 2.47 - 2.64 (m, 2 H) 2.81 - 3.07 (m, 4 H) 3.20 - 3.37 (m, 1 H) 4.16 (dd, *J*=10.94, 7.42 Hz, 2 H) 7.50 - 7.64 (m, 1 H) 7.72 - 7.88 (m, 1 H) 7.89 - 8.02 (m, 1 H).

### Preparation 135

### Ethyl 1-(4-(N'-hydroxycarbamimidoyl)-2-(trifluoromethyl)phenethyl)piperidine-4-carboxylate

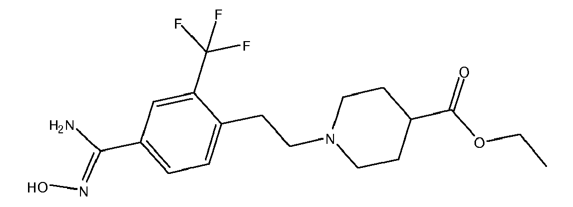

Obtained (74%) from Preparation 134 following General Method 1, heating with triethylamine to reflux.
LRMS: m/z 388 (M+1)⁺
Retention time: 3.18 min (Method B)
¹H NMR (400 MHz, CHLOROFORM*-d*) δ ppm 1.26 (t, *J*=7.03 Hz, 3 H) 1.73 - 1.87 (m, 2 H) 1.87 - 2.00 (m, 2 H) 2.17 (t, *J*=10.55 Hz, 1 H) 2.32 (t, *J*=10.94 Hz, 1 H) 2.62 (d, *J*=8.21 Hz, 2 H) 2.88 - 3.12 (m, 2 H) 4.14 (d, *J*=7.42 Hz, 2 H) 7.40 (d, *J*=8.21 Hz, 1 H) 7.65 - 7.83 (m, 1 H) 7.83 - 7.98 (m, 1 H).

### Preparation 136

### Ethyl 1-(4-(5-(5-cyclohexyl-1-ethyl-1H-pyrazol-3-yl)-1,2,4-oxadiazol-3-yl)-2-(tri fluoromethyl)phenethyl)piperidine-4-carboxylate

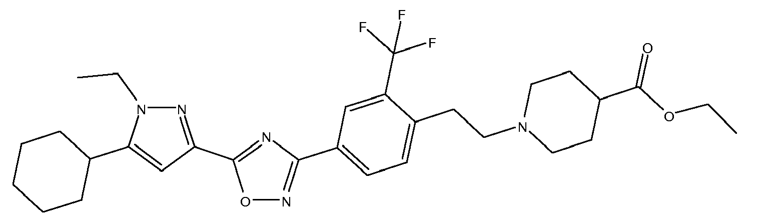

Obtained (61%) from Preparation 72 and Preparation 135 following General Method 2, using NMP as solvent.
LRMS: m/z 574 (M+1)⁺
Retention time: 6.33 min (Method B)

### Preparation 137

### 3-Methyl-1-phenyl-1H-pyrazole

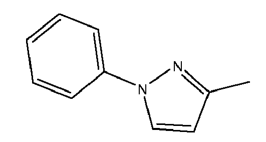

3-Methyl-1H-pyrazole (1 g, 12.18 mmol), phenylboronic acid (3 g, 24.36 mmol) and pyridine (2 mL, 24.36 mmol) were dissolved (160 mL) and Cu(AcO)₂ (3.32 g, 18.27 mmol) and molecular sieves were added and mixture was stirred at r.t. for 48 h. Suspension was filtered through Celite and concentrated and the crude thus obtained was purified by normal phase chromatography using hexane/ethyl acetate from 0% to 7.5% to yield the title compound (83% yield).
LRMS: m/z 159 (M+1)⁺
Retention time: 2.83 min (Method B)
¹H NMR (400 MHz, CHLOROFORM*-d*) δ ppm 2.38 (s, 3 H) 6.25 (d, *J*=2.34 Hz, 1 H) 7.18-7.32 (m, 1 H) 7.42 (t, 2 H) 7.65 (dd, *J*=8.60, 1.17 Hz, 2 H) 7.81 (d, *J*=2.34 Hz, 1 H).

### Preparation 138

### 1-phenyl-1H-pyrazole-3-carboxylic acid

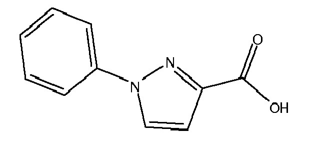

A suspension of Preparation 137 (2 g, 12.58 mmol) in water (50 mL) and *tert*-Butanol (2 mL) was heated at 70°C and a solution of KMnO₄ (4.97 g, 31.45 mmol) in water (120 mL) was dropwise added and mixture stirred at 70°C for 12 h. Crude was filtered through Celite, concentrated and acidified with 2 N HCl. Product was extracted with ethyl acetate, organic layers were combined and washed with brine, dried over sodium sulphate, filtered and concentrated to yield the title compound as a white solid (4% yield).
LRMS: m/z 189 (M+1)⁺
Retention time: 2.50 min (Method B)
¹H NMR (400 MHz, DMSO*-d*₆) δ ppm 6.96 (d, *J*=2.34 Hz, 1 H) 7.40 (t, *J*=7.42 Hz, 1 H) 7.55 (t, *J*=8.01 Hz, 2 H) 7.90 (d, *J*=7.82 Hz, 2 H) 8.61 (d, *J*=2.34 Hz, 1 H) 12.99 (br. s., 1H).

### Preparation 139

### 4-Bromo-1-ethyl-5-isobutyl-1H-pyrazole-3-carboxylic acid

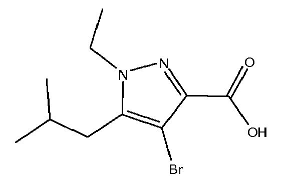

To a solution of Preparation 79 (700 mg, 3.57 mmol) in DMF (30 mL) N-bromosuccinimide (761.8 mg, 4.28 mmol) in DMF (10 mL) was slowly added and mixture was stirred at r.t. for 2 h. Solvent was removed, ethyl acetate was added and washed with 1N HCl, water and brine, dried over magnesium sulphate and concentrated to yield the title compound as an oil (88%).
LRMS: m/z 275/277 (M+1)⁺
Retention time: 5.80 min (Method B)

### Preparation 140

### Ethyl 4-bromo-1-ethyl-5-isobutyl-1H-pyrazole-3-carboxylate

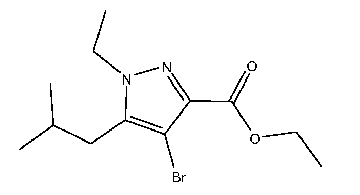

Obtained (85%) from Preparation 139 following the procedure described in Preparation 117.
LRMS: m/z 304 (M+1)⁺
Retention time: 6.63 min (Method B)
¹H NMR (400 MHz, CHLOROFORM-D) δ ppm 1.0 (d, *J*=6.6 Hz, 6 H) 1.4 (t, *J*=7.0Hz, 3 H) 1.5 (m, 3 H) 2.0 (m, 1 H) 2.6 (d, *J*=7.8Hz, 2 H) 4.2(q, *J*=7.4Hz, 2 H) 4.4 (q, *J*=7.0Hz, 2 H).

### Preparation 141

### Ethyl 1-ethyl-5-isobutyl-4-methyl-1H-pyrazole-3-carboxylate

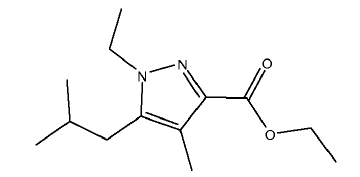

To a solution of Preparation 140 (665 mg, 2.19 mmol) in DMF (20 mL) trimethylboroxine (612 µL, 4.39 mmol) and K₂CO₃ were added under argon atmosphere. Then PdCl₂dppf.CH₂Cl₂ was added and mixture stirred at 110°C for 3 h. Solvent was removed, ethyl acetate was added and washed with water and brine, dried over magnesium sulphate, filtered and concentrated. Crude was purified by normal phase chromatography using hexane/ethyl acetate from 0% to 50% to yield the title compound (84% yield).
LRMS: m/z 239 (M+1)⁺
Retention time: 6.57 min (Method B)
¹H NMR (400 MHz, CHLOROFORM-D) δ ppm 0.9 (d, *J*=6.6 Hz, 6 H) 1.4 (t, *J*=6.4 Hz, 3 H) 1.4 (t, *J*=6.6 Hz, 3 H) 1.8 (m, 1 H) 2.2 (s, 3 H) 2.5 (d, *J*=7.4 Hz, 2 H)4.1 (q, *J*=7.0 Hz, 2 H) 4.4 (q, *J*=7.0 Hz, 2 H).

### Preparation 142

### 1-Ethyl-5-isobutyl-4-methyl-1H-pyrazole-3-carboxylic acid

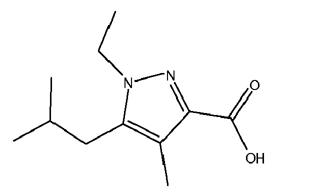

Obtained (89%) from Preparation 141 following General Method 3.
LRMS: m/z 211 (M+1)⁺
Retention time: 5.55 min (Method B)
¹H NMR (400 MHz, CHLOROFORM*-d*) δ ppm 0.94 (d, *J*=6.64 Hz, 6 H) 1.45 (t, *J*=7.23 Hz, 3 H) 1.73-1.98 (m, *J*=13.58, 7.00, 7.00, 7.00, 6.84 Hz, 1 H) 2.22 (s, 3 H) 2.47 (d, *J*=7.42 Hz, 2 H) 4.12 (q, *J*=7.42 Hz, 2 H).

### Preparation 143

### Ethyl 1-(4-(5-(1-ethyl-5-isobutyl-4-methyl-1H-pyrazol-3-yl)-1,2,4-oxadiazol-3-yl)-2, 6-dimethylphenethyl)piperidine-4-carboxylate

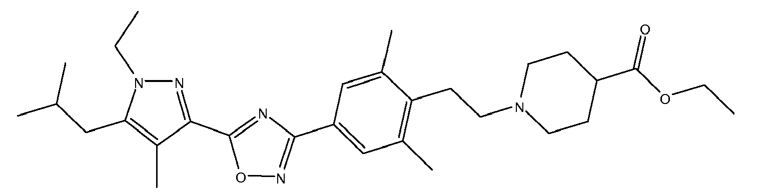

Obtained (27%) from Preparation 142 and Preparation 109 following General Method 2, using NMP as solvent.
LRMS: m/z 522 (M+1)⁺
Retention time: 5.92 min (Method B)

### Preparation 144

### Methyl 1-ethyl-5-isobutyl-1H-pyrazole-3-carboxylate

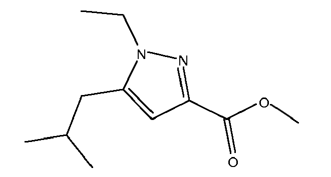

To Preparation 142 (590 mg, 3.01 mmol) 3N HCl in methanol (10 mL, 30 mmol) was added and solution was heated at 80°C for 4 h. Solvent was removed, water was added and mixture was basified, product extracted with ethyl acetate, dried over magnesium sulphate, filtered and concentrated to yield the title compound as an oil (54% yield).
LRMS: m/z 211 (M+1)⁺
Retention time: 5.82 min (Method B)

### Preparation 145

### 1-Ethyl-5-isobutyl-1H-pyrazole-3-carbohydrazide

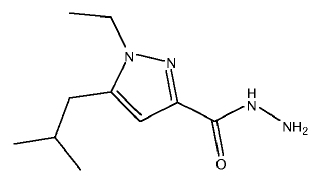

To a solution of Preparation 144 (430 mg, 2.04 mmol) in ethanol (20 mL) hidrazine hydrate (0.15 mL, 3.08 mmol) was added and mixture heated in the microwave at 120°C for 2 h. Solvent was removed, water was added and suspension was acidified with 2N HCl and washed with dichloromethane. Aquous layer was basified with NaHCO₃, desired product was extracted with dichloromethane, organic layers were combined, dried over magnesium sulphate, filtered and concentrated to yield the title compound as a brown oil (55% yield).
LRMS: m/z 211 (M+1)⁺
Retention time: 4.57 min (Method B)

### Preparation 146

### Ethyl 1-(4-cyano-2,6-dimethylphenethyl)piperidine-4-carboxylate

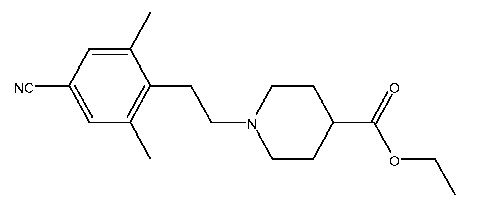

Obtained (36%) from Preparation 107 and ethyl piperidine-4-carboxylate following General Method 6.
LRMS: m/z 315 (M+1)⁺
Retention time: 3.82 min (Method B)

### Preparation 147

### Ethyl 1-(4-(ethoxy(imino)methyl)-2,6-dimethylphenethyl)piperidine-4-carboxylate

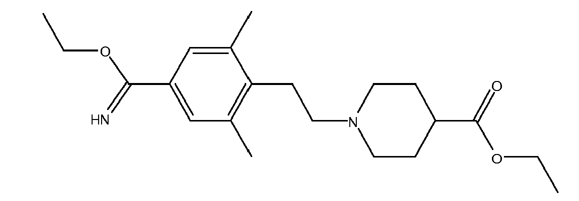

To Preparation 146 (250 mg, 0.79 mmol) saturated HCl in ethanol (5 mL, 108.2 mmol) was added at 0°C and mixture was stirred at this temperature for 48 h. Solvent was removed to yield the title compound as a brown solid (78% yield).
LRMS: m/z 361 (M+1)⁺
Retention time: 3.13 min (Method B)

### Preparation 148

### Ethyl 1-(4-(5-(1-ethyl-5-isobutyl-1H-pyrazol-3-yl)-4H-1,2,4-triazol-3-yl)-2,6-dimethyl phenethyl)piperidine-4-carboxylate

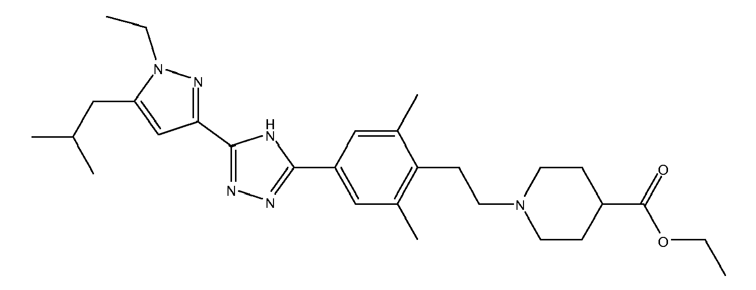

To a solution of Preparation 145 (60 mg, 0.29 mmol) in ethanol (2 mL) triethylamine (0.8 mL, 5.74 mmol) and molecular sieves were added and mixture stirred at room temperature for 3 h. Then Preparation 147 (125 mg, 0.35 mmol) was added and mixture stirred overnight at 80°C. Solvent was removed and crude redissolved in ethyl acetate, washed with water and brine, dried over magnesium sulphate, filtered and concentrated to yield the title compound as a browny oli (58% yield)..
LRMS: m/z 507 (M+1)⁺
Retention time: 5.80 min (Method B)

### Preparation 149

### Ethyl 5-phenyl-1H-pyrazole-3-carboxylate

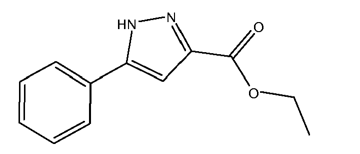

To a solution of Preparation 28 in ethanol (350 mL) hydrazine hydrate (2.51 g, 16.72 mmol) in ethanol (70 mL) and triethylamine (2.33 mL, 16.72 mmol) were added and mixture stirred at room temperature for 1 h. Solvent was removed and crude used for next reaction withouth further purification.
LRMS: m/z 217 (M+1)⁺
Retention time: 5.77 min (Method B)

### Preparation 150

### 1-(Cyclopropylmethyl)-5-phenyl-1H-pyrazole-3-carboxylic acid

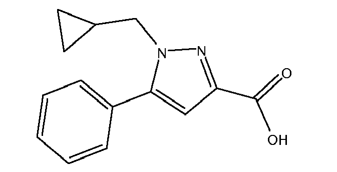

Obtained (14%) from Preparation 149 and (bromomethyl)cyclopropane following the procedure described in Preparation 23, using THF as solvent.
LRMS: m/z 243 (M+1)⁺
Retention time: 5.73 min (Method B)
¹H NMR (400 MHz, CHLOROFORM*-d*) δ ppm 0.19 (q, *J*=4.95 Hz, 2 H) 0.38-0.58 (m, 2 H) 1.08-1.31 (m, 1 H) 4.10 (d, *J*=7.03 Hz, 2 H) 6.90 (s, 1 H) 7.32-7.56 (m, 5 H).

### Preparation 151

### Ethyl 1-(4-(5-(1-(cyclopropylmethyl)-5-phenyl-1H-pyrazol-3-yl)-1,2,4-oxadiazol-3-yl)-2,6-dimethylphenethyl)piperidine-4-carboxylate

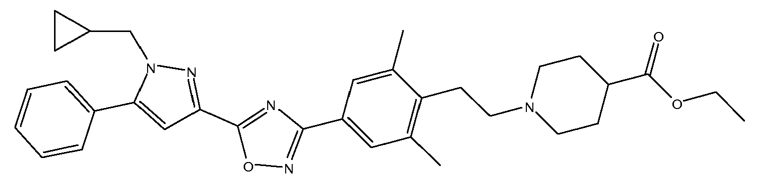

Obtained (55%) from Preparation 150 and Preparation 109 following General Method 2.
LRMS: m/z 554 (M+1)⁺
Retention time: 5.90 min (Method B)

### Preparation 152

### Methyl 1-ethyl-5-hydroxy-1H-pyrazole-3-carboxylate

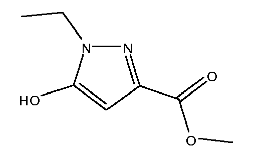

To a suspension of ethylhydrazine oxalic acid in THF triethylamine was added and mixture stirred at room temperature for 30 min. Then dimethyl but-2-ynedioate was added and reaction stirred at 80°C for 15 h. Salts were filtered off and filtrate was concentrated and purified by normal phase chromatography using hexane/ethyl acetate from 0% to 60% to yield the title compound (14% yield).
LRMS: m/z 171 (M+1)⁺
Retention time: 3.80 min (Method B)
¹H NMR (400 MHz, DMSO*-d*₆) δ ppm 1.27 (t, *J*=7.23 Hz, 3 H) 3.73 (s, 3 H) 3.94 (q, *J*=7.03 Hz, 2 H) 5.77 (s, 1 H).

### Preparation 153

### Methyl 1-ethyl-5-isopropoxy-1H-pyrazole-3-carboxylate

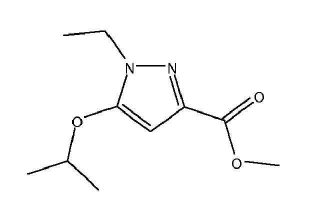

To a solution of Preparation 152 (230 mg, 1.35 mmol) in DMF (10 mL) K₂CO₃ (560 mg, 4.05 mmol) and 2-iodopropane (410 mL, 4.11 mmol) was added and mixture stirred at 80°C overnight. Ethyl acetate and water were added and organic layer washed with water and brine, dried over magnesium sulphate, filtered and concentrated to yield the title compound as an oil (100% yield).
LRMS: m/z 213 (M+1)⁺
Retention time: 5.42 min (Method B)

### Preparation 154

### 1-Ethyl-5-isopropoxy-1H-pyrazole-3-carboxylic acid

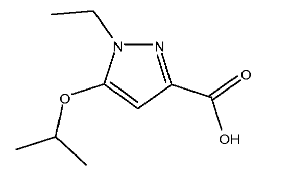

Obtained (95%) from Preparation 153 following General Method 3.
LRMS: m/z 199 (M+1)⁺
Retention time: 4.95 min (Method B)

### Preparation 155

### 3-(4-Allyl-3,5-dimethylphenyl)-5-(1-ethyl-5-isopropoxy-1H-pyrazol-3-yl)-1,2,4-oxadiazole

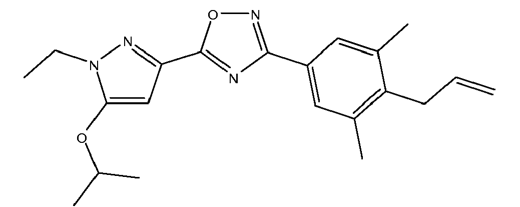

Obtained (66%) from Preparation 154 and Preparation 3 following General Method 2, using NMP as solvent.
LRMS: m/z 367 (M+1)⁺
Retention time: 7.80 min (Method B)

### Preparation 156

### 2-(4-(5-(1-ethyl-5-isopropoxy-1H-pyrazol-3-yl)-1,2,4-oxadiazol-3-yl)-2,6-di methylphenyl)acetaldehyde

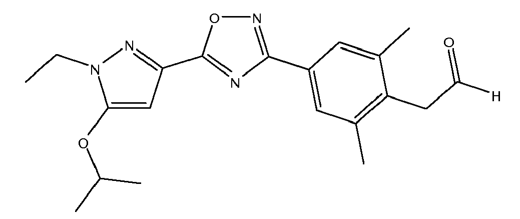

Obtained (65%) from Preparation 155 following the procedure described in Preparation 42.
LRMS: m/z 369 (M+1)⁺
Retention time: 7.00 min (Method B)

### Preparation 157

### 1-Ethyl-5-isobutyl-N'-(4-methoxy-3,5-dimethylbenzoyl)-1H-pyrazole-3-carbohydrazide

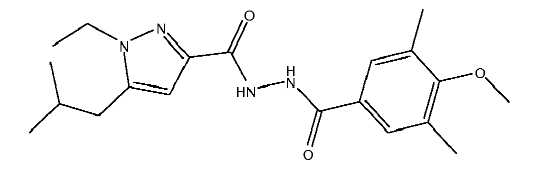

Obtained (90%) from Preparation 79 and Preparation 118 following General Method 5.
LRMS: m/z 373 (M+1)⁺
Retention time: 6.00 min (Method B)

### Preparation 158

### 2-(1-Ethyl-5-isobutyl-1H-pyrazol-3-yl)-5-(4-methoxy-3,5-dimethylphenyl)-1,3,4-thiadiazole

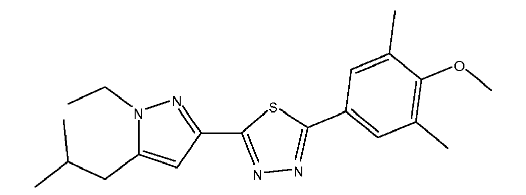

To a solution of Preparation 157 in toluene (40 mL) Laweson Reagent was added and mixture stirred at 105°C for 12 h. Solvent was removed and ethyl acetate and water was added, organic layer was dried over magnesium sulphate, filtered and concentrated to yield the title compound as a white solid (84% yield).
LRMS: m/z 371 (M+1)⁺
Retention time: 7.52 min (Method B)

### Preparation 159

### 4-(5-(1-Ethyl-5-isobutyl-1H-pyrazol-3-yl)-1,3,4-thiadiazol-2-yl)-2,6-dimethylphenol

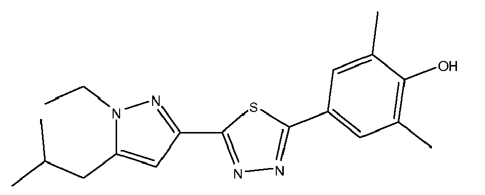

Obtained (91%) from Preparation 158 following the procedure described in Preparation 121.
LRMS: m/z 357 (M+1)⁺
Retention time: 7.52 min (Method B)

### Preparation 160

### 4-(5-(1-Ethyl-5-isobutyl-1H-pyrazol-3-yl)-1,3,4-thiadiazol-2-yl)-2,6-dimethylphenyl trifluoromethanesulfonate

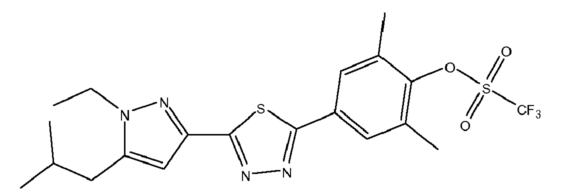

Obtained (68%) from Preparation 159 following the procedure described in Preparation 1.
LRMS: m/z 489 (M+1)⁺
Retention time: 7.93 min (Method B)

### Preparation 161

### 2-(4-Allyl-3,5-dimethylphenyl)-5-(1-ethyl-5-isobutyl-1H-pyrazol-3-yl)-1,3,4-thiadiazole

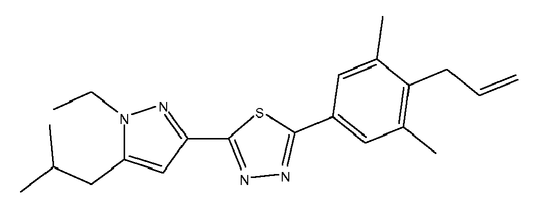

Obtained (50%) from Preparation 160 following the procedure described in Preparation 2.
LRMS: m/z 381 (M+1)⁺
Retention time: 7.85 min (Method B)

### Preparation 162

### 2-(4-(5-(1-Ethyl-5-isobutyl-1H-pyrazol-3-yl)-1,3,4-thiadiazol-2-yl)-2,6-dimethylphenyl)acetaldehyde

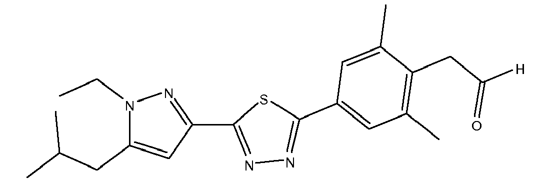

Obtained (5%) from Preparation 161 following the procedure described in Preparation 42.
LRMS: m/z 383 (M+1)⁺
Retention time: 7.15 min (Method B)

### Preparation 163

### 5-Cyclohexyl-1-ethyl-1H-pyrazole-3-carboxamide

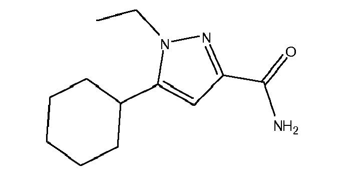

To Preparation 72 (1 g, 4.5 mmol) SOCl₂ (0.3 mL, 48 mmol) was added and mixture heated to reflux for 90 min. Then solvent was removed, crude redissolved in dioxane, 0.5M NH3 (24 mL, 12 mmol) in dioxane was added and mixture stirred at room temperature for 48 h. Reaction was concentrated, dichloromethane was added and suspension washed with saturated NaHCO₃. Organic layer was dried over magnesium sulphate, filtered and concentrated to yield the title compound as a yellow oil (99% yield).
LRMS: m/z 222 (M+1)⁺
Retention time: 5.50 min (Method B)

### Preparation 164

### 5-Cyclohexyl-1-ethyl-1H-pyrazole-3-carbonitrile

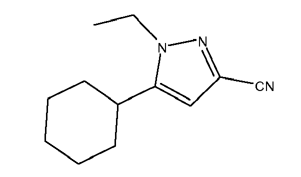

To a solution of Preparation 163 (2.82 g, 12.74 mmol) in pyridine (19 mL) at 0°C POCl₃ (1.40 mL, 15.34 mmol) was slowly added and mixture was stirred at room temperature for 2 h. Solvent was removed, diethyl ether was added and it was washed with water and brine, organic layer was dried over magnesium sulphate, filtered and concentrated to yield the title compound as a yellow solid (94% yield).
LRMS: m/z 204 (M+1)⁺
Retention time: 6.48 min (Method B)
¹H NMR (400 MHz, CHLOROFORM*-d*) δ ppm 1.19 - 1.43 (m, 5 H) 1.47 (t, *J*=7.23 Hz, 3 H) 1.64 - 2.04 (m, 5 H) 2.48 - 2.69 (m, 1 H) 4.13 (q, *J*=7.16 Hz, 2 H) 6.38 (s, 1 H).

### Preparation 165

### 5-Cyclohexyl-1-ethyl-N'-hydroxy-1H-pyrazole-3-carboximidamide

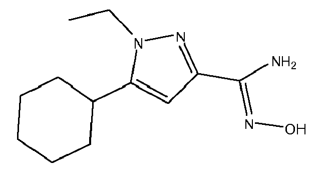

Obtained (26%) from Preparation 164 following General Method 1.
LRMS: m/z 237 (M+1)⁺
Retention time: 4.22 min (Method B)

### Preparation 166

### 4-Methoxy-3,5-dimethylbenzoic acid

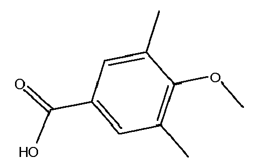

To a suspension of 4-methoxy-3,5-dimethylbenzonitrile in ethanol (32 mL) 8M NaOH (163 mL) was added and mixture stirred at 120°C overnight in a pressure reactor. Ethanol was removed, water was added and solution was washed with diethyl ether. Then aquous layer was acidified with concentrated HCl and product extracted with ethyl acetate, organic layer was washed with water and brine, dried over magnesium sulphate, filtered and concentrated to yield the title compound as a white solid (99% yield).
LRMS: m/z 181 (M+1)⁺
Retention time: 5.52 min (Method B)

### Preparation 167

### 3-(5-cyclohexyl-1-ethyl-1H-pyrazol-3-yl)-5-(4-methoxy-3,5-dimethylphenyl)-1,2,4-oxadiazole

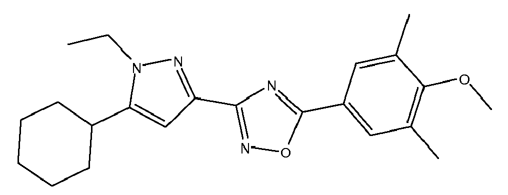

Obtained (53%) from Preparation 165 and Preparation 166 following General Method 2.
LRMS: m/z 381 (M+1)⁺
Retention time: 7.82 min (Method B)

### Preparation 168

### 4-(3-(5-Cyclohexyl-1-ethyl-1H-pyrazol-3-yl)-1,2,4-oxadiazol-5-yl)-2,6-dimethylphenol

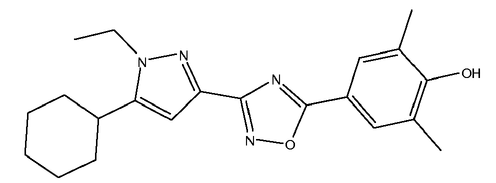

Obtained (30%) from Preparation 167 following the procedure described in Preparation 121.
LRMS: m/z 367 (M+1)⁺
Retention time: 7.42 min (Method B)
¹H NMR (400 MHz, CDCl3*-d*) δ ppm 1.21 - 1.48 (m, 5 H) 1.50 (t, *J*=7.23 Hz, 3 H) 1.72 - 2.01 (m, 5 H) 2.32 (s, 6 H) 2.61 (td, *J*=11.04, 2.93 Hz, 1 H) 4.23 (q, *J*=7.03 Hz, 2 H) 6.65 (s, 1 H) 7.92 (s, 2 H).

### Preparation 169

### 4-(3-(5-Cyclohexyl-1-ethyl-1H-pyrazol-3-yl)-1,2,4-oxadiazol-5-yl)-2,6-dimethylphenyl trifluoromethanesulfonate

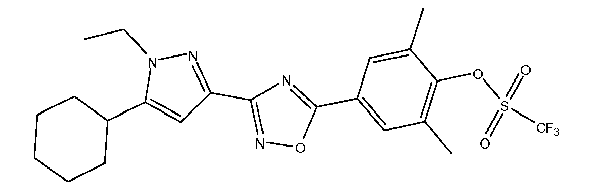

Obtained (68%) from Preparation 168 following the procedure described in Preparation 1.
LRMS: m/z 499 (M+1)⁺
Retention time: 8.05 min (Method B)

### Preparation 170

### 5-(4-Allyl-3,5-dimethylphenyl)-3-(5-cyclohexyl-1-ethyl-1H-pyrazol-3-yl)-1,2,4-oxadiazole

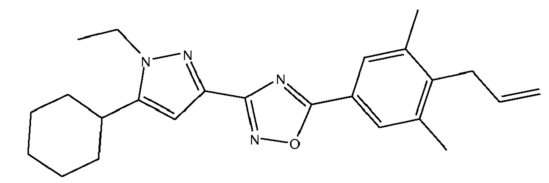

Obtained (100%) from Preparation 169 following the procedure described in Preparation 2.
LRMS: m/z 391 (M+1)⁺
Retention time: 7.45 min (Method B)

### Preparation 171

### 2-(4-(3-(5-Cyclohexyl-1-ethyl-1H-pyrazol-3-yl)-1,2,4-oxadiazol-5-yl)-2,6-dimethylphenyl)acetaldehyde

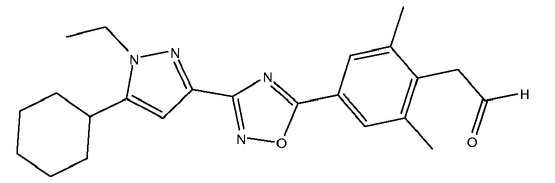

Obtained (100%) from Preparation 170 following the procedure described in Preparation 42.
LRMS: m/z 393 (M+1)⁺
Retention time: 7.45 min (Method B)

### Preparation 172

### Ethyl 1H-pyrazole-3-carboxylate

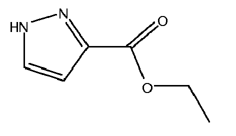

Obtained (72%) from 1H-pyrazole-3-carboxylic acid following the procedure described in Preparation 117, using ethanol as solvent.
LRMS: m/z 139 (M-1)⁺
Retention time: 2.13 min (Method A)

### Preparation 173

### Ethyl 1-ethyl-1H-pyrazole-3-carboxylate

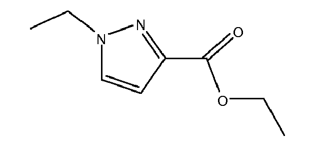

Obtained (35%) from Preparation 172 and iodoethane following the procedure described in Preparation 23, using dioxane as solvent.
LRMS: m/z 169 (M+1)⁺
Retention time: 2.40 min (Method A)
¹H NMR (400 MHz, CHLOROFORM*-d*) δ ppm 1.38 (t, *J*=7.23 Hz, 3 H) 1.44 (t, *J*=7.23 Hz, 3 H) 4.35 (q, *J*=7.16 Hz, 2 H) 4.61 (q, *J*=7.29 Hz, 2 H) 6.83 (d, *J*=1.95 Hz, 1 H) 7.48 (d, *J*=1.95 Hz, 1 H).

### Preparation 174

### Ethyl 4-bromo-1-ethyl-1H-pyrazole-3-carboxylate

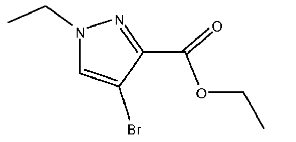

To a solution of Preparation 173 (1.33 g, 7.91 mmol) in chloroform (2 mL) at 0°C bromine (0.41 mL, 8 mmol) in chloroform (1 mL) was slowly added and mixture stirred at 0°C for 30 min. Reaction was diluted with chloroform and washed with saturated NaHCO₃ and brine, dried over sodium sulphate, filtered and concentrated. Crude thus obtained was purified by normal phase chromatography using hexane/ethyl acetate from 0% to 30% to yield the title compound as a colorless oil (62% yield).
LRMS: m/z 249 (M+1)⁺
Retention time: 5.32 min (Method B)
¹H NMR (400 MHz, CHLOROFORM*-d*) δ ppm 1.41 (t, 3 H) 1.52 (t, 3 H) 4.24 (q, *J*=7.42 Hz, 2 H) 4.43 (q, *J*=7.03 Hz, 2 H) 7.51 (s, 1 H).

### Preparation 175

### Ethyl 1-ethyl-4-phenyl-1H-pyrazole-3-carboxylate

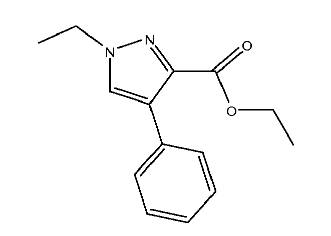

Obtained (51%) from Preparation 174 and phenylboronic acid following the procedure described in Preparation 142 using Pd(PPh₃)₄ as catalyst and heating to 140°C.
LRMS: m/z 245 (M+1)⁺
Retention time: 5.80 min (Method B)

### Preparation 176

### 1-Ethyl-4-phenyl-1H-pyrazole-3-carboxylic acid

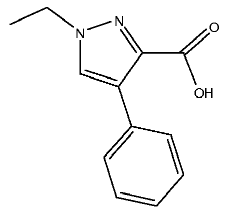

Obtained (100%) from Preparation 175 following General Method 3.
LRMS: m/z 217 (M+1)⁺
Retention time: 4.95 min (Method B)

### Preparation 177

### Ethyl 1-(4-(5-(1-ethyl-4-phenyl-1H-pyrazol-3-yl)-1,2,4-oxadiazol-3-yl)-2,6-dimethyl phenethyl)piperidine-4-carboxylate

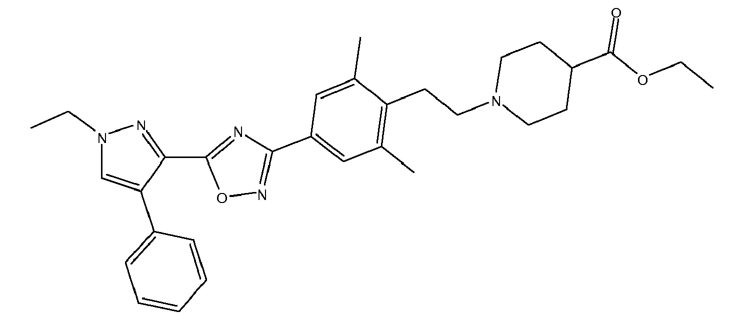

Obtained (66%) from Preparation 176 and Preparation 109 following General Method 2.
LRMS: m/z 528 (M+1)⁺
Retention time: 5.33 min (Method B)

### Preparation 178

### Ethyl 1-(4-(5-(1-ethyl-4-phenyl-1H-pyrazol-3-yl)-1,2,4-oxadiazol-3-yl)-2-(trifluoro methyl)phenethyl)piperidine-4-carboxylate

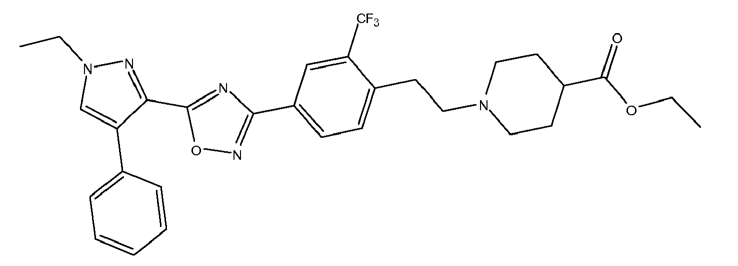

Obtained (76%) from Preparation 176 and Preparation 135 following General Method 2.
LRMS: m/z 568 (M+1)⁺
Retention time: 5.47 min (Method B)

### Preparation 179

### Ethyl 1-(4-(5-(1-ethyl-5-isobutyl-1H-pyrazol-3-yl)-1,2,4-oxadiazol-3-yl)-2-(trifluoro methyl)phenethyl)piperidine-4-carboxylate

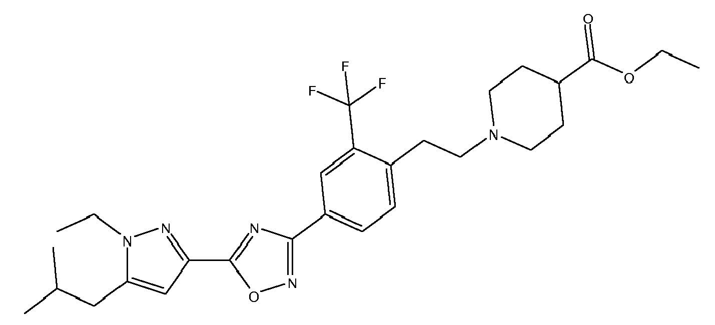

Obtained (65%) from Preparation 79 and Preparation 135 following General Method 2.
LRMS: m/z 548 (M+1)⁺
Retention time: 5.97 min (Method B)

### Preparation 180

### 3-(4-Allyl-3-(trifluoromethyl)phenyl)-5-(1-ethyl-5-isobutyl-1H-pyrazol-3-yl)-1,2,4-oxadiazole

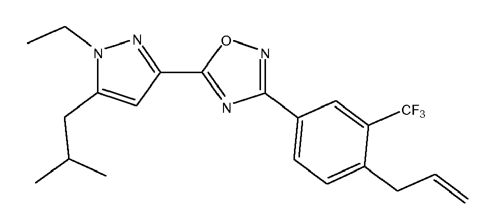

Obtained (66%) from Preparation 79 and Preparation 65 following General Method 2 in NMP.
LRMS: m/z 405 (M+1)⁺
Retention time: 8.00 min (Method B)
¹H NMR (400 MHz, CHLOROFORM*-d*) δ ppm 1.02 (d, *J*=6.64 Hz, 6 H) 1.51 (t, *J*=7.23 Hz, 3 H) 1.88 - 2.19 (m, 1 H) 2.57 (d, *J*=7.03 Hz, 2 H) 3.63 (d, *J*=6.25 Hz, 2 H) 4.25 (q, *J*=7.29 Hz, 2 H) 5.15 (d, *J*=8.60 Hz, 2 H) 5.77 - 6.21 (m, *J*=16.80, 10.16, 6.64, 6.64 Hz, 1 H) 6.77 (s, 1 H) 7.49 (d, *J*=7.82 Hz, 1 H) 8.30 (d, *J*=7.82 Hz, 1 H) 8.49 (s, 1 H).

### Preparation 181

### 2-(4-(5-(1-ethyl-5-isobutyl-1H-pyrazol-3-yl)-1,2,4-oxadiazol-3-yl)-2-(trifluoromethyl)phenyl)acetaldehyde

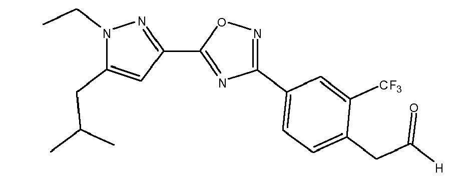

Obtained (96%) from Preparation 180 following the procedure described in Preparation 42.
LRMS: m/z 407 (M+32)⁺
Retention time: 7.28 min (Method B)

### Preparation 182

### Ethyl 5-amino-1-ethyl-1H-pyrazole-3-carboxylate

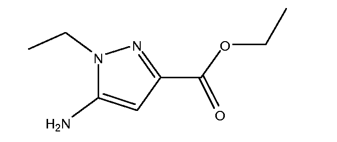

To a suspensión of ethyl 3-cyano-2-oxopropanoate in dioxane (200 mL) trifluoroacetic acid was added followed by a suspension of ethylhydrazine oxalic acid in dioxane (50 mL) and reaction was stirred at 100°C for 2.5 h. Precipitated salts were removed by filtration and filtrate was concentrated
LRMS: m/z 184 (M+1)⁺
Retention time: 3.20 min (Method B)

### Preparation 183

### Ethyl 5-(diethylamino)-1-ethyl-1H-pyrazole-3-carboxylate

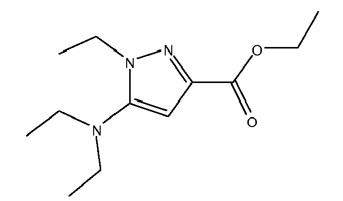

Obtained (10%) from Preparation 182 and acetaldehyde following General Method 6.
LRMS: m/z 240 (M+1)⁺
Retention time: 3.20 min (Method B)

### Preparation 184

### 5-(Diethylamino)-1-ethyl-1H-pyrazole-3-carboxylic acid

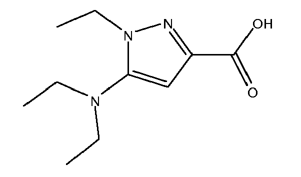

Obtained (66%) from Preparation 183 following General Method 3.
LRMS: m/z 212 (M+1)⁺
Retention time: 2.73 min (Method B)

### Preparation 185

### tert-Butyl 3-(4-(5-(5-(diethylamino)-1-ethyl-1H-pyrazol-3-yl)-1,2,4-oxadiazol-3-yl)-2, 6-dimethylphenyl)propanoate

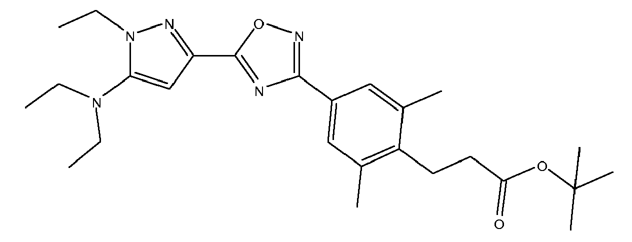

Obtained (26%) from Preparation 184 and Preparation 115 following General Method 2.
LRMS: m/z 468 (M+1)⁺
Retention time: 8.00 min (Method B)

### Preparation 186

### Methyl 1-ethyl-5-propoxy-1H-pyrazole-3-carboxylate

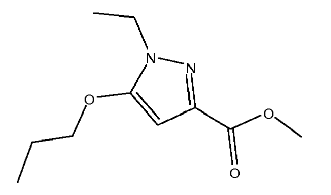

Obtained (91%) from Preparation 152 and 1-iodopropane following the procedure described in Preparation 153.
LRMS: m/z 213 (M+1)⁺
Retention time: 5.57 min (Method B)

### Preparation 187

### 1-Ethyl-5-propoxy-1H-pyrazole-3-carboxylic acid

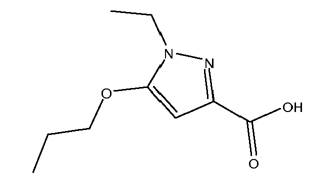

Obtained (86%) from Preparation 186 following General Method 3.
LRMS: m/z 199 (M+1)⁺
Retention time: 5.08 min (Method B)

### Preparation 188

### Ethyl 1-(4-(5-(1-ethyl-5-propoxy-1H-pyrazol-3-yl)-1,2,4-oxadiazol-3-yl)-2,6-dimethyl phenethyl)piperidine-4-carboxylate

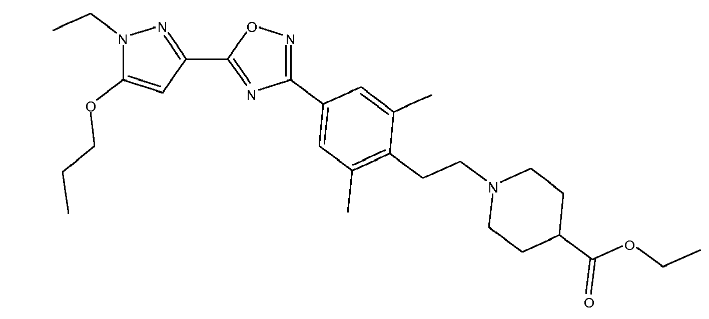

Obtained (89%) from Preparation 187 and Preparation 109 following General Method 2.
LRMS: m/z 510 (M+1)⁺
Retention time: 5.62 min (Method B)

### Preparation 189

### Ethyl1-(4-(5-(1-ethyl-5-isopropoxy-1H-pyrazol-3-yl)-1,2,4-oxadiazol-3-yl)-2-(trifluoromethyl)phenethyl)piperidine-4-carboxylate

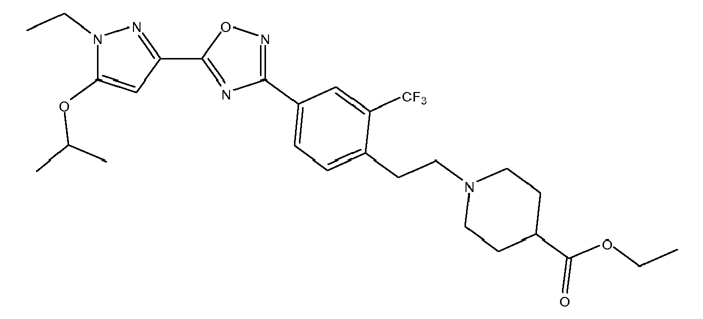

Obtained (90%) from Preparation 154 and Preparation 134 following General Method 2.
LRMS: m/z 550 (M+1)⁺
Retention time: 5.60 min (Method B)

### EXAMPLES

### EXAMPLE 1

### 3-{2,6-dimethyl-4-[5-(1-methyl-5-phenyl-1H-pyrazol-3-yl)-1,2,4-oxadiazol-3-yl]phenyl}propane-1,2-diol

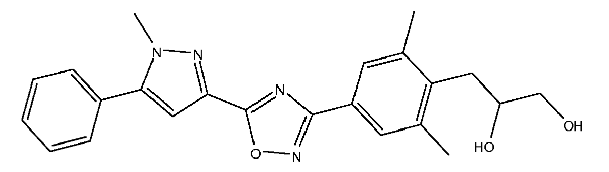

To a solution of Preparation 4 (0.59 g, 1,59 mmol) in a mixture of THF/tert-butanol (13 mL / 2 mL) 4-methylmorpholine 4-oxide (0.373 g, 3,28 mmol) and osmium(VIII) oxide (0.194 mL, 0,03 mmol) were added. The reaction mixture was stirred overnight at r.t. Then 40% solution of Na₂SO₃ was added and the mixture stirred for 30 min. Ethyl acetate was added and the organic layer separated, washed twice with water, dried over magnesium sulphate and concentrated to give the title compound (85% yield).
LRMS: m/z 405 (M+1)⁺
Retention time: 16.66 min (Method C)
¹H NMR (400 MHz, DMSO-d6) δ ppm 2.41 (s, 6 H) 2.68 (dd, J=13.50, 8.80 Hz, 1 H) 2.89 (dd, J=13.50, 3.91 Hz, 1 H) 3.33 - 3.37 (m, 1 H) 3.38 - 3.45 (m, 1 H) 3.61 - 3.71 (m, 1 H) 4.03 (s, 3 H) 4.66 (d, J=5.09 Hz, 1 H) 4.69 (t, J=5.67 Hz, 1 H) 7.24 (s, 1 H) 7.49 - 7.61 (m, 3 H) 7.67 (d, J=7.04 Hz, 2 H) 7.71 (s, 2 H).

### EXAMPLES 2 to 4

### N-(2-{2,6-dimethyl-4-[5-(1-methyl-5-phenyl-1H-pyrazol-3-yl)-1,2,4-oxadiazol-3-yl]phenyl}ethyl)-2,2,2-trifluoroethanamine

### N-(2-{2,6-dimethyl-4-[5-(1-methyl-5-phenyl-1H-pyrazol-3-yl)-1,2,4-oxadiazol-3-yl]phenyl}ethyl)glycine

### 1-(2-{2,6-dimethyl-4-[5-(1-methyl-5-phenyl-1H-pyrazol-3-yl)-1,2,4-oxadiazol-3-yl]phenyl}ethyl)piperidine-4-carboxylic acid

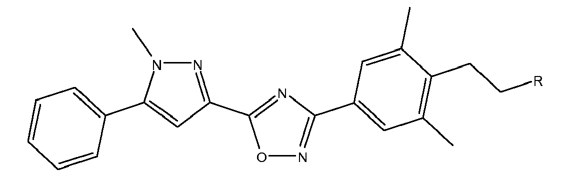

Examples 2 to 4 were obtained as hydrochloride salts from Preparation 5 and the corresponding amine or amino acids following General Method 6.

| **EXAMPLE** | **R** | **tᵣ (Method C) (min)** | **LRMS** **(M+1)** | **1H NMR δppm** |
|---|---|---|---|---|
| 2 | 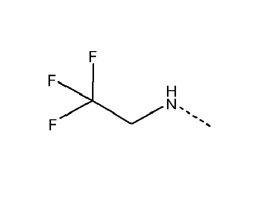 | 18.28 | 456 | ¹H NMR (200 MHz, DMSO-d6) δ ppm 2.45 (s, 6 H) 3.03-3.12 (m, 2 H) 3.35 - 3.42 (m, 4 H) 4.03 (s, 3 H) 4.04 - 4.17 (m, 2 H) 7.23 (s,1H) 7.50-7.60 (m, 3 H) 7.63 - 7.72 (m,2H)7.77(s,2H) |
| 3 | 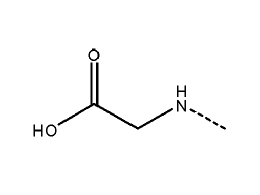 | 14.70 | 432 | ¹H NMR (200 MHz, DMSO-d6) δ ppm 2.43 (s, 6 H) 2.94-3.17 (m, 4 H) 3.27-3.41 (m, 2 H) 3.96 (s, 2 H) 4.03 (s, 3 H) 7.24 (s, 1 H) 7.42-7.84 (m, 7 H) 9.44 (s, 1H) |
| 4 | 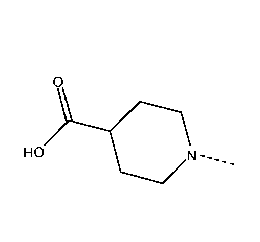 | 12.37 | 486 | ¹H NMR (400 MHz, DMSO-d₆) δ ppm 1.83 (br. s., 2 H) 2.11 (br. s., 2 H) 2.45 (s, 6 H) 2.82-3.23 (m, 6 H) 3.70 (br. s., 1 H) 4.03 (s, 3 H) 7.24 (s, 1 H) 7.45-7.61 (m, 3 H) 7.67 (d, *J*=6.64 Hz, 2 H) 7.77 (s, 2 H) 10.18 (br. s., 1 H) |

### EXAMPLE 5

### 3-ethyl-6-methyl-5-[5-(1-methyl-5-phenyl-1H-pyrazol-3-yl)-1,2,4-oxadiazol-3-yl]pyridin-2-ol

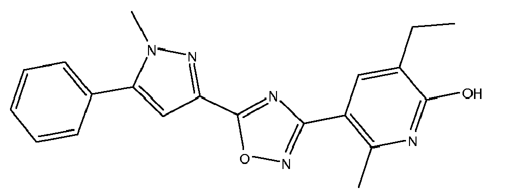

A mixture of Preparation 11 (270 mg, 0,72 mmol) and HBr 48% (7.81 mL) in THF (1 mL) was stirred at 100°C for 2 h in a pressure reactor. 8 N NaOH was slowly added until pH 6-7, product was extracted with chloroform, organic layer was washed with water and brine, dried over magnesium sulphate and concentrated to yield the desired compound as a white solid (69% yield).
LRMS: m/z 362 (M+1)⁺
Retention time: 16.23 min (Method B)
¹H NMR (300 MHz, DMSO*-d*₆) δ ppm 1.19 (t, *J*=7.55 Hz, 3 H) 2.50 (q, 2 H) 2.64 (s, 3 H) 4.08 (s, 3 H) 7.28 (s, 1 H) 7.50 - 7.67 (m, 3 H) 7.68 - 7.79 (m, 2 H) 7.88 (s, 1H) 12.11 (br. s., 1 H).

### EXAMPLE 6

### (2-{2-ethyl-6-methyl-4-[5-(1-methyl-5-phenyl-1H-pyrazol-3-yl)-1,2,4-oxadiazol-3-yl]phenyl}ethyl)amine

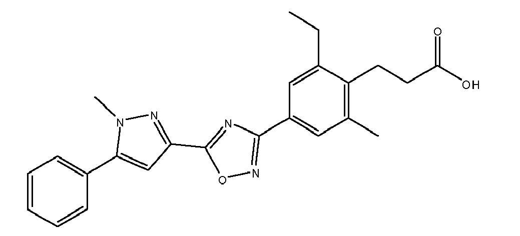

Obtained (93% yield) from Preparation 18 following General Method 3.
LRMS: m/z 417 (M+1)⁺
Retention time: 18.36 min (Method B)
¹H NMR (400 MHz, DMSO-d6) δ ppm 1.22 (t, J=7.56 Hz, 3 H) 2.13 - 2.24 (m, 2 H) 2.39 (s, 3 H) 2.73 (q, J=7.56 Hz, 2 H) 2.89 (m, 2 H) 4.03 (s, 3 H) 7.25 (s, 1 H) 7.49 - 7.63 (m, 3 H) 7.64 - 7.78 (m, 4 H).

### EXAMPLE 7

### (2-{2-ethyl-6-methyl-4-[5-(1-methyl-5-phenyl-1H-pyrazol-3-yl)-1,2,4-oxadiazol-3-yl]phenyl}ethyl)amine

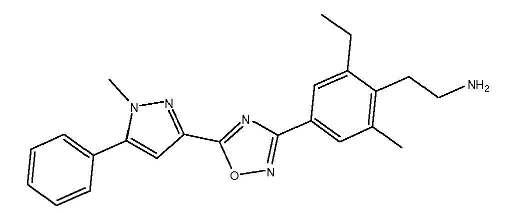

Obtained (62% yield) as hydrochloride salt from Example 6 following General Method 7.
LRMS: m/z 388 (M+1)⁺
Retention time: 12.16 min (Method B)
¹H NMR (400 MHz, DMSO-d6) δ ppm 1.21 (t, J=7.42 Hz, 3 H) 2.41 (s, 3 H) 2.74 (q, J=7.42 Hz, 2 H) 2.78 - 2.88 (m, 2 H) 2.94 - 3.06 (m, 2 H) 4.00 (s, 3 H) 7.21 (s, 1 H) 7.47 - 7.58 (m, 3 H) 7.64 (d, J=6.64 Hz, 2 H) 7.74 (s, 2 H) 8.09 (s, 3 H).

### EXAMPLE 8

### [3-({3-ethyl-6-methyl-5-[5-(1-methyl-5-phenyl-1H-pyrazol-3-yl)-1,2,4-oxadiazol-3-yl]pyridin-2-yl}oxy)propyl]amine

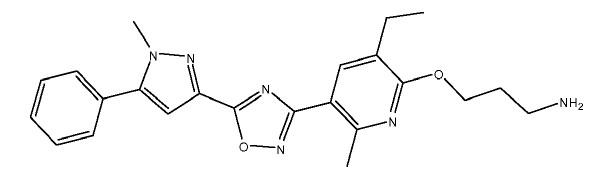

Obtained (86% yield) as hydrochloride salt from Preparation 19 following General Method 4.
LRMS: m/z 419 (M+1)⁺
Retention time: 12.92 min (Method B)
¹H NMR (300 MHz, DMSO*-d*₆) δ ppm 1.26 (t, 3 H) 1.93 - 2.30 (m, 2 H) 2.70 (q, 2 H) 2.80 (s, 3 H) 3.06 (t, 2 H) 4.11 (s, 3 H) 4.34 - 4.66 (m, 2 H) 7.32 (br. s., 0 H) 7.52 - 8.07 (m, 7 H) 8.19 (s, 2 H).

### EXAMPLE 9

### (2-{2,6-dimethyl-4-[5-(1-methyl-5-phenyl-1H-pyrazol-3-yl)-1,2,4-oxadiazol-3-yl]phenoxy}ethyl)amine

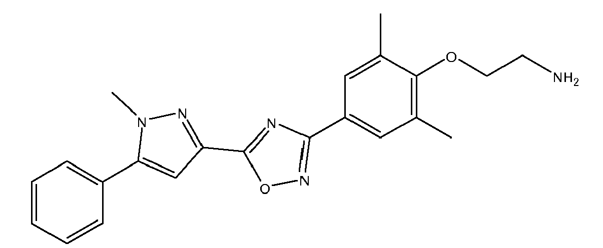

Obtained (87% yield) as hydrochloride salt from Preparation 22 following General Method 4.
LRMS: m/z 390 (M+1)⁺
Retention time: 11.70 min (Method B)
¹H NMR (400 MHz, DMSO-d6) d ppm 2.37 (s, 6 H) 3.20 - 3.29 (m, 2 H) 3.90 - 4.12 (m, 5 H) 7.23 (s, 1 H) 7.51 - 7.61 (m, 3 H) 7.67 (d, J=7.03 Hz, 2 H) 7.81 (s, 2 H) 8.27 (s, 3 H).

### EXAMPLE 10

### 2-{2,6-dimethyl-4-[5-(1-methyl-5-phenyl-1H-pyrazol-3-yl)-1,2,4-oxadiazol-3-yl]phenoxy}-2,2-difluoroacetamide

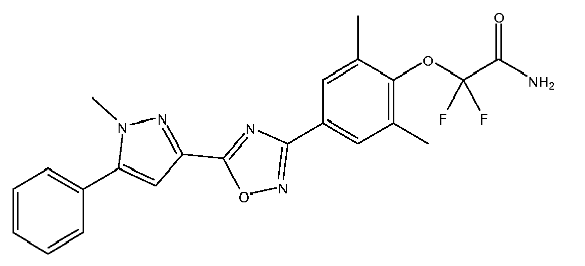

To Preparation 23 (98 mg, 0.21 mmol) NH₃ 7N in MeOH (5 mL) was added and mixture stirred at r.t. for 4 h. Solvent was removed and crude purified by normal phase chromatography with hexane/ethyl acetate 1:1 to yield the title compound (94% yield).
LRMS: m/z 440 (M+1)⁺
Retention time: 17.63 min (Method B)
¹H NMR (400 MHz, DMSO*-d*6) δ ppm 2.37 (s, 6 H) 4.03 (s, 3 H) 7.25 (s, 1 H) 7.49 - 7.62 (m, 3 H) 7.67 (d, J=6.64 Hz, 2 H) 7.88 (s, 2 H) 8.27 (s, 1 H) 8.54 (s, 1 H).

### EXAMPLE 11

### (2-{2,6-dimethyl-4-[5-(1-methyl-5-phenyl-1H-pyrazol-3-yl)-1,2,4-oxadiazol-3-yl]phenoxy}-2,2-difluoroethyl)amine

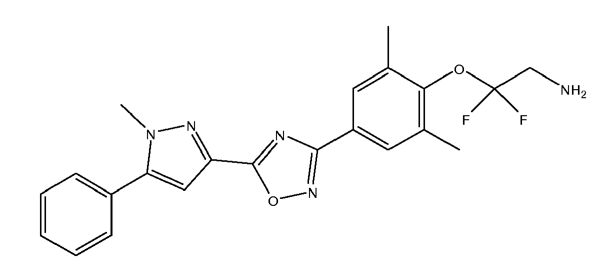

Obtained (25% yield) as hydrochloride salt from Example 10 following General Method 8.
LRMS: m/z 426 (M+1)⁺
Retention time: 12.75 min (Method B)
¹H NMR (400 MHz, DMSO-d6) δ ppm 2.38 (s, 6 H) 3.96 (t, J=8.79 Hz, 2 H) 4.04 (s, 3 H) 7.25 (s, 1 H) 7.50 - 7.62 (m, 3 H) 7.68 (d, J=7.03 Hz, 2 H) 7.89 (s, 2 H) 8.92 (s, 3 H).

### EXAMPLE 12

### {2-[4-[5-(1-methyl-5-phenyl-1H-pyrazol-3-yl)-1,2,4-oxadiazol-3-yl]-2-(trifluoromethyl)phenoxy]ethyl}amine

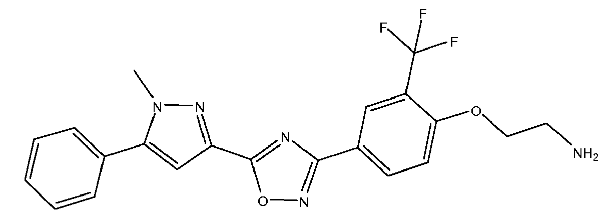

Obtained (89% yield) as hydrochloride salt from Preparation 26 following General Method 4.
LRMS: m/z 430 (M+1)⁺
Retention time: 11.90 min (Method B)
¹H NMR (300 MHz, METHANOL-*d*₄) δ ppm 3.47 (t, 2 H) 4.03 (s, 3 H) 4.47 (t, *J*=5.22 Hz, 2 H) 7.15 (s, 1 H) 7.37 - 7.65 (m, 6 H) 8.27 - 8.50 (m, 2 H).

### EXAMPLE 13

### 1-(2-{2,6-dimethyl-4-[5-(1-methyl-5-phenyl-1H-pyrazol-3-yl)-1,2,4-oxadiazol-3-yl]phenoxy}ethyl)piperidine-4-carboxylic acid

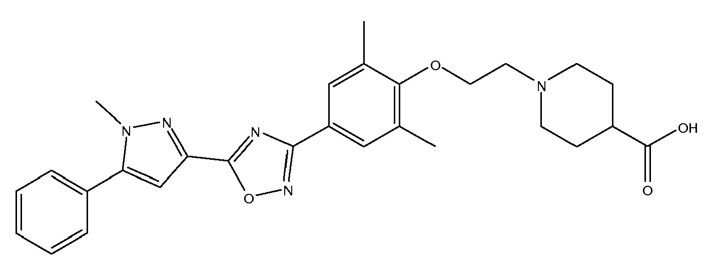

To a suspension of Preparation 27 (190 mg, 0.42 mmol) in acetonitrile (3 mL) ethyl piperidine-4-carboxylate (71 µL, 0.46 mmol) and triethylamine (65 µL, 0.47 mmol) were added under nitrogen atmosphere and mixture was heated at 70°C for 2 days. Reaction was diluted with ethyl acetate and washed with water and brine, dried over magnesium sulphate, filtered and concentrated. Crude was dissolved in ethanol (10 mL), 2N NaOH was added (5 mL) and mixture was stirred at r.t. overnight. Solvent was removed, water and glaciar acetic acid were added and product was extracted with ethyl acetate, organic layer was washed with water, dried over magnesium sulphate, filtered and concentrated. The white solid thus obtained was suspended in THF (10 mL) and 4N HCl in dioxane (10 mL) and stirred at r.t. overnight. Solvent was removed and the solid obtained was washed with diethyl ether to yield the title compound (35% yield).
LRMS: m/z 502 (M+1)⁺
Retention time: 12.56 min (Method B)
¹H NMR (400 MHz, DMSO-d6) δppm 1.86 - 2.01 (m, 2 H) 2.03 - 2.19 (m, 3 H) 2.38 (s, 6 H) 3.03 - 3.23 (m, 2 H) 3.47 - 3.76 (m, 4 H) 4.03 (s, 3 H) 4.15 - 4.30 (m, 2 H) 7.24 (s, 1 H) 7.47 - 7.61 (m, 3 H) 7.67 (d, J=6.64 Hz, 2 H) 7.81 (s, 2 H) 10.62 (s, 1H) 12.56 (s, 1H).

### EXAMPLE 14

### 3-ethyl-5-[5-(1-ethyl-5-phenyl-1H-pyrazol-3-yl)-1,2,4-oxadiazol-3-yl]-6-methylpyridin-2-ol

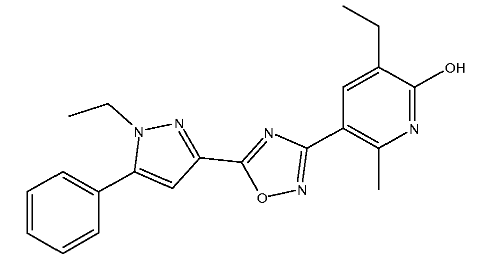

Obtained (82% yield) from Preparation 30 following the procedure described in Example 5.
LRMS: m/z 376 (M+1)⁺
Retention time: 17.44 min (Method B)
¹H NMR (400 MHz, DMSO-d6) δ ppm 1.05 - 1.17 (m, 3 H) 1.29 - 1.43 (m, 3 H) 2.40 - 2.46 (m, 2 H) 2.56 (s, 3 H) 4.28 (q, J=7.03 Hz, 2 H) 7.15 (s, 1 H) 7.46 - 7.66 (m, 5 H) 7.80 (s, 1 H) 12.02 (s, 1 H).

### EXAMPLE 15

### 5-{5-[5-(2-chlorophenyl)-1-ethyl-1H-pyrazol-3-yl]-1,2,4-oxadiazol-3-yl}-3-ethyl-6-methylpyridin-2-ol

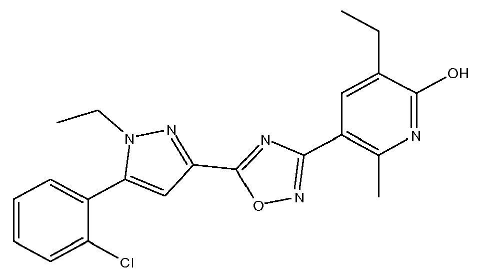

Obtained (72% yield) from Preparation 32 following the procedure described in Example 5.
LRMS: m/z 410 (M+1)⁺
Retention time: 17.95 min (Method B)
¹H NMR (400 MHz, DMSO-d6) δ ppm 1.13 (t, J=6.74 Hz, 3 H) 1.32 (t, J=6.74 Hz, 3 H) 2.45 (q, J=6.74 Hz, 2 H) 2.58 (s, 3 H) 4.06 (q, J=6.74 Hz, 2 H) 7.16 (s, 1 H) 7.49 - 7.65 (m, 3 H) 7.72 (d, J=7.03 Hz, 1 H) 7.82 (s, 1 H) 12.05 (s, 1 H).

### EXAMPLE 16

### {2-[4-[5-(1-methyl-5-phenyl-1H-pyrazol-3-yl)-1,2,4-oxadiazol-3-yl]-2-(trifluoromethyl)phenyl]ethyl}amine

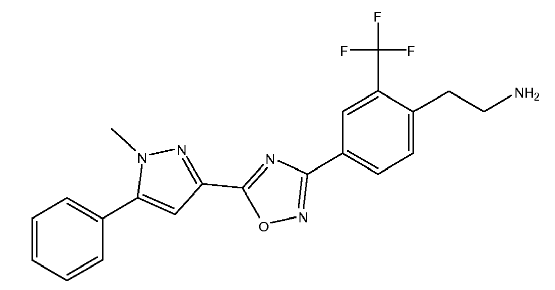

Obtained (28% yield) from Preparation 38 following the General Method 7.
LRMS: m/z 414 (M+1)⁺
Retention time: 12.19 min (Method B)
¹H NMR (400 MHz, DMSO*-d*₆) δ ppm 2.93 - 3.30 (m, 4 H) 4.04 (s, 3 H) 7.30 (s, 1 H) 7.45 - 7.65 (m, 3 H) 7.68 (d, *J*=6.64 Hz, 2 H) 7.82 (d, *J*=7.82 Hz, 1 H) 8.17 (br. s., 3 H) 8.29 - 8.49 (m, 2 H).

### EXAMPLE 17

### 5-{5-[3-(2-chlorophenyl)-1-ethyl-1H-pyrazol-5-yl]-1,2,4-oxadiazol-3-yl}-3-ethyl-6-methylpyridin-2-ol

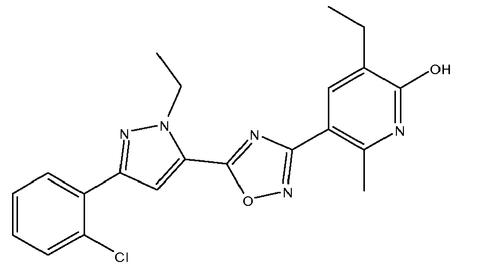

Obtained (86% yield) from Preparation 40 following the procedure described in Example 5.
LRMS: m/z 410 (M+1)⁺
Retention time: 20.41 min (Method B)
¹H NMR (400 MHz, DMSO*-d*₆) δ ppm 1.12 (t, *J*=7.42 Hz, 3 H) 1.49 (t, *J*=7.03 Hz, 3 H) 2.33 - 2.53 (m, 2 H) 2.54 - 2.67 (m, 3 H) 4.74 (q, *J*=6.90 Hz, 3 H) 7.30 - 7.52 (m, 2 H) 7.52 - 7.72 (m, 2 H) 7.72 - 7.96 (m, 2 H) 12.06 (s, 1 H).

### EXAMPLES 18 to 23

### 1-(2-{4-[5-(1-ethyl-5-phenyl-1H-pyrazol-3-yl)-1,2,4-oxadiazol-3-yl]-2,6-dimethylphenyl}ethyl)piperidine-4-carboxylic acid

### N-(2{4-[5-(1-ethyl-5-phenyl-1H-pyrazol-3-yl)-1,2,4-oxadiazol-3-yl]-2,6-dimethylphenyl}ethyl)glycine

### 1-(2-{4-[5-(1-ethyl-5-phenyl-1H-pyrazol-3-yl)-1,2,4-oxadiazol-3-yl]-2,6-dimethylphenyl}ethyl)azetidine-3-carboxylic acid

### N-(2-{4-[5-(1-ethyl-5-phenyl-1H-pyrazol-3-yl)-1,2,4-oxadiazol-3-yl]-2,6-dimethylphenyl}ethyl)-N-methylglycine

### [1-(2-{4-[5-(1-ethyl-5-phenyl-1H-pyrazol-3-yl)-1,2,4-oxadiazol-3-yl]-2,6-dimethylphenyl}ethyl)piperidin-4-yl]acetic acid

### 1-(2-{4-[5-(1-ethyl-5-phenyl-1H-pyrazol-3-yl)-1,2,4-oxadiazol-3-yl]-2,6-dimethylphenyl}ethyl)pyrrolidine-3-carboxylic acid

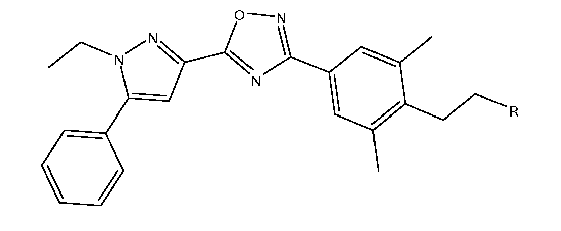

Examples 18 to 23 were obtained as hydrochloride salts from Preparation 42 and the corresponding amino acids following General Method 6.

| **EXAMPLE** | **R** | **tᵣ (Method C) (min)** | **LRMS (M+1)** | **¹H NMR** δ **ppm** |
|---|---|---|---|---|
| 18 | 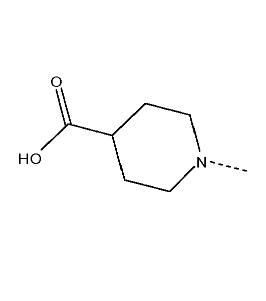 | 13.48 | 500 | ¹H NMR (400 MHz, DMSO*-d*₆) δ ppm 1.40 (t, *J*=7.23 Hz, 3 H) 1.74 - 2.01 (m, 2 H) 2.11 (d, *J*=15.24Hz,3H)2.46(s,6 H) 2.86 - 3.25 (m, 6 H) 3.69 (d, *J*=8.60 Hz, 1 H) 4.30 (q, *J*=7.03 Hz, 2 H) 7.19 (s, 1 H) 7.43 - 7.70 (m, 5 H) 7.78 (s, 2 H) 10.51 (br. s., 1 H) 12.57 (br. s., 1 H) |
| 19 | 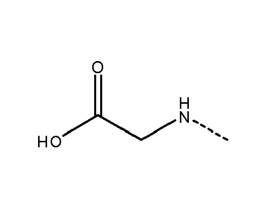 | 15.46 | 446 | ¹H NMR (400 MHz, DMSO*-d*₆) δ ppm 1.40 (t, *J*=7.23 Hz, 3 H) 2.43 (s, 6 H) 2.90 - 3.18 (m, 4 H) 3.93 (s, 2 H) 4.30 (q, *J*=7.03 Hz, 2 H) 7.18 (s, 1 H) 7.49 - 7.67 (m, 5 H) 7.77 (s, 2 H) |
| 20 | 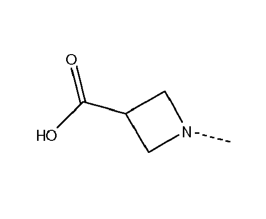 | 14.43 | 472 | ¹H NMR (400 MHz, DMSO*-d*₆) δ ppm 1.40 (t, *J*=7.23 Hz, 3 H) 2.45 (s, 6 H) 2.79 - 2.95 (m, 2 H) 3.59 - 3.73 (m, 1 H) 4.03 - 4.42 (m, 6 H) 7.18 (s, 1H) 7.49 - 7.68 (m, 5 H) 7.78 (s, 3 H) |
| 21 | 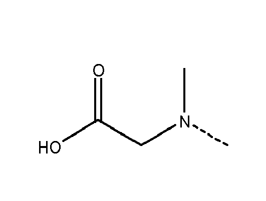 | 15.30 | 460 | 1H NMR (400 MHz, DMSO*-d*₆) δ ppm 1.40 (t, *J*=7.23 Hz, 3 H) 2.44 (s, 6 H) 2.97 (s, 3 H) 3.04 - 3.25 (m, 4 H) 4.16 (br. s., 2 H) 4.30 (q, *J*=7.42 Hz, 2 H) 7.18 (s, 1H) 7.43 - 7.68 (m, 5 H) 7.78 (s, 2 H) |
| 22 | 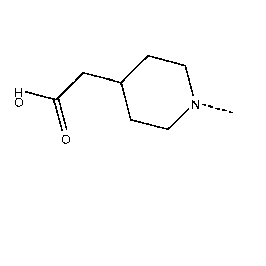 | 13.11 | 514 | ¹H NMR (400 MHz, DMSO*-d*₆) δ ppm 1.40 (t, *J*=7.23 Hz, 3 H) 1.93 (d, *J*=13.29 Hz, 3 H) 2.26 (d, *J*=4.69 Hz, 2 H) 2.45 (s, 6 H) 2.93 - 3.19 (m, 6 H) 3.57 (s, 2 H) 3.67 (d, *J*=12.90 Hz, 2 H) 4.30 (q, *J*=7.29 Hz, 2 H) 7.18 (s, 1 H) 7.47 - 7.70 (m, 5 H) 7.78 (s, 2 H) |
| 23 | 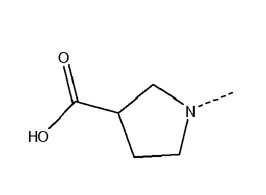 | 13.86 | 486 | ¹H NMR (400 MHz, DMSO*-d*₆) δ ppm 1.40 (t, *J*=7.23 Hz, 3 H) 2.46 (s, 6 H) 2.98 - 3.28 (m, 6 H) 3.57 (s, 4 H) 4.30 (q, *J*=7.16 Hz, 2 H) 7.18 (s, 1 H) 7.48 - 7.69 (m, 5 H) 7.78 (s, 2 H) |

### EXAMPLE 24

### 1-[2-(4-{5-[5-(2-chlorophenyl)-1-ethyl-1H-pyrazol-3-yl]-1,2,4-oxadiazol-3-yl}-2,6-dimethylphenyl)ethyl]piperidine-4-carboxylic acid

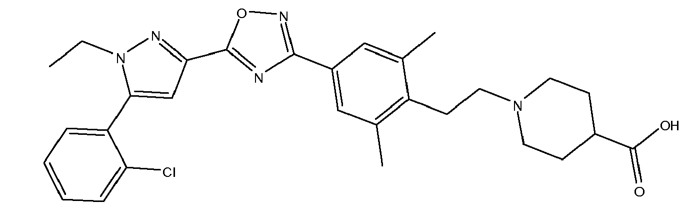

Obtained (30% yield) as hydrochloride salt from Preparation 44 and piperidine-4-carboxylic acid following General Method 6.
LRMS: m/z 535 (M+1)⁺
Retention time: 13.95 min (Method B)
¹H NMR (400 MHz, D MSO*-d*₆) δ ppm 1.31 (t, *J*=7.23 Hz, 3 H) 1.76 - 1.98 (m, 2 H) 2.09 (d, *J*=12.50 Hz, 3 H) 2.43 (s, 6 H) 2.92 - 3.22 (m, 6 H) 3.67 (d, *J*=11.33 Hz, 2 H) 4.04 (q, *J*=7.03 Hz, 2 H) 7.15 (s, 1 H) 7.48 - 7.64 (m, 3 H) 7.66 - 7.80 (m, 3 H).

### EXAMPLE 25

### 1-(2-{4-[5-(1-ethyl-5-isopropyl-1H-pyrazol-3-yl)-1,2,4-oxadiazol-3-yl]-2,6-dimethylphenyl}ethyl)piperidine-4-carboxylic acid

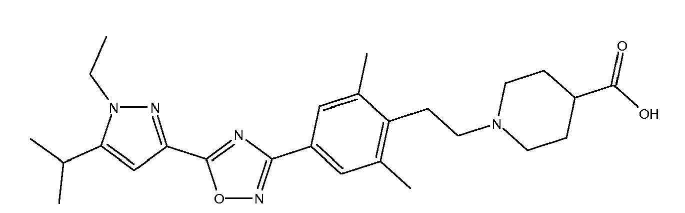

Obtained (64% yield) as hydrochloride salt from Preparation 47 and piperidine-4-carboxylic acid following General Method 6.
LRMS: m/z 466 (M+1)⁺
Retention time: 12.34 min (Method B)
¹H NMR (400 MHz, DMSO*-d*₆) δ ppm 1.27 (d, *J*=7.03 Hz, 6 H) 1.42 (t, *J*=7.03 Hz, 3 H) 1.82 - 2.00 (m, 2 H) 2.03 - 2.19 (m, 3 H) 2.45 (s, 6 H) 2.93 - 3.23 (m, 7 H) 3.69 (d, *J*=10.16 Hz, 2 H) 4.25 (q, *J*=7.29 Hz, 2 H) 6.88 (s, 1 H) 7.75 (s, 2 H).

### EXAMPLE 26

### 1-[2-(4-{5-[5-(3-chlorophenyl)-1-ethyl-1H-pyrazol-3-yl]-1,2,4-oxadiazol-3-yl}-2,6-dimethylphenyl)ethyl]piperidine-4-carboxylic acid

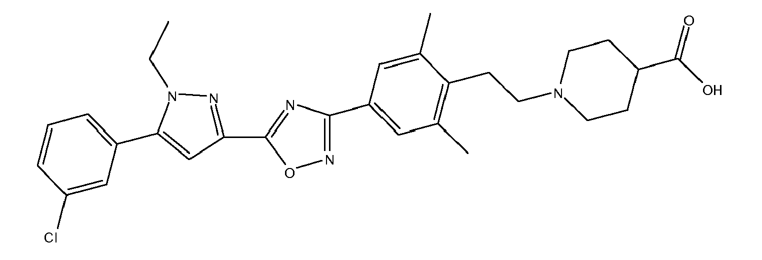

Obtained (23% yield) as hydrochloride salt from Preparation 51 and piperidine-4-carboxylic acid following General Method 6.
LRMS: m/z 535 (M+1)⁺
Retention time: 14.53 min (Method B)
¹H NMR (400 MHz, DMSO*-d*₆) δ ppm 1.40 (t, *J*=7.23 Hz, 3 H) 1.77 - 1.95 (m, 2 H) 2.02 - 2.21 (m, 3 H) 2.46 (s, 6 H) 2.99 - 3.20 (m, 6 H) 3.70 (d, *J*=11.33 Hz, 2 H) 4.30 (q, *J*=7.03 Hz, 2 H) 7.27 (s, 1 H) 7.55 - 7.67 (m, 3 H) 7.72 (s, 1 H) 7.77 (s, 2 H).

### EXAMPLE 27

### 1-(2-{3-methyl-4-[5-(1-methyl-5-phenyl-1H-pyrazol-3-yl)-1,2,4-oxadiazol-3-yl]phenyl}ethyl)piperidine-4-carboxylic acid

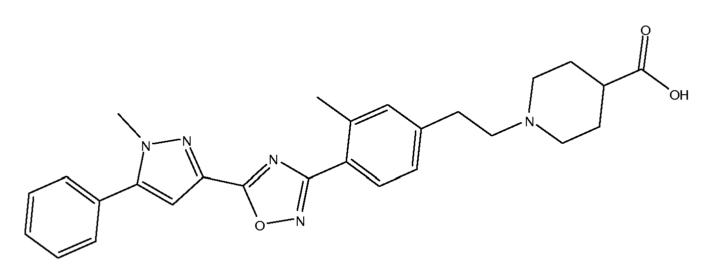

Obtained (22% yield) as hydrochloride salt from Preparation 55 and piperidine-4-carboxylic acid following General Method 6.
LRMS: m/z 472 (M+1)⁺
Retention time: 12.06 min (Method B)
¹H NMR (400 MHz, DMSO*-d*₆) δ ppm 1.48 - 1.67 (m, 2 H) 1.79 (d, *J*=13.29 Hz, 2 H) 2.02 (t, *J*=10.55 Hz, 2 H) 2.13 - 2.32 (m, 1 H) 2.61 (s, 3 H) 2.77 (d, *J*=10.94 Hz, 2 H) 3.50 (s, 2 H) 4.03 (s, 3 H) 7.23 (s, 1 H) 7.29 - 7.39 (m, 2 H) 7.48 - 7.61 (m, 3 H) 7.67 (d, *J*=6.64 Hz, 2 H) 7.98 (d, *J*=7.82 Hz, 1 H).

### EXAMPLE 28

### 1-[2-(4-{5-[1-ethyl-5-(3-fluorophenyl)-1H-pyrazol-3-yl]-1,2,4-oxadiazol-3-yl}-2,6-dimethylphenyl)ethyl]piperidine-4-carboxylic acid

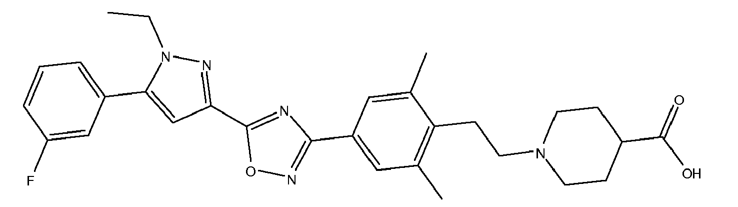

Obtained (40% yield) as hydrochloride salt from Preparation 59 and piperidine-4-carboxylic acid following General Method 6.
LRMS: m/z 518 (M+1)⁺
Retention time: 13.34 min (Method B)
¹H NMR (400 MHz, DMSO*-d*₆) δ ppm 1.40 (t, *J*=7.03 Hz, 3 H) 1.78 - 1.97 (m, 2 H) 2.11 (d, *J*=13.29 Hz, 3 H) 2.46 (s, 6 H) 2.98 - 3.20 (m, 6 H) 3.69 (d, *J*=10.55 Hz, 2 H) 4.23 - 4.42 (m, 2 H) 7.26 (s, 1 H) 7.35 - 7.55 (m, 3 H) 7.58 - 7.68 (m, 1 H) 7.77 (s, 2 H).

### EXAMPLE 29

### 1-(2-{2-methyl-4-[5-(1-methyl-5-phenyl-1H-pyrazol-3-yl)-1,2,4-oxadiazol-3-yl]phenyl}ethyl)piperidine-4-carboxylic acid

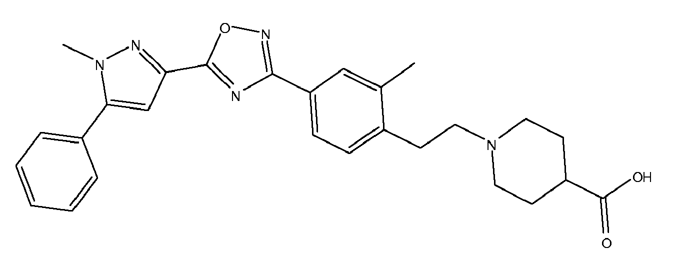

Obtained (1% yield) as hydrochloride salt from Preparation 63 and piperidine-4-carboxylic acid following General Method 6.
LRMS: m/z 472 (M+1)⁺
Retention time: 11.90 min (Method B)
¹H NMR (400 MHz, DMSO*-d*₆) δ ppm 1.70 - 1.96 (m, 2 H) 2.01 - 2.23 (m, 3 H) 2.45 (s, 3 H) 2.87 - 3.21 (m, 5 H) 3.66 (d, *J*=10.55 Hz, 2 H) 4.03 (s, 3 H) 7.21 (s, 1 H) 7.43 (d, *J*=7.82 Hz, 1 H) 7.50 - 7.61 (m, 3 H) 7.64 - 7.71 (m, 2 H) 7.83 - 8.01 (m, 2 H).

### EXAMPLE 30

### 1-{2-[4-[5-(1-methyl-5-phenyl-1H-pyrazol-3-yl)-1,2,4-oxadiazol-3-yl]-2-(trifluoromethyl)phenyl]ethyl}piperidine-4-carboxylic acid

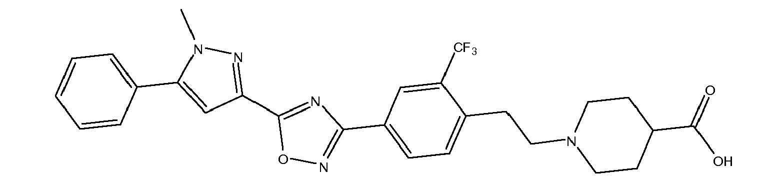

Obtained (1% yield) as hydrochloride salt from Preparation 67 and piperidine-4-carboxylic acid following General Method 6.
LRMS: m/z 526 (M+1)⁺
Retention time: 12.98 min (Method B)
¹H NMR (400 MHz, DMSO*-d*₆) δ ppm 1.69 - 1.93 (m, 2 H) 1.96 - 2.23 (m, 3 H) 2.87 - 3.17 (m, 5 H) 4.04 (s, 3 H) 7.29 (s, 1 H) 7.50 - 7.62 (m, 3 H) 7.68 (d, *J*=7.03 Hz, 2 H) 7.84 (d, *J*=7.03 Hz, 1 H) 8.23 - 8.47 (m, 2 H).

### EXAMPLE 31

### 1-(2-{4-[5-(5-tert-butyl-1-methyl-1H-pyrazol-3-yl)-1,2,4-oxadiazol-3-yl]-2,6-dimethylphenyl}ethyl)piperidine-4-carboxylic acid

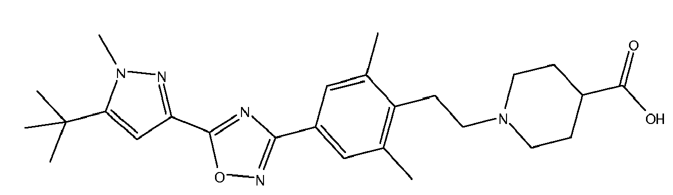

Obtained (45% yield) as hydrochloride salt from Preparation 70 and piperidine-4-carboxylic acid following General Method 6.
LRMS: m/z 466 (M+1)⁺
Retention time: 12.03 min (Method B)
¹H NMR (400 MHz, DMSO*-d*₆) δ ppm 1.41 (s, 9 H) 1.70 - 1.93 (m, 2 H) 1.94 - 2.07 (m, 1 H) 2.12 (d, *J*=12.90 Hz, 2 H) 2.44 (s, 6 H) 2.92 - 3.21 (m, 6 H) 3.70 (d, *J*=11.33 Hz, 2 H) 4.08 (s, 3 H) 6.82 (s, 1 H) 7.75 (s, 2 H).

### EXAMPLES 32 to 33

### 1-(2-{4-[5-(5-cyclohexyl-1-ethyl-1H-pyrazol-3-yl)-1,2,4-oxadiazol-3-yl]-2,6-dimethylphenyl}ethyl)piperidine-4-carboxylic acid

### 1-(2-{4-[5-(5-cyclohexyl-1-ethyl-1H-pyrazol-3-yl)-1,2,4-oxadiazol-3-yl]-2,6-dimethylphenyl}ethyl)azetidine-3-carboxylic acid

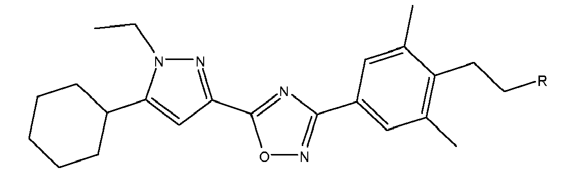

Examples 32 to 33 were obtained as hydrochloride salts from Preparation 74 and the corresponding amino acids following General Method 6.

| **EXAMPLE** | **R** | **tᵣ (Method C) (min)** | **LRMS (M+1)** | **1H NMR δ ppm** |
|---|---|---|---|---|
| 32 | 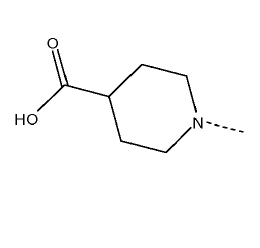 | 14.21 | 506 | ¹H NMR (400 MHz, DMSO*-d*₆) δ ppm 1.17 - 1.33 (m, 1H) 1.35 - 1.50 (m, 7 H) 1.63 - 1.97 (m, 7 H) 2.11 (d, *J*=13.68 Hz, 2 H) 2.45 (s, 6 H) 2.70 - 2.87 (m, 1 H) 2.93 - 3.21 (m, 6 H) 3.69 (d, *J*=10.16 Hz, 2H) 4.25 (q, *J*=7.29 Hz, 2 H) 6.83 (s, 1 H) 7.75 (s, 2 H) |
| 33 | 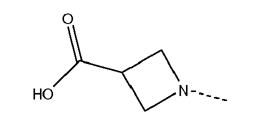 | 15.49 | 478 | ¹H NMR (400 MHz, DMSO*-d*₆) δ ppm 1.17 - 1.34 (m, 1 H) 1.34 - 1.55 (m, 7 H) 1.66 - 2.03 (m, 5 H) 2.44 (s, 6 H) 2.71-2.96 (m, 3 H) 3.16 - 3.43 (m, 3 H) 3.60 - 3.76 (m, 1 H) 4.07 - 4.39 (m, 5 H) 6.84 (s, 1 H) 7.75 (s, 2 H) |

### EXAMPLE 34

### 1-[2-(4-{5-[1-ethyl-5-(4-methoxyphenyl)-1H-pyrazol-3-yl]-1,2,4-oxadiazol-3-yl}-2,6-dimethylphenyl)ethyl]piperidine-4-carboxylic acid

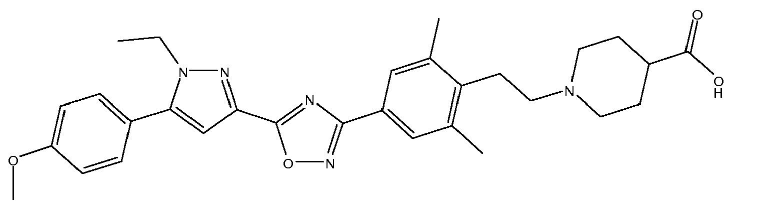

Obtained (54% yield) as hydrochloride salt from Preparation 77 and piperidine-4-carboxylic acid following General Method 6.
LRMS: m/z 530 (M+1)⁺
Retention time: 13.24 min (Method B)
¹H NMR (400 MHz, DMSO*-d*₆) δ ppm 1.39 (t, *J*=7.23 Hz, 3 H) 1.79 - 1.98 (m, 2 H) 2.05 - 2.19 (m, 3 H) 2.46 (s, 6 H) 2.91 - 3.24 (m, 6 H) 3.69 (d, *J*=10.55 Hz, 2 H) 3.84 (s, 3 H) 4.27 (q, *J*=7.03 Hz, 2 H) 7.01 - 7.23 (m, 3 H) 7.53 (d, *J*=8.99 Hz, 2 H) 7.77 (s, 2 H).

### EXAMPLES 35 to 36

### 1-(2-{4-[5-(1-ethyl-5-isobutyl-1H-pyrazol-3-yl)-1,2,4-oxadiazol-3-yl]-2,6-dimethylphenyl}ethyl)piperidine-4-carboxylic acid

### 1-(2-{4-[5-(1-ethyl-5-isobutyl-1H-pyrazol-3-yl)-1,2,4-oxadiazol-3-yl]-2,6-dimethylphenyl}ethyl)azetidine-3-carboxylic acid

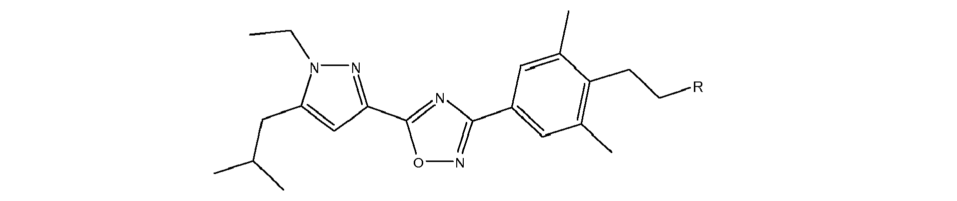

Examples 35 to 36 were obtained as hydrochloride salts from Preparation 81 and the corresponding amino acids following General Method 6.

| **EXAMPLE** | **R** | **tᵣ (Method C) (min)** | **LRMS (M+1)** | **1H NMR δ ppm** |
|---|---|---|---|---|
| 35 | 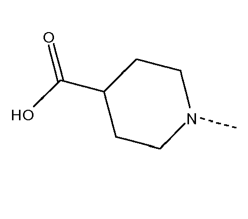 | 12.96 | 480 | ¹H NMR (400 MHz, DMSO*-d*₆) δ ppm 0.95 (s, 6H) 1.39 (s,3H) 1.76-2.04 (m, 3 H) 2.11 (d, *J*=14.46 Hz, 2 H) 2.45 (s, 6 H) 2.62 (d, *J*=3.91 Hz, 2H) 2.89 - 3.22 (m, 6H) 3.61 - 3.82 (m, 2 H) 4.23 (d, *J*=7.03 Hz, 2 H) |
| 36 | 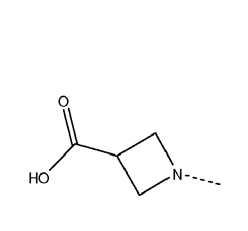 | 14.07 | 452 | ¹H NMR (400 MHz, DMSO*-d*₆) δ ppm 0.96 (d, *J*=6.64 Hz, 6 H) 1.39 (t, *J*=7.03 Hz, 3 H) 1.85 - 2.06 (m, 1 H) 2.44 (s, 6 H) 2.63 (d, *J*=7.03 Hz, 2 H) 2.79 - 2.97 (m, 2 H) 2.81 - 2.96 (m, 2 H) 3.15 - 3.30 (m, 2 H) 3.65 (sxt, 1 H) 4.04 - 4.34 (m, 6 H) 6.85 (s, 1 H) 7.75 (s, 2 H) |

### EXAMPLE 37

### 1-[2-(4-{5-[1-ethyl-5-(4-fluorophenyl)-1H-pyrazol-3-yl]-1,2,4-oxadiazol-3-yl}-2,6-dimethylphenyl)ethyl]piperidine-4-carboxylic acid

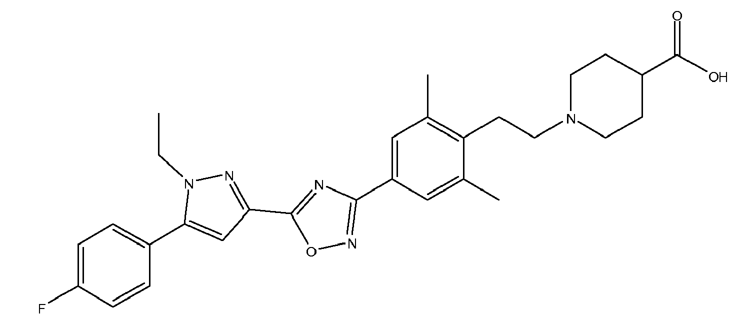

Obtained (49% yield) as hydrochloride salt from Preparation 85 and piperidine-4-carboxylic acid following General Method 6.
LRMS: m/z 518 (M+1)⁺
Retention time: 13.18 min (Method B)
¹H NMR (300 MHz, DMSO-*d*₆) δ ppm 1.39 (t, *J*=7.28 Hz, 3 H) 1.87 (t, 1 H) 2.12 (d, *J*=13.73 Hz, 3 H) 2.46 (s, 6 H) 2.92 - 3.20 (m, 8 H) 4.28 (q, *J*=7.14 Hz, 2 H) 7.19 (s, 1 H) 7.35 - 7.49 (m, 2 H) 7.62 - 7.71 (m, 2 H) 7.77 (s, 2 H).

### EXAMPLE 38

### 1-[2-(4-{5-[1-ethyl-5-(2-methoxyphenyl)-1H-pyrazol-3-yl]-1,2,4-oxadiazol-3-yl}-2,6-dimethylphenyl)ethyl]piperidine-4-carboxylic acid

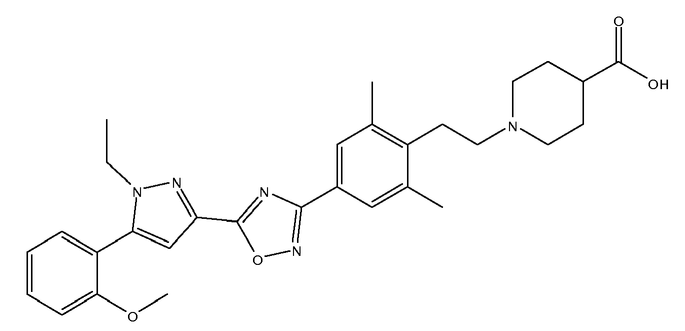

Obtained (97% yield) as hydrochloride salt from Preparation 89 and piperidine-4-carboxylic acid following General Method 6.
LRMS: m/z 530 (M+1)⁺
Retention time: 12.98 min (Method B)
¹H NMR (400 MHz, DMSO*-d*₆) δ ppm 1.34 (t, *J*=7.23 Hz, 3 H) 1.89 (q, *J*=13.16 Hz, 2 H) 2.11 (d, *J*=12.50 Hz, 2 H) 2.46 (s, 6 H) 3.69 (d, *J*=11.33 Hz, 2 H) 3.82 (s, 3 H) 4.04 (q, *J*=7.42 Hz, 2 H) 7.02 (s, 1 H) 7.12 (d, *J*=14.85 Hz, 1 H) 7.23 (d, *J*=8.21 Hz, 1 H) 7.37 (d, *J*=6.64 Hz, 1 H) 7.55 (t, *J*=8.21 Hz, 1 H) 7.77 (s, 2 H).

### EXAMPLE 39

### 1-(2-{4-[5-(1-ethyl-5-phenyl-1H-pyrazol-3-yl)-1,2,4-oxadiazol-3-yl]-2,5-dimethylphenyl}ethyl)piperidine-4-carboxylic acid

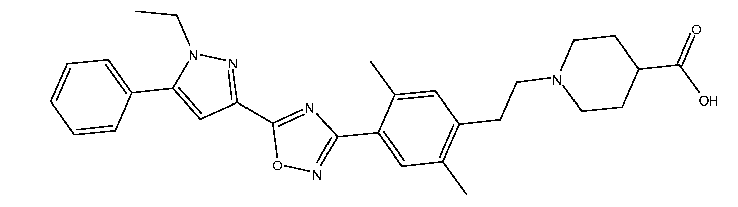

Obtained (43% yield) as hydrochloride salt from Preparation 94 and piperidine-4-carboxylic acid following General Method 6.
LRMS: m/z 500 (M+1)⁺
Retention time: 13.12 min (Method B)
¹H NMR (400 MHz, DMSO*-d*₆) δ ppm 1.40 (t, *J*=7.23 Hz, 3 H) 1.85 (t, *J*=15.04 Hz, 2 H) 1.94 - 2.18 (m, 3 H) 2.39 (s, 3 H) 2.57 (s, 3 H) 2.93 - 3.15 (m, 4 H) 3.17 - 3.29 (m, 1 H) 3.66 (d, *J*=11.72 Hz, 2 H) 4.30 (q, *J*=7.29 Hz, 2 H) 7.18 (s, 1 H) 7.26 - 7.31 (m, 1 H) 7.51 - 7.70 (m, 5 H) 7.86 (s, 1 H).

### EXAMPLE 40

### 1-[2-(4-{5-[1-ethyl-5-(2-fluorophenyl)-1H-pyrazol-3-yl]-1,2,4-oxadiazol-3-yl}-2,6-dimethylphenyl)ethyl]piperidine-4-carboxylic acid

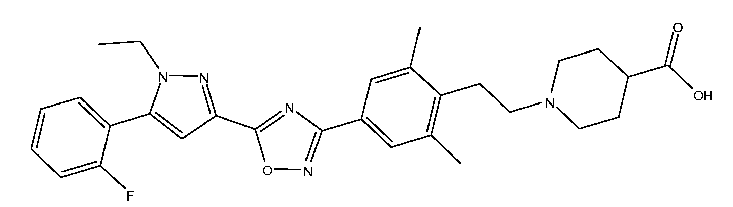

Obtained (71% yield) as hydrochloride salt from Preparation 98 and piperidine-4-carboxylic acid following General Method 6.
LRMS: m/z 518 (M+1)⁺
Retention time: 13.01 min (Method B)
¹H NMR (400 MHz, DMSO*-d*₆) δ ppm 1.37 (t, *J*=7.23 Hz, 3 H) 1.81 - 1.97 (m, 2 H) 2.11 (d, *J*=13.68 Hz, 3 H) 2.46 (s, 6 H) 2.94 - 3.23 (m, 6 H) 3.69 (d, *J*=11.72 Hz, 2 H) 4.16 (q, *J*=7.42 Hz, 2 H) 7.21 (s, 1 H) 7.35 - 7.52 (m, 2 H) 7.54 - 7.71 (m, 2 H) 7.77 (s, 2 H).

### EXAMPLE 41

### 1-(2-{4-[5-(1-ethyl-4-methyl-5-phenyl-1H-pyrazol-3-yl)-1,2,4-oxadiazol-3-yl]-2,6-dimethylphenyl}ethyl)piperidine-4-carboxylic acid

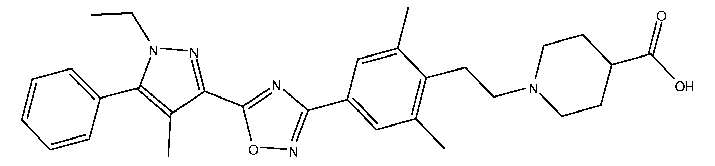

Obtained (45% yield) as hydrochloride salt from Preparation 102 and piperidine-4-carboxylic acid following General Method 6.
LRMS: m/z 514 (M+1)⁺
Retention time: 13.92 min (Method B)
¹H NMR (400 MHz, DMSO*-d*₆) δ ppm 1.31 (t, *J*=7.03 Hz, 3 H) 1.81 - 1.99 (m, 2 H) 2.11 (d, *J*=12.11 Hz, 3 H) 2.30 (s, 3 H) 2.46 (s, 6 H) 2.92 - 3.23 (m, 6 H) 3.70 (d, *J*=10.16 Hz, 2 H) 4.05 - 4.23 (m, 2 H) 7.35 - 7.70 (m, 5 H) 7.78 (s, 2 H).

### EXAMPLE 42

### 1-{2-[4-(5-{1-ethyl-5-[2-(trifluoromethyl)phenyl]-1H-pyrazol-3-yl}-1,2,4-oxadiazol-3-yl)-2,6-dimethylphenyl]ethyl}piperidine-4-carboxylic acid

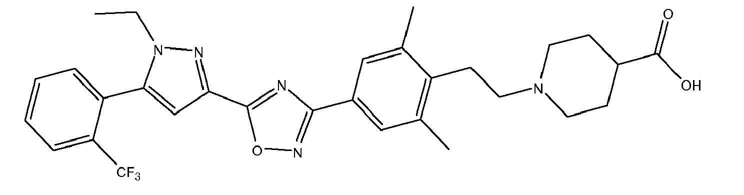

Obtained (28% yield) as hydrochloride salt from Preparation 106 and piperidine-4-carboxylic acid following General Method 6.
LRMS: m/z 568 (M+1)⁺
Retention time: 13.80 min (Method B)
¹H NMR (400 MHz, DMSO*-d*₆) δ ppm 1.31 (t, *J*=7.23 Hz, 3 H) 1.70 - 2.00 (m, 2 H) 2.11 (d, *J*=10.94 Hz, 3 H) 2.46 (s, 6 H) 2.88 - 3.22 (m, 6 H) 3.69 (d, *J*=10.94 Hz, 2 H) 4.01 (q, *J*=7.29 Hz, 2 H) 7.14 (s, 1 H) 7.58 - 8.17 (m, 6 H).

### EXAMPLE 43

### 1-[2-(4-{5-[1-ethyl-5-(2-methylphenyl)-1H-pyrazol-3-yl]-1,2,4-oxadiazol-3-yl}-2,6-dimethylphenyl)ethyl]piperidine-4-carboxylic acid

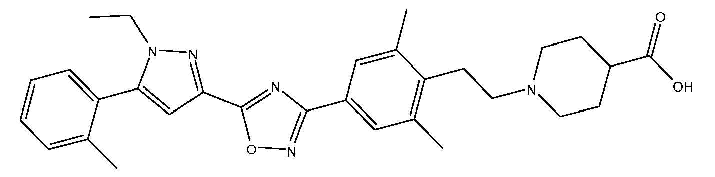

Obtained (33% yield) as hydrochloride salt from Preparation 112 following General Method 3.
LRMS: m/z 514 (M+1)⁺
Retention time: 14.06 min (Method B)
¹H NMR (400 MHz, DMSO*-d*₆) δ ppm 1.30 (t, *J*=7.23 Hz, 3 H) 1.75 - 1.98 (m, 2 H) 2.01 - 2.16 (m, 3 H) 2.46 (s, 6 H) 2.92 - 3.24 (m, 6 H) 3.70 (d, *J*=12.90 Hz, 2 H) 4.03 (q, *J*=7.03 Hz, 2 H) 7.11 (s, 1 H) 7.32 - 7.51 (m, 4 H) 7.78 (s, 2 H).

### EXAMPLE 44

### 3-{4-[5-(1-ethyl-5-phenyl-1H-pyrazol-3-yl)-1,2,4-oxadiazol-3-yl]-2,6-dimethylphenyl}propanoic acid

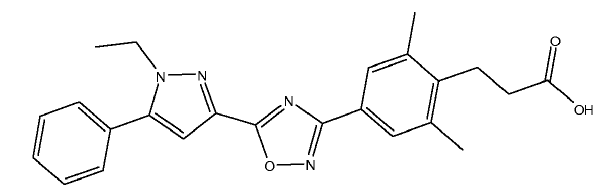

Obtained (49% yield) from Preparation 116 following General Method 4.
LRMS: m/z 417 (M+1)⁺
Retention time: 18.68 min (Method B)
¹H NMR (400 MHz, DMSO*-d*₆) δ ppm 1.40 (t, *J*=7.26 Hz, 3 H) 2.31 - 2.45 (m, 8 H) 2.84 - 3.00 (m, 2 H) 4.30 (q, *J*=7.26 Hz, 2 H) 7.19 (s, 1 H) 7.47 - 7.65 (m, 5 H) 7.74 (s, 2 H) 12.30 (s, 1H).

### EXAMPLE 45

### N-(2-{2,6-dimethyl-4-[5-(1-methyl-5-phenyl-1H-pyrazol-3-yl)-1,3,4-oxadiazol-2-yl]phenyl}ethyl)glycine

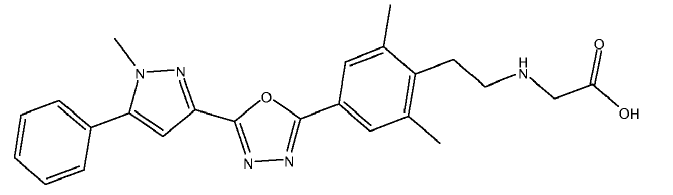

Obtained (2% yield) as hydrochloride salt from Preparation 124 and 2-aminoacetic acid following General Method 6.
LRMS: m/z 432 (M+1)⁺
Retention time: 12.74 min (Method B)
¹H NMR (400 MHz, DMSO*-d*₆) δ ppm 2.45 (s, 6 H) 2.91 - 3.19 (m, 4 H) 3.89 - 4.06 (m, 5 H) 7.17 (s, 1 H) 7.47 - 7.62 (m, 3 H) 7.66 (d, *J*=7.03 Hz, 2 H) 7.77 (s, 2 H) 9.43 (s, 2 H).

### EXAMPLE 46

### 1-(2-{4-[5-(5-benzyl-1-ethyl-1H-pyrazol-3-yl)-1,2,4-oxadiazol-3-yl]-2,6-dimethylphenyl}ethyl)piperidine-4-carboxylic acid

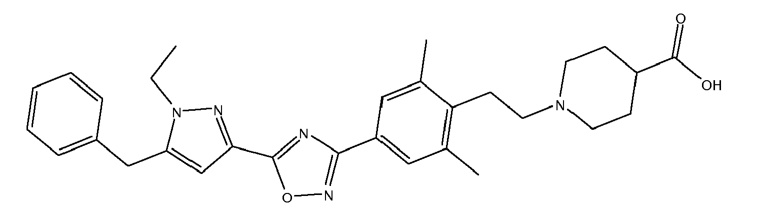

Obtained (33% yield) as hydrochloride salt from Preparation 128 and piperidine-4-carboxylic acid following General Method 6.
LRMS: m/z 514 (M+1)⁺
Retention time: 13.37 min (Method B)
¹H NMR (400 MHz, DMSO*-d*₆) δ ppm 1.27 (t, *J*=7.23 Hz, 3 H) 1.71 - 1.98 (m, 2 H) 2.11 (d, *J*=14.85 Hz, 3 H) 2.44 (s, 6 H) 2.87 - 3.21 (m, 6 H) 3.69 (d, *J*=10.16 Hz, 2 H) 4.06 - 4.36 (m, 5 H) 6.74 (s, 1 H) 7.18 - 7.50 (m, 5 H) 7.74 (s, 2 H).

### EXAMPLE 47

### 1-[2-(4-{5-[5-(2,2-dimethylpropyl)-1-ethyl-1H-pyrazol-3-yl]-1,2,4-oxadiazol-3-yl}-2,6-dimethylphenyl)ethyl]piperidine-4-carboxylic acid

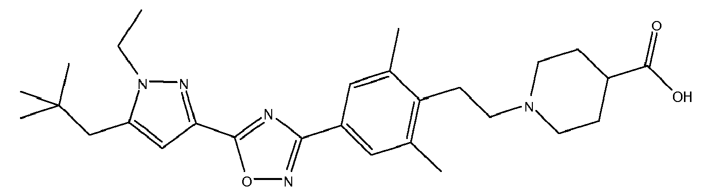

Obtained (42% yield) as hydrochloride salt from Preparation 132 and piperidine-4-carboxylic acid following General Method 6.
LRMS: m/z 494 (M+1)⁺
Retention time: 13.96 min (Method B)
¹H NMR (400 MHz, DMSO*-d*₆) δ ppm 0.96 (s, 9 H) 1.41 (t, *J*=7.23 Hz, 3 H) 1.76 -1.97 (m, 2 H) 2.11 (d, *J*=13.68 Hz, 3 H) 2.45 (s, 6 H) 2.69 (s, 2 H) 2.89 - 3.21 (m, 6 H) 3.57 (s, 3 H) 3.69 (d, *J*=9.38 Hz, 2 H) 4.26 (q, *J*=7.16 Hz, 2 H) 6.82 (s, 1 H) 7.76 (s, 2 H).

### EXAMPLE 48

### 1-{2-[4-[5-(1-ethyl-5-cyclohexyl-1H-pyrazol-3-yl)-1,2,4-oxadiazol-3-yl]-2-(trifluoromethyl)phenyl]ethyl}piperidine-4-carboxylic acid

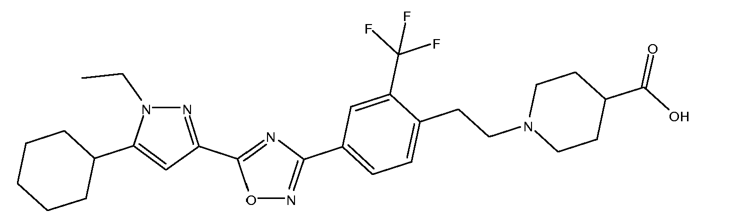

Obtained (17% yield) as hydrochloride salt from Preparation 136 following General Method 3.
LRMS: m/z 546 (M+1)⁺
Retention time: 15.10 min (Method B)
¹H NMR (400 MHz, DMSO-*d*₆) δ ppm 1.10 (d, *J*=5.47 Hz, 3 H) 1.26 (br. s., 2 H) 1.42 (br. s., 8 H) 1.53 - 1.66 (m, 2 H) 1.68 - 1.96 (m, 10 H) 2.71 - 3.08 (m, 7 H) 4.26 (br. s., 2 H) 6.89 (br. s., 1 H) 7.77 (br. s., 1 H) 8.29 (br. s., 2 H).

### EXAMPLE 49

### 1-(2-{4-[5-(1-ethyl-5-isobutyl-4-methyl-1H-pyrazol-3-yl)-1,2,4-oxadiazol-3-yl]-2,6-dimethylphenyl}ethyl)piperidine-4-carboxylic acid

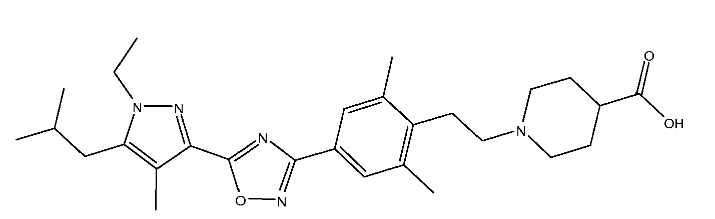

Obtained (23% yield) as hydrochloride salt from Preparation 143 following General Method 3.
LRMS: m/z 494 (M+1)⁺
Retention time: 13.99 min (Method B)
¹H NMR (400 MHz, DMSO*-d*₆) δ ppm 0.93 (d, *J*=6.64 Hz, 6 H) 1.40 (t, *J*=7.23 Hz, 3 H) 1.71 - 1.97 (m, 3 H) 2.11 (d, *J*=15.63 Hz, 3 H) 2.32 (s, 3 H) 2.45 (s, 6 H) 2.60 (d, *J*=7.42 Hz, 2 H) 2.88 - 3.22 (m, 6 H) 3.69 (d, *J*=11.33 Hz, 2 H) 4.19 (q, *J*=7.29 Hz, 2 H) 7.76 (s, 2 H).

### EXAMPLE 50

### 1-(2-{4-[5-(1-ethyl-5-isobutyl-1H-pyrazol-3-yl)-4H-1,2,4-triazol-3-yl]-2,6-dimethylphenyl}ethyl)piperidine-4-carboxylic acid

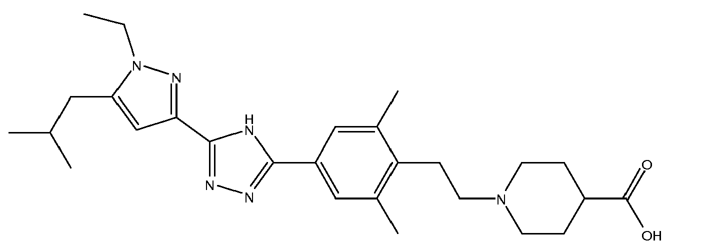

Obtained (11% yield) as hydrochloride salt from Preparation 148 following General Method 3.
LRMS: m/z 479 (M+1)⁺
Retention time: 10.77 min (Method B)
¹H NMR (400 MHz, DMSO*-d*₆) δ ppm 0.96 (d, *J*=6.64 Hz, 6 H) 1.37 (t, *J*=7.23 Hz, 3 H) 1.81 - 1.99 (m, 2 H) 2.06 - 2.14 (m, 2 H) 2.42 (s, 6 H) 2.58 (d, *J*=7.03 Hz, 2 H) 2.95 - 3.13 (m, 6 H) 3.68 (d, *J*=13.68 Hz, 2 H) 4.16 (q, *J*=7.03 Hz, 2 H) 6.57 (s, 1 H) 7.70 - 7.76 (m, 2 H).

### EXAMPLE 51

### 1-[2-(4-{5-[1-(cyclopropylmethyl)-5-phenyl-1H-pyrazol-3-yl]-1,2,4-oxadiazol-3-yl}-2,6-dimethylphenyl)ethyl]piperidine-4-carboxylic acid

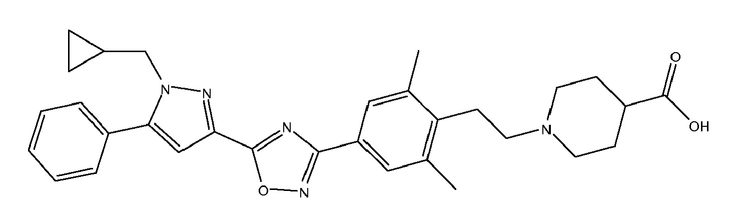

Obtained (33% yield) as hydrochloride salt from Preparation 151 following General Method 3.
LRMS: m/z 526 (M+1)⁺
Retention time: 13.92 min (Method B)
¹H NMR (400 MHz, DMSO*-d*₆) δ ppm 0.20 (d, *J*=4.69 Hz, 2 H) 0.46 (d, *J*=7.82 Hz, 2 H) 1.07 - 1.26 (m, 1 H) 1.76 - 1.98 (m, 2 H) 2.00 - 2.21 (m, 3 H) 2.39 - 2.53 (m, 6 H) 2.46 (s, 6 H) 2.91 - 3.25 (m, 6 H) 3.57 - 3.58 (m, 1 H) 3.70 (d, *J*=8.99 Hz, 2 H) 4.18 (d, *J*=7.03 Hz, 2 H) 7.19 (s, 1 H) 7.47 - 7.71 (m, 5 H) 7.78 (s, 2 H).

### EXAMPLE 52

### 1-(2-{4-[5-(1-ethyl-5-isopropoxy-1H-pyrazol-3-yl)-1,2,4-oxadiazol-3-yl]-2,6-dimethylphenyl}ethyl)piperidine-4-carboxylic acid

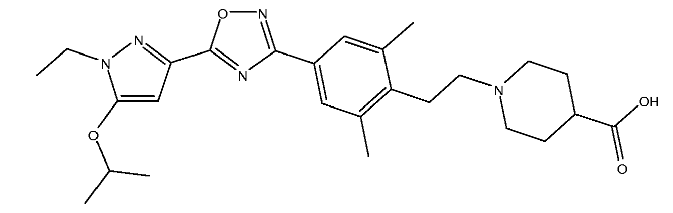

Obtained (45% yield) as hydrochloride salt from Preparation 156 and piperidine-4-carboxylic acid following General Method 6.
LRMS: m/z 482 (M+1)⁺
Retention time: 12.27 min (Method B)
¹H NMR (400 MHz, DMSO*-d*₆) δ ppm 1.34 (t, *J*=7.03 Hz, 3 H) 1.36 (d, *J*=6.25 Hz, 6 H) 1.82 - 2.00 (m, 2 H) 2.05 - 2.17 (m, 3 H) 2.45 (s, 6 H) 2.94 - 3.23 (m, 6 H) 3.48 - 3.74 (m, 2 H) 4.06 (q, *J*=7.03 Hz, 2 H) 4.61 - 4.73 (m, 1 H) 6.53 (s, 1 H) 7.59 - 7.79 (m, 2 H) 10.72 (s, 1 H) 12.59 (s, 1 H).

### EXAMPLE 53

### 1-(2-{4-[5-(1-ethyl-5-isobutyl-1H-pyrazol-3-yl)-1,3,4-thiadiazol-2-yl]-2,6-dimethylphenyl}ethyl)piperidine-4-carboxylic acid

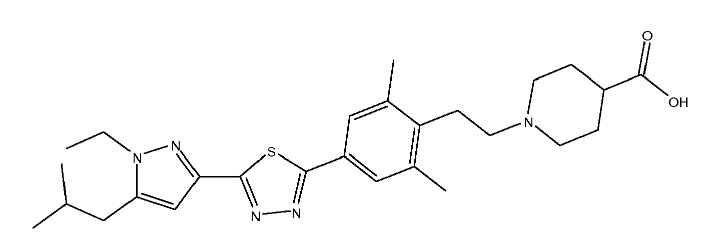

Obtained (16% yield) as hydrochloride salt from Preparation 162 and piperidine-4-carboxylic acid following General Method 6.
LRMS: m/z 496 (M+1)⁺
Retention time: 12.44 min (Method B)
¹H NMR (400 MHz, DMSO*-d*₆) δ ppm 0.96 (d, *J*=6.25 Hz, 6 H) 1.37 (t, *J*=7.23 Hz, 3 H) 1.69 - 1.90 (m, 2 H) 1.91 - 2.04 (m, 1 H) 2.11 (d, *J*=12.50 Hz, 3 H) 2.43 (s, 6 H) 2.60 (d, *J*=7.42 Hz, 2 H) 2.89 - 3.22 (m, 6 H) 3.62 - 3.79 (m, 2 H) 4.16 (q, *J*=7.42 Hz, 2 H) 6.71 (s, 1 H) 7.70 (s, 2 H).

### EXAMPLE 54

### 1-(4-(3-(5-cyclohexyl-1-ethyl-1H-pyrazol-3-yl)-1,2,4-oxadiazol-5-yl)-2,6-dimethylphenethyl)piperidine-4-carboxylic acid

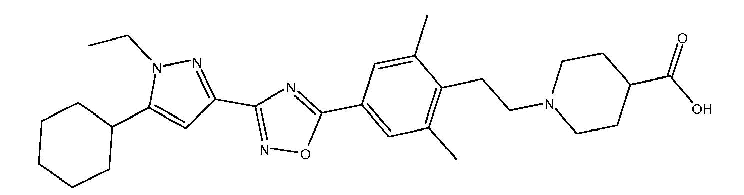

Obtained (16% yield) as hydrochloride salt from Preparation 171 and piperidine-4-carboxylic acid following General Method 6.
LRMS: m/z 506 (M+1)⁺
Retention time: 12.85 min (Method B)
1H NMR (400 MHz, DMSO*-d*₆) δ ppm 1.14 - 1.33 (m, 1 H) 1.39 (t, *J*=7.03 Hz, 6 H) 1.61 - 2.23 (m, 10 H) 2.47 (s, 6 H) 2.67 - 2.90 (m, 2 H) 2.92 - 3.24 (m, 6 H) 3.70 (d, *J*=11.72 Hz, 2 H) 4.20 (q, *J*=7.03 Hz, 2 H) 6.62 (s, 1 H) 7.86 (s, 2 H).

### EXAMPLE 55

### 1-(4-(5-(1-ethyl-4-phenyl-1H-pyrazol-3-yl)-1,2,4-oxadiazol-3-yl)-2,6-dimethylphenethyl)piperidine-4-carboxylic acid

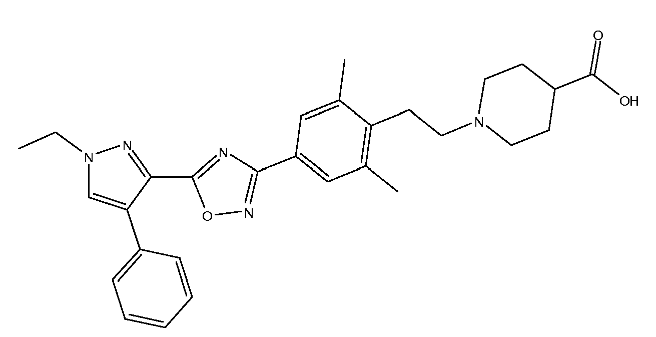

Obtained (13% yield) from Preparation 177 following General Method 3.
LRMS: m/z 500 (M+1)⁺
Retention time: 12.40 min (Method B)
¹H NMR (400 MHz, DMSO*-d*₆) δ ppm 1.50 (t, *J*=7.23 Hz, 3 H) 1.52 - 1.63 (m, 2 H) 1.76 - 1.86 (m, 2 H) 2.03 - 2.14 (m, 2 H) 2.14 - 2.26 (m, 1 H) 2.37 (s, 6 H) 2.44 - 2.52 (m, 2 H) 2.76 - 2.85 (m, 2 H) 2.86 - 2.95 (m, 2 H) 4.33 (q, *J*=7.16 Hz, 2 H) 7.38 (d, *J*=7.42 Hz, 1 H) 7.40 - 7.49 (m, 2 H) 7.55 - 7.66 (m, 4 H) 8.29 (s, 1 H).

### EXAMPLE 56

### 1-(4-(5-(1-ethyl-4-phenyl-1H-pyrazol-3-yl)-1,2,4-oxadiazol-3-yl)-2-(trifluoromethyl)phenethyl)piperidine-4-carboxylic acid

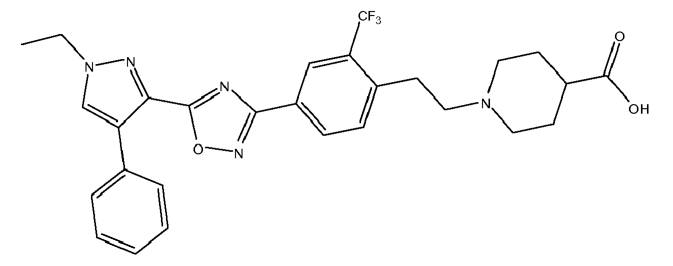

Obtained (34% yield) from Preparation 178 following General Method 3.
LRMS: m/z 540 (M+1)⁺
Retention time: 12.95 min (Method B)
¹H NMR (400 MHz, DMSO*-d*₆) δ ppm 1.51 (t, *J*=7.42 Hz, 3 H) 1.52 - 1.63 (m, 2 H) 1.75 - 1.85 (m, 2 H) 2.09 (m, 2 H) 2.14 - 2.26 (m, 1 H) 2.52 - 2.62 (m, 2 H) 2.79 - 2.91 (m, 2 H) 2.91 - 3.03 (m, 2 H) 4.34 (q, 2 H) 7.41 (s, 3 H) 7.59 - 7.65 (m, *J*=7.03 Hz, 2 H) 7.73 (d, *J*=8.21 Hz, 1 H) 8.16 - 8.21 (m, 2 H) 8.30 (s, 1 H).

### EXAMPLE 57

### 1-(4-(5-(1-ethyl-5-isobutyl-1H-pyrazol-3-yl)-1,2,4-oxadiazol-3-yl)-2-(trifluoromethyl)phenethyl)piperidine-4-carboxylic acid

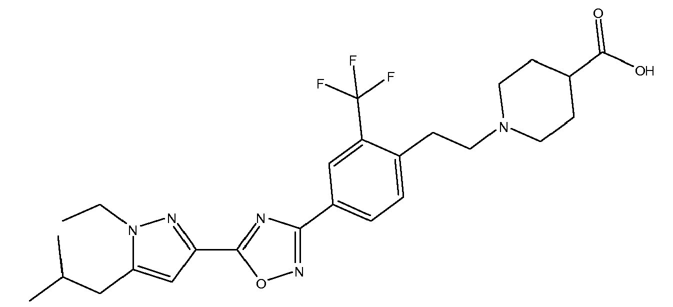

Obtained (24% yield) as hydrochloride salt from Preparation 179 following General Method 3.
LRMS: m/z 520 (M+1)⁺
Retention time: 7.38 min (Method D)
¹H NMR (400 MHz, DMSO*-d*₆) δ ppm 0.96 (d, *J*=6.64 Hz, 6 H) 1.40 (t, *J*=7.23 Hz, 3 H) 1.98 (td, *J*=13.38, 6.45 Hz, 4 H) 2.63 (d, *J*=7.42 Hz, 3 H) 4.24 (q, *J*=7.16 Hz, 2 H) 6.90 (s, 1 H) 7.81 (d, *J*=6.25 Hz, 1 H) 8.26 - 8.54 (m, 2 H).

### EXAMPLES 58 to 59

### 1-(4-(5-(1-ethyl-5-isobutyl-1H-pyrazol-3-yl)-1,2,4-oxadiazol-3-yl)-2-(trifluoromethyl)phenethyl)azetidine-3-carboxylic acid,

### 2-((4-(5-(1-ethyl-5-isobutyl-1H-pyrazol-3-yl)-1,2,4-oxadiazol-3-yl)-2-(trifluoromethyl)phenethyl)(methyl)amino)acetic acid

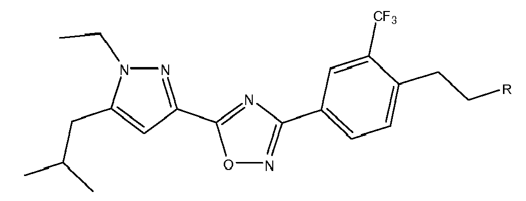

Examples 58 to 59 were obtained as hydrochloride salts from Preparation 181 and the corresponding amino acids following General Method 6.

| **EXAMPLE** | **R** | **tᵣ (Method C) (min)** | **LRMS(M+1)** | **1H NMR** δ **ppm** |
|---|---|---|---|---|
| 58 | 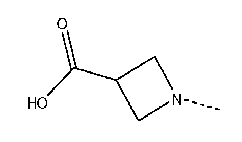 | 13.72 | 492 | ¹H NMR (400 MHz, DMSO*-d*₆) δ ppm 0.96 (d, *J*=6.64 Hz, 6 H) 1.40 (t, *J*=7.23 Hz, 3 H) 1.86 - 2.06 (m, *J*=13.41, 6.71, 6.71, 6.71, 6.71 Hz, 1 H) 2.63 (d, *J*=7.42 Hz, 2 H) 3.10 (d, *J*=8.21 Hz, 2 H) 3.46 (d, *J*=6.64 Hz, 2 H) 3.64 (quin, *J*=8.40 Hz, 1 H) 4.08 - 4.44 (m, 6 H) 6.90 (s, 1 H) 7.87 (d, *J*=7.82 Hz, 1 H) 8.32 (s, 1 H) 8.36 (d, *J*=8.21 Hz, 1 H) |
| 59 | 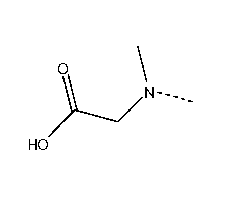 | 13.75 | 480 | ¹H NMR (400 MHz, DMSO*-d*₆) δ ppm 0.96 (d, *J*=6.64 Hz, 6 H) 1.40 (t, *J*=7.23 Hz, 3 H) 1.85 - 2.09 (m, *J*=13.43, 6.80, 6.80, 6.80, 6.80 Hz, 1 H) 2.63 (d, 2 H) 2.95 (s, 3 H) 3.35 - 3.51 (m, 4 H) 4.19 (s, 2 H) 4.24 (q, *J*=7.03 Hz, 2 H) 6.90 (s, 1H) 7.83 (d, *J*=8.21 Hz, 1 H) 8.32 (s, 1 H) 8.37 (d, *J*=8.21 Hz, 1 H) |

### EXAMPLE 60

### 3-(4-(5-(5-(diethylamino)-1-ethyl-1H-pyrazol-3-yl)-1,2,4-oxadiazol-3-yl)-2,6-dimethylphenyl)propanoic acid

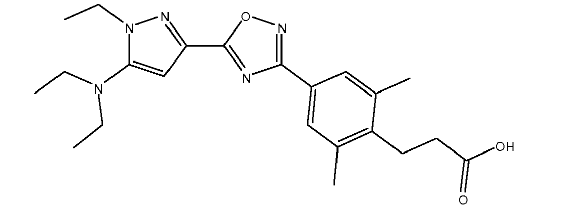

Obtained (75% yield) from Preparation 185 following General Method 4.
LRMS: m/z 412 (M+1)⁺
Retention time: 9.33 min (Method D)
¹H NMR (400 MHz, DMSO*-d*₆) δ ppm 0.98 (t, *J*=7.03 Hz, 6 H) 1.40 (t, *J*=7.03 Hz, 3 H) 2.35 - 2.42 (m, 8 H) 2.88 - 2.95 (m, 2 H) 2.99 (q, *J*=7.03 Hz, 4 H) 4.15 (q, *J*=7.03 Hz, 2 H) 6.82 (s, 1 H) 7.71 (s, 2 H) 12.23 (s, 1 H).

### EXAMPLE 61

### 1-(4-(5-(1-ethyl-5-propoxy-1H-pyrazol-3-yl)-1,2,4-oxadiazol-3-yl)-2,6-dimethylphenethyl)piperidine-4-carboxylic acid

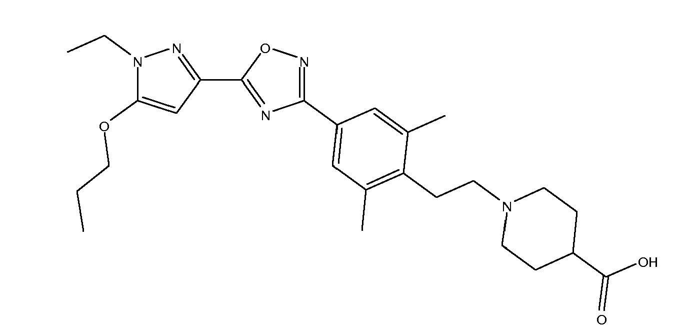

Obtained (14% yield) as hydrochloride salt from Preparation 188 following General Method 3.
LRMS: m/z 482 (M+1)⁺
Retention time: 11.65 min (Method B)
¹H NMR (400 MHz, DMSO*-d*₆) δ ppm 1.00 (t, *J*=7.42 Hz, 3 H) 1.35 (t, *J*=7.23 Hz, 3 H) 1.78 (d, *J*=7.42 Hz, 2 H) 1.83 - 1.99 (m, 2 H) 2.11 (d, *J*=12.90 Hz, 3 H) 2.35 - 2.49 (m, 6 H) 3.69 (d, *J*=11.33 Hz, 2 H) 4.09 (q, *J*=7.03 Hz, 2 H) 4.19 (t, *J*=6.45 Hz, 2 H) 6.51 (s, 1 H) 7.74 (s, 2 H).

### EXAMPLE 62

### 1-(4-(5-(1-ethyl-5-isopropoxy-1H-pyrazol-3-yl)-1,2,4-oxadiazol-3-yl)-2-(trifluoromethyl)phenethyl)piperidine-4-carboxylic acid

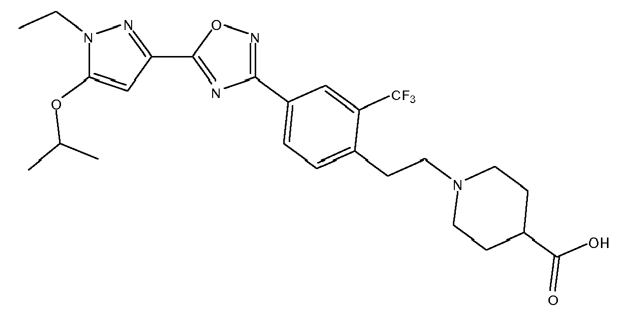

Obtained (10% yield) as hydrochloride salt from Preparation 189 following General Method 3.
LRMS: m/z 522 (M+1)⁺
Retention time: 11.98 min (Method B)

### PHARMACOLOGICAL ACTIVITY

### 35S-GTP binding assay:

The effect of the compounds was measured using a 35S-GTP binding assay. Briefly, membranes were incubated in a buffer containing 20 mM HEPES pH 7.4, 100 mM NaCl, 10 mM MgCl2, 10µM GDP, 50µg/ml saponin and 0.2% fatty acid-free BSA at various concentrations (0.1nM-10µM) and 0.1nM 35S-GTPyS. 10µM S1P was used as 100% maximum efficacy. The assay was incubated for 90 min at room temperature with gentle mixing, and terminated by filtrating the reaction mixture through GF/C filter plates using the Manifold Filtration System. The filters were immediately washed with sodium phosphate pH 7.4 buffers. After drying the filter plate's scintillant liquid were added to each well and 35S-GTPyS binding was measured on a Trilux Scintillation Counter. The results are shown in Table 1.

**Table 1**

| **EXAMPLES** | **EC₅₀ (nM)** |
|---|---|
| 2 | 15 |
| 7 | 2.0 |
| 14 | 16 |
| 19 | 0.9 |
| 25 | 18 |
| 40 | 4.7 |
| 42 | 6.0 |
| 53 | 3.1 |
| 60 | 20 |

Compounds of the present invention have high affinities for sphingosine-1-phosphate receptors. Thus the EC₅₀ of the preferred compounds of the invention is lower than 100 nM, preferably less than 50 nM, more preferably less than 20 nM. The most preferred compounds of the invention have an EC₅₀ lower than 10 nM.

The pyrazole derivatives of the invention may also be combined with other active compounds in the treatment of diseases known to be susceptible to improvement by treatment with a sphingosine-1-phosphate receptor agonist (S1P1).

The combinations of the invention can optionally comprise one or more additional active substances which are known to be useful in the treatment of autoimmune diseases, chronic immune and inflammatory diseases, transplant rejection, malignant neoplastic diseases, angiogenic-related disorders, pain, neurological diseases,viral and infectious diseases, such as (a) Interferons comprising Interferon beta 1a such as Avonex from Biogen Idec, CinnoVex from CinnaGen and Rebif from EMD Serono, and Interferon beta 1b such as Betaferon from Schering and Betaseron from Berlex, (b), immunomodulators such as glatiramer acetate, (c) inhibitors of DNA synthesis and repair, such as Mitoxantrone, (d) anti-alpha 4 integrin antibodies, such as Natalizumab (Tysabri), (e) alpha 4 integrin antagonists such as R-1295 , TBC-4746, CDP-323, ELND-002, Firategrast and TMC-2003, (f), dyhydrofolate reductase inhibitors, such as Methotrexate or CH-1504, (g) glucocorticoids such as prednisone or methylprednisolone, (h), DHODH inhibitors such as Teriflunomide and leflunomide (i) fumaric acid esters, such as *BG-12*, (j) immunomodulators such as Laquinimod, (k) anti-CD20 monoclonal antibodies such as Rituximab, Ocrelizumab Ofatumumab or TRU-015, (l) anti-CD52 such as alemtuzumab, (m) anti-CD25 such as daclizumab, (n) anti-CD88, such as eculizumab or pexilizumab, (o) calcineurin inhibitors such as cyclosporine A or tacrolimus, (p) IMPDH inhibitors, such as mycophenolate mophetyl, (q) cannabinoid receptor agonists such as Sativex, (r) chemokine CCR1 antagonists such as MLN-3897 or PS-031291, (s) chemokine CCR2 antagonists such as INCB-8696, (t) interferon alpha such as Sumiferon MP, (u) NF-kappaB activation inhibitors such as FAE and MLN-0415, (v) JAK inhibitors such as tesocitinib citrate or INCB018424, (W) Syk inhibitors, such as R-112, (x) PKC inhibitors, such as NVP-AEB071, (y) phosphosdiesterase IV inhibitors such as GRC-4039, (z) P38 Inhibitors such as ARRY-797, and (aa) MEK inhibitors, such as ARRY-142886 or ARRY-438162

The combinations of the invention may be used in the treatment of disorders which are susceptible to amelioration by sphingosine-1-phosphate receptors agonists (S1P1). Thus, the present application encompasses methods of treatment of these disorders, as well as the use of the combinations of the invention in the manufacture of a medicament for the treatment of these disorders.

Preferred examples of such disorders are multiple sclerosis, transplant rejection, systemic lupus erythematosus, asthma, psoriasis, rheumatoid arthritis, psoriatic arthritis and Crohn's disease, more preferably multiple sclerosis, transplant rejection, asthma and rheumatoid arthritis, and most preferably multiple sclerosis.

The active compounds in the combinations of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compounds in the combination, i.e. the sphingosine-1-phosphate agonist of the invention, and the other optional active compounds may be administered together in the same pharmaceutical composition or in different compositions intended for separate, simultaneous, concomitant or sequential administration by the same or a different route.

One execution of the present invention consists of a kit of parts comprising a sphingosine-1-phosphate agonist of the invention together with instructions for simultaneous, concurrent, separate or sequential use in combination with another active compound useful in the treatment of multiple sclerosis, transplant rejection, systemic lupus erythematosus, asthma, psoriasis, rheumatoid arthritis, psoriatic arthritis and Crohn's disease,

Another execution of the present invention consists of a package comprising a sphingosine-1-phosphate agonist of formula (I) and another active compound useful in the treatment of multiple sclerosis, transplant rejection, systemic lupus erythematosus, asthma, psoriasis, rheumatoid arthritis, psoriatic arthritis and Crohn's disease,

The pharmaceutical formulations may conveniently be presented in unit dosage form and may be prepared by any of the methods well known in the art of pharmacy.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2µg and 150 µg of each therapeutically active ingredient. Alternatively, the active ingredient (s) may be presented without excipients.

Packaging of the formulation for inhalation may be carried out by using suitable inhaler devices such as Genuair^{®} (formerly known as Novolizer SD2FL) which is described in the following patent applications: WO 97/000703, WO 03/000325, WO 03/061742 and WO 2006/008027.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

Effective doses are normally in the range of 2-2000 mg of active ingredient per day. Daily dosage may be administered in one or more treatments, preferably from 1 to 4 treatments, per day. Preferably, the active ingredients are administered once or twice a day.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The following preparations forms are cited as formulation examples:

### COMPOSITION EXAMPLE 1

50,000 capsules, each containing 100 mg of 2-{2,6-dimethyl-4-[5-(1-methyl-5-phenyl-1H-pyrazol-3-yl)-1,2,4-oxadiazol-3-yl]phenoxy}-2,2-difluoroacetamide (active ingredient), were prepared according to the following formulation:

| | |
|---|---|
| Active ingredient | 5 Kg |
| Lactose monohydrate | 10 Kg |
| Colloidal silicon dioxide | 0.1 Kg |
| Corn starch | 1 Kg |
| Magnesium stearate | 0.2 Kg |

### Procedure

The above ingredients were sieved through a 60 mesh sieve, and were loaded into a suitable mixer and filled into 50,000 gelatine capsules.

### COMPOSITION EXAMPLE 2

50,000 tablets, each containing 50 mg of 2-{2,6-dimethyl-4-[5-(1-methyl-5-phenyl-1H-pyrazol-3-yl)-1,2,4-oxadiazol-3-yl]phenoxy}-2,2-difluoroacetamide (active ingredient), were prepared from the following formulation:

| | |
|---|---|
| Active ingredient | 2.5 Kg |
| Microcrystalline cellulose | 1.95 Kg |
| Spray dried lactose | 9.95 Kg |
| Carboxymethyl starch | 0.4 Kg |
| Sodium stearyl fumarate | 0.1 Kg |
| Colloidal silicon dioxide | 0.1 Kg |

### Procedure

All the powders were passed through a screen with an aperture of 0.6 mm, then mixed in a suitable mixer for 20 minutes and compressed into 300 mg tablets using 9 mm disc and flat bevelled punches. The disintegration time of the tablets was about 3 minutes.

## Claims

1. A compound of formula (I), or a pharmaceutically acceptable salt or N-oxide or solvate or deuterated derivative thereof 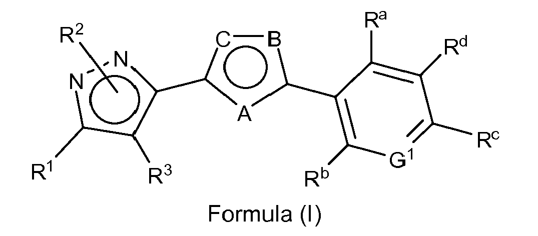 wherein:
• A is selected from the group consisting of -N(H)ᵢ-, -O- and -S-, wherein i is as defined below,
• B and C are independently selected from the group consisting of -N- and -O-, with the proviso that at least two of A, B and C are nitrogen atoms,
• i is 1 when B and C both represent N, and is otherwise 0,
• G¹ is selected from the group consisting of -CR^{e}= and -N=,
• R¹ represents a hydrogen atom, a linear or branched C₁₋₆ alkyl group, a -(C₁₋₄ alkyl)(C₃₋₇ cycloalkyl) group, a linear or branched C₁₋₄ alkoxy group, a mono- or di- (C₁₋₂ alkyl)amino group, a C₃₋₇ cycloalkyl group, a benzyl group or a C₅₋₁₀ aryl group, wherein the aryl group is optionally substituted with one or more substituents selected from halogen atoms, a C₁₋₂ alkyl group, a C₁₋₂ alkoxy group, and a -CF₃ group,
• R² represents a linear or branched C₁₋₄ alkyl group, a -(C₁₋₄ alkyl)(C₃₋₇ cycloalkyl) group, a C₅₋₁₀ aryl group, or a benzyl group, with the proviso that only one of R¹ R² and R³ is aryl,
• R³ represents a hydrogen atom, a linear or branched C₁₋₄ alkyl group, a -(C₁₋₄ alkyl)(C₃₋₇ cycloalkyl) group, a linear or branched C₁₋₄ alkoxy group, or a C₅₋₁₀ aryl group, wherein the aryl group is optionally substituted with one or more substituents selected from halogen atoms, a C₁₋₂ alkyl group, a C₁₋₂ alkoxy group, and a -CF₃ group, with the proviso that R³ and R¹ cannot be both a hydrogen atom,
• R^{a} and R^{b} independently represent a hydrogen atom or a linear or branched C₁₋₄ alkyl group,
• R^{d} and R^{e} independently represent a hydrogen atom, a linear or branched C₁₋₄ alkyl group, a C₁₋₂ alkoxy group, a C₃₋₄ cycloalkyl group or a -CF₃ group,
• R^{c} represents a hydroxy group, a C₂₋₄ alkyl group substituted with one or more substituents selected from halogen atom, hydroxy group, carboxy group and amino group; a C₁₋₃ alkoxy group substituted with one or more substituents selected from amino group, halogen atoms and carbamoyl group, or R^{c} represents -(CH₂)₂NR'R" group or-O-(CH₂)₂NR'R" group, wherein
o R' represents a hydrogen atom or a C₁₋₄ alkyl group, and R" represents a C₁₋₂ alkyl group substituted with one or more substituents selected from halogen atoms, and carboxy group; or
o R' and R" together with the nitrogen atom to which they are attached form a 4 to 6 membered, saturated heterocyclic ring optionally containing one or two additional nitrogen atom as heteroatom and substituted with one or more substituents selected from a carboxy group and a C₁₋₂ alkyl group substituted with a carboxy group.

2. A compound according to claim 1, wherein both A and B are -N- groups and C is -O- group.

3. A compound according to claim 1, wherein both A and C are -N- groups and B is -O- group

4. A compound according to claim 1 wherein A represents -O- or -S- and both B and C represent -N- groups

5. A compound according to any one of claims 1 to 4 wherein G¹ represents -CR^{e}= and R^{d} and/or R^{e} represents a hydrogen atom or a C₁₋₂ alkyl group, preferably a methyl group.

6. A compound according to any one of claims 1 to 5 wherein R¹ represents a branched C₃₋₄ alkyl group, a linear or branched C₁₋₄ alkoxy group, a di- (C₁₋₂ alkyl)amino group, a C₄₋₆ cycloalkyl group, a benzyl group or a C₅₋₆ aryl group, wherein the aryl group is optionally substituted with one substituent selected from a halogen atom, a C₁₋₂ alkoxy group and a -CF₃ group.

7. A compound according to claim 6 wherein R¹ represents a branched C₃₋₄ alkyl group, a linear or branched C₃₋₄ alkoxy group, a di-(C₁₋₂ alkyl)amino group, a cyclohexyl or a phenyl group, wherein the phenyl group is optionally substituted with one substituent selected from chlorine atom, a methoxy group and a -CF₃ group, preferably R¹ represents a branched C₃₋₄ alkyl group, a linear or branched C₃₋₄ alkoxy group or a di- (C₁₋₂ alkyl)amino group.

8. A compound according to any one of claims 1 to 7 wherein R² represents a C₁₋₂ alkyl group or a -(C₁₋₂ alkyl)(C₃₋₄ cycloalkyl) group, preferably R² represents a methyl group, an ethyl group or a cyclopropyl-methyl group, more preferably methyl or ethyl group.

9. A compound according to any one of claims 1 to 8, wherein R³ represents a hydrogen atom, a C₁₋₂ alkyl group, a -(C₁₋₂ alkyl)(C₃₋₄ cycloalkyl) group, a C₁₋₂ alkoxy group, preferably R³ represents a hydrogen atom or a methyl group, more preferably a hydrogen atom.

10. A compound according to any one of claims 1 to 9 wherein R^{a} and R^{b} independently represent a hydrogen atom or a methyl group, preferably both R^{a} and R^{b} represent a hydrogen atom.

11. A compound according to any one of claims 1 to 10 wherein R^{d} and R^{e} independently represent a hydrogen atom, a C₁₋₂ alkyl group, or a -CF₃ group, preferably R^{d} represents a hydrogen atom, a methyl group, an ethyl group or - CF₃ group, more preferably a methyl group or CF₃ group.

12. A compound according to any one of claims 1-11, wherein R^{c} represents a C₂₋₄ alkyl group substituted with one or more substituents selected from a hydroxy group, a carboxy group and an amino group or R^{c} represents -(CH₂)₂NR'R" group, wherein
o R' represents a hydrogen atom or a methyl group, and R" represents a C₁₋₂ alkyl group substituted with one or more substituents selected from halogen atoms and carboxy group; or
o R' and R" together with the nitrogen atom to which they are attached form a 4 to 6 membered, saturated heterocyclic ring which is substituted with one substituent selected from carboxy group and methyl group substituted with a carboxy group.

13. A compound according to claim 12, wherein R^{c} represents -(CH₂)₂NR'R" group, wherein
o R' represents a hydrogen atom or a methyl group, and R" represents a C₁₋₂ alkyl group substituted with a carboxy group; or
o R' and R" together with the nitrogen atom to which they are attached form a 4 to 6 membered, saturated heterocyclic ring which is substituted with a carboxy group.

14. A compound according to any one of claims 1-13 wherein R¹ represents a branched C₃₋₄ alkyl group, a linear or branched C₃₋₄ alkoxy group or a di-(C₁₋₂ alkyl)amino group, R² represents a methyl or ethyl group, R³ represents a hydrogen atom, both R^{a} and R^{b} represent a hydrogen atom, G¹ represents - CR^{e}=, one of R^{d} and R^{e} represents a methyl group or CF₃ group, and the other represents a hydrogen atom or a methyl group, and R^{c} represents -(CH₂)₂NR'R" group, wherein
o R' represents a hydrogen atom or a methyl group, and R" represents a C₁₋₂ alkyl group substituted with a carboxy group; or
o R' and R" together with the nitrogen atom to which they are attached form a 4 to 6 membered, saturated heterocyclic ring which is substituted with a carboxy group.

15. A compound according to claim 14 wherein both R^{d} and R^{e} are methyl group.

16. A compound according to claim 14 wherein one of R^{d} and R^{e} represents -CF₃ group and the other is a hydrogen atom.

17. A compound according to claim 1, wherein
• G¹ is selected from the group consisting of -CR^{e}= and -N=,
• R¹ represents a hydrogen atom, an isopropyl group, an isobutyl group, a tert-butyl group, a neopentyl group, a propoxy group, an isopropoxy group, a diethylamino group, a cyclohexyl group, a benzyl group, a phenyl group which is optionally substituted with one substituent selected from a chlorine atom, a fluorine atom, a methyl group, a methoxy group and a -CF₃ group,
• R² represents a methyl group, an ethyl group, a cycloproylmethyl group or a phenyl group,
• R³ represents a hydrogen atom, a methyl group or a phenyl group, with the proviso that R³ and R¹ cannot be both a hydrogen atom,
• R^{a} and R^{b} independently represent a hydrogen atom or a methyl group,
• R^{d} and R^{e} independently represent a hydrogen atom, a methyl group, an ethyl group or a -CF₃ group,
• R^{c} represents a hydroxy group, a C₂₋₄ alkyl group substituted with 1 or 2 substituents selected from a halogen atom, a hydroxy group, a carboxy group and an amino group; a C₁₋₃ alkoxy group substituted with 1 to 3 substituents selected from an amino group, a fluorine atom and a carbamoyl group, or R^{c} represents -(CH₂)₂NR'R" group or-O-(CH₂)₂NR'R" group, wherein
o R' represents a hydrogen atom or a methyl group, and R" represents a C₁₋₂ alkyl group substituted with 1 to 3 substituents selected from a fluorine atom and a carboxy group; or
o R' and R" together with the nitrogen atom to which they are attached form a 4 to 6 membered, saturated heterocyclic ring which is substituted with one substituent selected from a carboxy group and a carboxymethyl group.

18. A compound according to claim 1 which is one of
3-{2,6-dimethyl-4-[5-(1-methyl-5-phenyl-1H-pyrazol-3-yl)-1,2,4-oxadiazol-3-yl]phenyl}propane-1,2-diol,
N-(2-{2,6-dimethyl-4-[5-(1-methyl-5-phenyl-1H-pyrazol-3-yl)-1,2,4-oxadiazol-3-yl]phenyl}ethyl)-2,2,2-trifluoroethanamine,
N-(2-{2,6-dimethyl-4-[5-(1-methyl-5-phenyl-1H-pyrazol-3-yl)-1,2,4-oxadiazol-3-yl]phenyl}ethyl)glycine,
1-(2-{2,6-dimethyl-4-[5-(1-methyl-5-phenyl-1H-pyrazol-3-yl)-1,2,4-oxadiazol-3-yl]phenyl}ethyl)piperidine-4-carboxylic acid,
3-ethyl-6-methyl-5-[5-(1-methyl-5-phenyl-1H-pyrazol-3-yl)-1,2,4-oxadiazol-3-yl]pyridin-2-ol,
(2-{2-ethyl-6-methyl-4-[5-(1-methyl-5-phenyl-1H-pyrazol-3-yl)-1,2,4-oxadiazol-3-yl]phenyl}ethyl)amine,
(2-{2-ethyl-6-methyl-4-[5-(1-methyl-5-phenyl-1H-pyrazol-3-yl)-1,2,4-oxadiazol-3-yl]phenyl}ethyl)amine,
[3-({3-ethyl-6-methyl-5-[5-(1-methyl-5-phenyl-1H-pyrazol-3-yl)-1,2,4-oxadiazol-3-yl]pyridin-2-yl}oxy)propyl]amine,
(2-{2,6-dimethyl-4-[5-(1-methyl-5-phenyl-1H-pyrazol-3-yl)-1,2,4-oxadiazol-3-yl]phenoxy}ethyl)amine,
2-{2,6-dimethyl-4-[5-(1-methyl-5-phenyl-1H-pyrazol-3-yl)-1,2,4-oxadiazol-3-yl]phenoxy}-2,2-difluoroacetamide,
(2-{2,6-dimethyl-4-[5-(1-methyl-5-phenyl-1H-pyrazol-3-yl)-1,2,4-oxadiazol-3-yl]phenoxy}-2,2-difluoroethyl)amine,
{2-[4-[5-(1-methyl-5-phenyl-1H-pyrazol-3-yl)-1,2,4-oxadiazol-3-yl]-2-(trifluoromethyl)phenoxy]ethyl}amine,
1-(2-{2,6-dimethyl-4-[5-(1-methyl-5-phenyl-1H-pyrazol-3-yl)-1,2,4-oxadiazol-3-yl]phenoxy}ethyl)piperidine-4-carboxylic acid,
3-ethyl-5-[5-(1-ethyl-5-phenyl-1H-pyrazol-3-yl)-1,2,4-oxadiazol-3-yl]-6-methylpyridin-2-ol,
5-{5-[5-(2-chlorophenyl)-1-ethyl-1H-pyrazol-3-yl]-1,2,4-oxadiazol-3-yl}-3-ethyl-6-methylpyridin-2-ol,
{2-[4-[5-(1-methyl-5-phenyl-1H-pyrazol-3-yl)-1,2,4-oxadiazol-3-yl]-2-(trifluoromethyl)phenyl]ethyl}amine,
5-{5-[3-(2-chlorophenyl)-1-ethyl-1H-pyrazol-5-yl]-1,2,4-oxadiazol-3-yl}-3-ethyl-6-methylpyridin-2-ol,
1-(2-{4-[5-(1-ethyl-5-phenyl-1H-pyrazol-3-yl)-1,2,4-oxadiazol-3-yl]-2,6-dimethylphenyl}ethyl)piperidine-4-carboxylic acid,
N-(2-{4-[5-(1-ethyl-5-phenyl-1H-pyrazol-3-yl)-1,2,4-oxadiazol-3-yl]-2,6-dimethylphenyl}ethyl)glycine,
1-(2-{4-[5-(1-ethyl-5-phenyl-1H-pyrazol-3-yl)-1,2,4-oxadiazol-3-yl]-2,6-dimethylphenyl}ethyl)azetidine-3-carboxylic acid,
N-(2-{4-[5-(1-ethyl-5-phenyl-1H-pyrazol-3-yl)-1,2,4-oxadiazol-3-yl]-2,6-dimethylphenyl}ethyl)-N-methylglycine,
[1-(2-{4-[5-(1-ethyl-5-phenyl-1H-pyrazol-3-yl)-1,2,4-oxadiazol-3-yl]-2,6-dimethylphenyl}ethyl)piperidin-4-yl]acetic acid,
1-(2-{4-[5-(1-ethyl-5-phenyl-1H-pyrazol-3-yl)-1,2,4-oxadiazol-3-yl]-2,6-dimethylphenyl}ethyl)pyrrolidine-3-carboxylicacid,
1-[2-(4-{5-[5-(2-chlorophenyl)-1-ethyl-1H-pyrazol-3-yl]-1,2,4-oxadiazol-3-yl}-2,6-dimethylphenyl)ethyl]piperidine-4-carboxylic acid,
1-(2-{4-[5-(1-ethyl-5-isopropyl-1H-pyrazol-3-yl)-1,2,4-oxadiazol-3-yl]-2,6-dimethylphenyl}ethyl)piperidine-4-carboxylicacid,
1-[2-(4-{5-[5-(3-chlorophenyl)-1-ethyl-1H-pyrazol-3-yl]-1,2,4-oxadiazol-3-yl}-2,6-dimethylphenyl)ethyl]piperidine-4-carboxylic acid,
1-(2-{3-methyl-4-[5-(1-methyl-5-phenyl-1H-pyrazol-3-yl)-1,2,4-oxadiazol-3-yl]phenyl}ethyl)piperidine-4-carboxylic acid,
1-[2-(4-{5-[1-ethyl-5-(3-fluorophenyl)-1H-pyrazol-3-yl]-1,2,4-oxadiazol-3-yl}-2,6-dimethylphenyl)ethyl]piperidine-4-carboxylic acid,
1-(2-{2-methyl-4-[5-(1-methyl-5-phenyl-1H-pyrazol-3-yl)-1,2,4-oxadiazol-3-yl]phenyl}ethyl)piperidine-4-carboxylic acid,
1-{2-[4-[5-(1-methyl-5-phenyl-1H-pyrazol-3-yl)-1,2,4-oxadiazol-3-yl]-2-(trifluoromethyl)phenyl]ethyl}piperidine-4-carboxylic acid,
1-(2-{4-[5-(5-tert-butyl-1-methyl-1H-pyrazol-3-yl)-1,2,4-oxadiazol-3-yl]-2,6-dimethylphenyl}ethyl)piperidine-4-carboxylic acid,
1-(2-{4-[5-(5-cyclohexyl-1-ethyl-1H-pyrazol-3-yl)-1,2,4-oxadiazol-3-yl]-2,6-dimethylphenyl}ethyl)piperidine-4-carboxylic acid,
1-(2-{4-[5-(5-cyclohexyl-1-ethyl-1H-pyrazol-3-yl)-1,2,4-oxadiazol-3-yl]-2,6-dimethylphenyl}ethyl)azetidine-3-carboxylic acid,
1-[2-(4-{5-[1-ethyl-5-(4-methoxyphenyl)-1H-pyrazol-3-yl]-1,2,4-oxadiazol-3-yl}-2,6-dimethylphenyl)ethyl]piperidine-4-carboxylic acid,
1-(2-{4-[5-(1-ethyl-5-isobutyl-1H-pyrazol-3-yl)-1,2,4-oxadiazol-3-yl]-2,6-dimethylphenyl}ethyl)piperidine-4-carboxylic acid,
1-(2-{4-[5-(1-ethyl-5-isobutyl-1H-pyrazol-3-yl)-1,2,4-oxadiazol-3-yl]-2,6-dimethylphenyl}ethyl)azetidine-3-carboxylic acid,
1-[2-(4-{5-[1-ethyl-5-(4-fluorophenyl)-1H-pyrazol-3-yl]-1,2,4-oxadiazol-3-yl}-2,6-dimethylphenyl)ethyl]piperidine-4-carboxylic acid,
1-[2-(4-{5-[1-ethyl-5-(2-methoxyphenyl)-1H-pyrazol-3-yl]-1,2,4-oxadiazol-3-yl}-2,6-dimethylphenyl)ethyl]piperidine-4-carboxylic acid,
1-(2-{4-[5-(1-ethyl-5-phenyl-1H-pyrazol-3-yl)-1,2,4-oxadiazol-3-yl]-2,5-dimethylphenyl}ethyl)piperidine-4-carboxylic acid,
1-[2-(4-{5-[1-ethyl-5-(2-fluorophenyl)-1H-pyrazol-3-yl]-1,2,4-oxadiazol-3-yl}-2,6-dimethylphenyl)ethyl]piperidine-4-carboxylic acid,
1-(2-{4-[5-(1-ethyl-4-methyl-5-phenyl-1H-pyrazol-3-yl)-1,2,4-oxadiazol-3-yl]-2,6-dimethylphenyl}ethyl)piperidine-4-carboxylic acid,
1-{2-[4-(5-{1-ethyl-5-[2-(trifuoromethyl)phenyl]-1H-pyrazol-3-yl}-1,2,4-oxadiazol-3-yl)-2,6-dimethylphenyl]ethyl}piperidine-4-carboxylic acid,
1-[2-(4-{5-[1-ethyl-5-(2-methylphenyl)-1H-pyrazol-3-yl]-1,2,4-oxadiazol-3-yl}-2,6-dimethylphenyl)ethyl]piperidine-4-carboxylic acid,
3-{4-[5-(1-ethyl-5-phenyl-1H-pyrazol-3-yl)-1,2,4-oxadiazol-3-yl]-2,6-dimethylphenyl}propanoic acid,
N-(2-{2,6-dimethyl-4-[5-(1-methyl-5-phenyl-1H-pyrazol-3-yl)-1,3,4-oxadiazol-2-yl]phenyl}ethyl)glycine,
1-(2-{4-[5-(5-benzyl-1-ethyl-1H-pyrazol-3-yl)-1,2,4-oxadiazol-3-yl]-2,6-dimethylphenyl}ethyl)piperidine-4-carboxylic acid,
1-[2-(4-{5-[5-(2,2-dimethylpropyl)-1-ethyl-1H-pyrazol-3-yl]-1,2,4-oxadiazol-3-yl}-2,6-dimethylphenyl)ethyl]piperidine-4-carboxylic acid,
1-{2-[4-[5-(1-ethyl-5-cyclohexyl-1H-pyrazol-3-yl)-1,2,4-oxadiazol-3-yl]-2-(trifluoromethyl)phenyl]ethyl}piperidine-4-carboxylic acid,
1-(2-{4-[5-(1-ethyl-5-isobutyl-4-methyl-1H-pyrazol-3-yl)-1,2,4-oxadiazol-3-yl]-2,6-dimethylphenyl}ethyl)piperidine-4-carboxylic acid,
1-(2-{4-[5-(1-ethyl-5-isobutyl-1H-pyrazol-3-yl)-4H-1,2,4-triazol-3-yl]-2,6-dimethylphenyl}ethyl)piperidine-4-carboxylic acid,
1-[2-(4-{5-[1-(cyclopropylmethyl)-5-phenyl-1H-pyrazol-3-yl]-1,2,4-oxadiazol-3-yl}-2,6-dimethylphenyl)ethyl]piperidine-4-carboxylic acid,
1-(2-{4-[5-(1-ethyl-5-isopropoxy-1H-pyrazol-3-yl)-1,2,4-oxadiazol-3-yl]-2,6-dimethylphenyl}ethyl)piperidine-4-carboxylic acid,
1-(2-{4-[5-(1-ethyl-5-isobutyl-1H-pyrazol-3-yl)-1,3,4-thiadiazol-2-yl]-2,6-dimethylphenyl}ethyl)piperidine-4-carboxylic acid,
1-(4-(3-(5-cyclohexyl-1-ethyl-1H-pyrazol-3-yl)-1,2,4-oxadiazol-5-yl)-2,6-dimethylphenethyl)piperidine-4-carboxylic acid,
1-(4-(5-(1-ethyl-4-phenyl-1H-pyrazol-3-yl)-1,2,4-oxadiazol-3-yl)-2,6-dimethylphenethyl)piperidine-4-carboxylic acid,
1-(4-(5-(1-ethyl-4-phenyl-1H-pyrazol-3-yl)-1,2,4-oxadiazol-3-yl)-2-(trifluoromethyl)phenethyl)piperidine-4-carboxylic acid,
1-(4-(5-(1-ethyl-5-isobutyl-1H-pyrazol-3-yl)-1,2,4-oxadiazol-3-yl)-2-(trifluoromethyl)phenethyl)piperidine-4-carboxylic acid,
1-(4-(5-(1-ethyl-5-isobutyl-1H-pyrazol-3-yl)-1,2,4-oxadiazol-3-yl)-2-(trifluoromethyl)phenethyl)azetidine-3-carboxylic acid,
2-((4-(5-(1-ethyl-5-isobutyl-1H-pyrazol-3-yl)-1,2,4-oxadiazol-3-yl)-2-(trifluoromethyl)phenethyl)(methyl)amino)acetic acid,
3-(4-(5-(5-(diethylamino)-1-ethyl-1H-pyrazol-3-yl)-1,2,4-oxadiazol-3-yl)-2,6-dimethylphenyl)propanoic acid,
1-(4-(5-(1-ethyl-5-propoxy-1H-pyrazol-3-yl)-1,2,4-oxadiazol-3-yl)-2,6-dimethylphenethyl)piperidine-4-carboxylic acid,
1-(4-(5-(1-ethyl-5-isopropoxy-1H-pyrazol-3-yl)-1,2,4-oxadiazol-3-yl)-2-(trifluoromethyl)phenethyl)piperidine-4-carboxylic acid,
or a pharmaceutically acceptable salt or N-oxides or solvates or deuterated derivative thereof

19. A compound according to any one of claims 1-18 for use in the treatment of the human or animal body by therapy.

20. A compound according to any one of claims 1 to 18 for use in the treatment of a pathological condition or disease susceptible to amelioration by sphingosine-1-phosphate receptors (S1P1) agonists, which condition or disease is preferably selected from autoimmune diseases, chronic immune and inflammatory diseases, transplant rejection, malignant neoplastic diseases, angiogenic-related disorders, pain, neurological diseases,viral and infectious diseases, more preferably selected from multiple sclerosis, transplant rejection, systemic lupus erythematosus, asthma, psoriasis, rheumatoid arthritis, psoriatic arthritis and Crohn's disease.

21. A pharmaceutical composition comprising a compound as defined in any one of claims 1 to 18 in association with a pharmaceutically acceptable diluent or carrier.

22. A method for treating a subject afflicted with a pathological condition or disease as defined in claim 20, which comprises administering to said subject an effective amount of a compound as defined in any one of claims 1 to 18.

23. A combination product comprising (i) a compound according to any one of claims 1 to 18; and (ii) another compound selected from:
a) Interferons comprising Interferon beta 1 a such as Avonex from Biogen Idec, CinnoVex from CinnaGen and Rebif from EMD Serono, and Interferon beta 1b such as Betaferon from Schering and Betaseron from Berlex,
b) Immunomodulators such as glatiramer acetate,
c) Inhibitors of DNA synthesis and repair, such as Mitoxantrone,
d) Anti-alpha 4 integrin antibodies, such as Natalizumab (Tysabri),
e) Alpha 4 integrin antagonists such as R-1295 , TBC-4746, CDP-323, ELND-002, Firategrast and TMC-2003,
f) Dyhydrofolate reductase inhibitors, such as Methotrexate or CH-1504,
g) Glucocorticoids such as prednisone or methylprednisolone,
h) DHODH inhibitors such as teriflunomide and leflunomide,
*i)* Fumaric acid esters, such as *BG-12*,
*j)* Immunomodulators such as Laquinimod,
k) Anti-CD20 monoclonal antibodies such as Rituximab, Ocrelizumab Ofatumumab or TRU-015,
l) Anti-CD52 such as alemtuzumab,
m) Anti-CD25 such as daclizumab,
n) Anti-CD88, such as eculizumab or pexilizumab,
o) Calcineurin inhibitors such as cyclosporine A or tacrolimus,
p) IMPDH inhibitors, such as mycophenolate mophetyl,
q) Cannabinoid receptor agonists such as Sativex,
r) Chemokine CCR1 antagonists such as MLN-3897 or PS-031291,
s) Chemokine CCR2 antagonists such as INCB-8696,
t) Interferon alpha such as Sumiferon MP,
u) NF-kappaB activation inhibitors such as FAE and MLN-0415,
v) JAK inhibitors such as tasocitinib citrate or INCB018424,
w) Syk inhibitors, such as R-112,
x) PKC inhibitors, such as NVP-AEB071,
y) Phosphosdiesterase IV inhibitors such as GRC-4039,
z) P38 Inhibitors such as ARRY-797, and
aa) MEK inhibitors, such as ARRY-142886 or ARRY-438162,
